(19) Europäisches Patentamt
European Patent Office
Office européen des brevets

(11) EP 1 180 991 B1

(12) EUROPEAN PATENT SPECIFICATION

(45) Date of publication and mention of the grant of the patent:
**19.05.2010 Bulletin 2010/20**

(21) Application number: **00905839.7**

(22) Date of filing: **01.02.2000**

(51) Int Cl.:
***A61K 31/352*** *(2006.01)* ***A61P 35/00*** *(2006.01)*
***A61P 35/02*** *(2006.01)* ***A61P 29/00*** *(2006.01)*
***A61P 37/06*** *(2006.01)* ***A61P 17/00*** *(2006.01)*
***C07D 493/20*** *(2006.01)*

(86) International application number:
**PCT/US2000/002332**

(87) International publication number:
**WO 2000/044216 (03.08.2000 Gazette 2000/31)**

(54) **GAMBOGIC ACID DERIVATIVES AS ACTIVATORS OF CASPASES AND INDUCERS OF APOPTOSIS**

GAMBOGISCHE SÄURE DERIVATIVE ALS CASPASENAKTIVATOREN UND APOPTOSIS INDUKTIONEN

ACIDE DE GOMME-GUTE DERIVES EN TANT QU'ACTIVATEURS DE CASPASES ET INDUCTEURS D'APOPTOSE

(84) Designated Contracting States:
**AT BE CH CY DE DK ES FI FR GB GR IE IT LI LU MC NL PT SE**
Designated Extension States:
**AL LT LV MK RO SI**

(30) Priority: **01.02.1999 US 118102 P**
**21.05.1999 US 135424 P**

(43) Date of publication of application:
**27.02.2002 Bulletin 2002/09**

(73) Proprietor: **Cytovia, Inc.**
**San Diego, CA 92121 (US)**

(72) Inventors:
- **CAI, Sui, Xiong**
**Sand Diego, CA 92130 (US)**
- **ZHANG, Han-Zhong**
**San Diego, CA 92123 (US)**
- **WANG, Yan**
**Sand Diego, CA 92129 (US)**
- **TSENG, Ben**
**Sand Diego, CA 92130 (US)**
- **KASIBHATLA, Shailaja**
**San Diego, CA 92126 (US)**
- **DREWE, John, A.**
**Costa Mesa, CA 92627 (US)**

(74) Representative: **Duckett, Anthony Joseph et al**
**Mathys & Squire LLP**
**120 Holborn**
**London**
**EC1N 2SQ (GB)**

(56) References cited:
- **CAO, SHU-GENG ET AL: "Cytotoxic caged tetraprenylated xanthonoids from Garcinia gaudichaudii (Guttiferae)" TETRAHEDRON LETTERS , 39(20), 3353-3356 CODEN: TELEAY; ISSN: 0040-4039, 1998, XP002302372**
- **CAO, SHU-GENG ET AL: "Novel cytotoxic polyprenylated xanthonoids from Garcinia gaudichaudii (Guttiferae)" TETRAHEDRON , 54 (36), 10915-10924 CODEN: TETRAB; ISSN: 0040-4020, 1998, XP002302373**
- **LIN ET AL: "Isogambonic acid and isomorellinol from Garcinia hanburyi" CHEMICAL ABSTRACTS + INDEXES, AMERICAN CHEMICAL SOCIETY. COLUMBUS, US, vol. 12, no. 119, 20 September 1993 (1993-09-20), XP002116433 ISSN: 0009-2258**
- **DATABASE BIOSIS [Online] BIOSCIENCES INFORMATION SERVICE, PHILADELPHIA, PA, US; 1991, QU B-X ET AL: "THE EXPERIMENTAL STUDIES ON ANTINEOPLASTIC ACTION OF GAMBOGIC II" XP002302374 Database accession no. PREV199191109495 & ZHONGGUO ZHONGLIU LINCHUANG, vol. 18, no. 1, 1991, pages 50-52, XP008037521 ISSN: 1000-8179**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- JUN ASANO ET AL: "CYTOTOXIC XANTHONES FROM GARCINIA HANBURYI" PHYTOCHEMISTRY, PERGAMON PRESS, GB, vol. 41, no. 3, 1996, pages 815-820, XP002928653 ISSN: 0031-9422
- SCHEINMANN, F. ET AL: "Application of the Claisen rearrangement to the synthesis of heterocyclic bicyclo[2.2.2]octenones. Approach to the morellins based on new biogenetic suggestions" JOURNAL OF THE CHEMICAL SOCIETY [SECTION] D: CHEMICAL COMMUNICATIONS , (16), 966-7 CODEN: CCJDAO; ISSN: 0577-6171, 1971, XP008037445
- YATES P ET AL: "ACETYL-ALPHA-GAMBOGIC ACID" TETRAHEDRON LETTERS, ELSEVIER SCIENCE PUBLISHERS, AMSTERDAM, NL, no. 24, 1963, pages 1623-1629, XP002928652 ISSN: 0040-4039
- ADAWADKAR, P. D. ET AL: "Coloring matters of Garcinia morella: Part VIII. Morellinol, dihydromorelloflavone and morelloflavone-7''-.beta.-glucoside" INDIAN JOURNAL OF CHEMISTRY, SECTION B: ORGANIC CHEMISTRY INCLUDING MEDICINAL CHEMISTRY , 14B(1), 19-21 CODEN: IJSBDB; ISSN: 0376-4699, 1976, XP008037372
- KARANJGAONKAR C G ET AL: "MORELLIC, ISOMORELLIC AND GAMBOGIC ACIDS A,B" TETRAHEDRON LETTERS, ELSEVIER SCIENCE PUBLISHERS, AMSTERDAM, NL, no. 7, 1966, pages 687-691, XP002928911 ISSN: 0040-4039
- SCHWARTZ S M: "Cell death and the caspase cascade." CIRCULATION. 27 JAN 1998, vol. 97, no. 3, 27 January 1998 (1998-01-27), pages 227-229, XP002298456 ISSN: 0009-7322
- VILLA P ET AL: "Caspases and caspase inhibitors" TIBS TRENDS IN BIOCHEMICAL SCIENCES, ELSEVIER PUBLICATION, CAMBRIDGE, EN, vol. 22, no. 10, 1 October 1997 (1997-10-01), pages 388-393, XP004091999 ISSN: 0968-0004
- DATABASE WPI Section Ch, Week 199951 Derwent Publications Ltd., London, GB; Class B04, AN 1999-600152 XP002302375 & PH 27 489 A (LEE S) 23 July 1993 (1993-07-23)
- CAO, S. G. ET. AL.: 'Novel cytotoxic polyprenylated xanthonoids from Garcinia gaudichaudii (Guttiferae)' TET. vol. 54, no. 36, September 1998, pages 10915 - 10924, XP004130029
- OLLIS, W. D. ET. AL.: 'The constitution of Gambogic acid' TET. vol. 21, 1965, pages 1453 - 1470, XP002928650
- AHMED, S. A. ET. AL.: 'Gamboge' J. CHEM. SOC. (C) 1966, pages 772 - 779, XP002928651
- YATES, P. ET. AL.: 'Aceyl-alpha Gambogic' TET. LET. no. 24, 1963, pages 1623 - 1629, XP002928652
- KARANJGAONKAR, C. G. ET. AL.: 'Coloring matters of Garcinia morella-(VI)morellic, isomorellic, and gambogic acids' TET. LETT. no. 7, 1966, pages 687 - 691, XP002928911
- ASANO, J. ET. AL.: 'Cytotoxic Xanthones from Garcinia Hanuryl' PHYTOCHEMISTRY vol. 41, no. 3, 1996, pages 815 - 820, XP002928653
- G.S.R. SUBBA RAO, ET. AL.: "Structure of moreollin, a prigment isolated from Garcinia Morella Desser" PROC. INDIAN ACAD. SCI, vol. 87a, 1978, pages 75-86,

## Description

### *Background of the Invention*

### *Field of the Invention*

This invention is in the field of medicinal chemistry. In particular, the invention relates to novel analogs of gambogic acid and derivatives of gambogic acid, and the discovery that these compounds are activators of caspases and inducers of apoptosis. The invention also relates to the use of these compounds as therapeutically effective anti-cancer agents.

### *Description of Background Art*

Organisms eliminate unwanted cells by a process variously known as regulated cell death, programmed cell death or apoptosis. Such cell death occurs as a normal aspect of animal development as well as in tissue homeostasis and aging (Glucksmann, A., Biol. Rev. Cambridge Philos. Soc. 26:59-86 (1951); Glucksmann, A., Archives de Biologie 76:419-437 (1965); Ellis, et al., Dev. 112:591-603 (1991); Vaux, et al., Cell 76:777-779 (1994)). Apoptosis regulates cell number, facilitates morphogenesis, removes harmful or otherwise abnormal cells and eliminates cells that have already performed their function. Additionally, apoptosis occurs in response to various physiological stresses, such as hypoxia or ischemia (PCT published application WO96/20721).

There are a number of morphological changes shared by cells experiencing regulated cell death, including plasma and nuclear membrane blebbing, cell shrinkage (condensation of nucleoplasm and cytoplasm), organelle relocalization and compaction, chromatin condensation and production of apoptotic bodies (membrane enclosed particles containing intracellular material) (Orrenius, S., J Internal Medicine 237:529-536 (1995)).

Apoptosis is achieved through an endogenous mechanism of cellular suicide (Wyllie, A.H., in Cell Death in Biology and Pathology, Bowen and Lockshin, eds., Chapman and Hall (1981), pp. 9-34). A cell activates its internally encoded suicide program as a result of either internal or external signals. The suicide program is executed through the activation of a carefully regulated genetic program (Wyllie, et al., Int. Rev. Cyt. 68:251 (1980); Ellis, et al., Ann. Rev. Cell Bio. 7:663 (1991)). Apoptotic cells and bodies are usually recognized and cleared by neighboring cells or macrophages before lysis. Because of this clearance mechanism, inflammation is not induced despite the clearance of great numbers of cells (Orrenius, S., J Internal Medicine 237:529-536 (1995)).

It has been found that a group of proteases are a key element in apoptosis (see, e.g. Thornberry, Chemistry and Biology 5:R97-R103 (1998); Thornberry, British Med Bull. 53:478-490 (1996)). Genetic studies in the nematode *Caenorhabditis elegans* revealed that apoptotic cell death involves at least 14 genes, two of which are the pro-apoptotic (death-promoting) *ced* (for *cell death abnormal*) genes, *ced-3* and *ced-4.* CED-3 is homologous to interleukin 1 beta-converting enzyme, a cysteine protease, which is now called caspase-1. When these data were ultimately applied to mammals, and upon further extensive investigation, it was found that the mammalian apoptosis system appears to involve a cascade of caspases, or a system that behaves like a cascade of caspases. At present, the caspase family of cysteine proteases comprises 14 different members, and more may be discovered in the future. All known caspases are synthesized as zymogens that require cleavage at an aspartyl residue prior to forming the active enzyme. Thus, caspases are capable of activating other caspases, in the manner of an amplifying cascade.

Apoptosis and caspases are thought to be crucial in the development of cancer (Apoptosis and Cancer Chemotherapy, Hickman and Dive, eds., Humana Press (1999)). There is mounting evidence that cancer cells, while containing caspases, lack parts of the molecular machinery that activates the caspase cascade. This makes the cancer cells lose their capacity to undergo cellular suicide and the cells become cancerous. In the case of the apoptosis process, control points are known to exist that represent points for intervention leading to activation. These control points include the CED-9-BCL-like and CED-3-ICE-like gene family products. These are intrinsic proteins that regulate the decision of a cell to survive or die and they execute part of the cell death process itself (see Schmitt, et al., Biochem. Cell. Biol. 75: 301-314 (1997)). BCL-like proteins include BCL-xL and BAX-alpha, which appear to function upstream of caspase activation. BCL-xL appears to prevent activation of the apoptotic protease cascade, whereas BAX-alpha accelerates activation of the apoptotic protease cascade.

It has been shown that chemotherapeutic (anti-cancer) drugs can trigger cancer cells to undergo suicide by activating the dormant caspase cascade. This may be a crucial aspect of the mode of action of most, if not all, known anticancer drugs (Los et al., Blood, Vol. 90, No 8:3118-3129 (1997); Friesen, et al., Nat. Med. 2:574 (1996)). The mechanism of action of current antineoplastic drugs frequently involves an attack at specific phases of the cell cycle. In brief, the cell cycle refers to the stages through which cells normally progress during their lifetimes. Normally, cells exist in a resting phase termed $G_o$. During multiplication, cells progress to a stage in which DNA synthesis occurs, termed S. Later, cell division, or mitosis occurs, in a phase called M. Antineoplastic drugs such as cytosine arabinoside, hydroxyurea,

6-mercaptopurine, and methotrexate are S phase specific, whereas antineoplastic drugs such as vincristine, vinblastine, and paclitaxel are M phase specific. Many antineoplastic drugs slow growing tumors. For example, colon cancers exist primarily in the $G_o$ phase, whereas rapidly proliferating normal tissues, for example bone marrow, exist primarily in the S or M phase. Thus, a drug like 6-mercaptopurine can cause bone marrow toxicity while remaining ineffective toward a slow growing tumor. Other aspects of the chemotherapy of neoplastic diseases are known to those skilled in the art (see, e.g., Hardman, et al., eds., Goodman and Gilman's The Pharmacological Basis of Therapeutics, Ninth Edition, McGraw-Hill, New York (1996), pp. 1225-1287). Thus, it is clear that the possibility exists for the activation of the caspase cascade, although the exact mechanisms for doing so are not clear at this point. It is equally clear that insufficient activity of the caspase cascade and consequent apoptotic events are implicated in various types of cancer. The development of caspase cascade activators and inducers of apoptosis is a highly desirable goal in the development of therapeutically effective antineoplastic agents. Moreover, since autoimmune diseases and certain degenerative diseases also involve the proliferation of abnormal cells, therapeutic treatment for these diseases could also involve the enhancement of the apoptotic process through the administration of appropriate caspase cascade activators and inducers of apoptosis.

Gambogic acid was isolated from gamboge and the structure was deduced from the $^1H$ NMR spectrum and by comparison with morellin, which also has the xanthone core of gambogic acid (Ahmad, S.A., et al. J Chem. Soc. (C) 772-779 (1966); Ollis, W.D., et al. Tetrahedron, 21:1453-1470 (1965)).

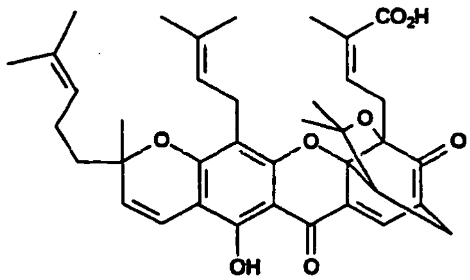

Asano J., et al., Phytochemistry, 41:815-820 (1996), reported the isolation of several xanthones, including gambogic acid from gamboge. They reported that gambogic acid is cytotoxic to both HeLa and HEL cells.

Lin, L.-J., et al., Magn. Reson. Chem. 31:340-347 (1993), reported the isolation of gambogic acid, as well as isogambogic acid and isomorellinol. All three compounds were reported to be cytotoxic against KB and KB-V1 cell lines.

Cao, S.-G., et al., Tetrahedron, 54:10915-10924 (1998) and Tetrahedron Letters, 39:3353-3356 (1998), reported the isolation of gaudichaudiones A-H, gaudichaudiic acids A-E, morellic acid and forbesione from *Garcinia gaudichaudii.* These compounds were said to exhibit significant cytotoxicity against several cancer cell lines.

Adawadkar, P.D., et al., Indian Journal of Chemistry 14B:19-21 (1976), reported the isolation and structural assignment of the pigment morellinol from the hexane extract of *Garcinia morella* bark.

Yates, P., et al., Tetrahedron Letters, 24:1623-1629 (1963), reported a structural assignment of acetyl-α-gambogic acid.

Subba Rao, G.S.R., et al., Proc. Indian Acad. Sci., 87A:75-86 (1978), reported the structure of moreollin and isomoreollin on the basis of structural data and partial synthesis.

### *Summary of the Invention*

The present invention is related to the discovery that gambogic acid analogs and derivatives, as represented in Formulae I-III, are activators of the caspase cascade and inducers of apoptosis.

The present invention accordingly provides the use of a compound having one of the Formulae I-III, or a pharmaceutically acceptable salt or prodrug thereof, as recited in claim 1, for the manufacture of a medicament for treating cancer in an animal.

The present invention also provides the use of a compound having one of the Formulae I-III, or a pharmaceutically acceptable salt or prodrug thereof, as recited in claim 9, for the manufacture of a medicament for treating drug resistant cancer in an animal.

The present invention also provides a compound having the Formula I, or a pharmaceutically acceptable salt or prodrug thereof, as recited in claim 15.

The present invention also provides a compound having the Formula II, or a pharmaceutically acceptable salt or prodrug thereof, as recited in claim 19.

The present invention also provides a pharmaceutical composition as recited in claim 26.

The present invention also provides a compound having one of the Formulae I-III, or a pharmaceutically acceptable salt or prodrug thereof, as defined in claim 1, for use in treating cancer in an animal.

The present invention also provides a compound having one of the Formulae I-III, or a pharmaceutically acceptable salt or prodrug thereof, as defined in claim 1, for use in treating drug resistant cancer in an animal.

***Brief Description of the Drawings***

Figs. 1A-C depict photographs of T47D human breast cancer cells treated with gambogic acid: control cells (Fig. 1A); cells treated with 2.5 μM of gambogic acid for 2 h (Fig. 1 B); cells treated with 2.5 μM of gambogic acid for 6 h (Fig. 1C).

Figs. 2A-B depict fluorescent photographs of T47D human breast cancer cells treated with gambogic acid and stained with a fluorescent DNA probe: control cells (Fig. 2A); cells treated with 10 μM of gambogic acid for 24 h (Fig. 2B).

Figs. 3A-C depict photographs of Jurkat leukemia cells treated with gambogic acid: control cells (Fig. 3A); cells treated with 10 μM of gambogic acid for 30 min (Fig. 3B); cells treated with 10 μM of gambogic acid in the presence of 10 μM of caspase inhibitor cbz-Val-Asp-fmk (Fig. 3C).

Fig. 4 depicts the caspase activity in T47D human breast cancer cells and MRC5 human non-transformed fibroblast cells treated for 2 h with different concentrations of gambogic acid.

Figs. 5A-D depict western blots of poly(ADP)ribose polymerase (PARP) cleavage. Fig. 5A, Jurkat leukemia cells: (a) DMSO control, (b) treated with 1 μM of staurosporine for 2 h, (c) inactive control, (d) treated with 2.5 μM of gambogic acid for 2 h. Fig. 5B, HL-60 human leukemia cancer cells: (a) DMSO control, (b) treated with 1 μM of staurosporine for 2 h, (c) inactive control, (d) treated with 2.5 μM of gambogic acid for 2 h. Fig. 5C, T47D human breast cancer cells: (a) DMSO control, (b) treated with 1 μM of staurosporine for 2 h, (c) treated with 2.5 μM of gambogic acid for 2 h, (d) treated with 5 μM of gambogic acid for 2 h, (e) DMSO control, (f) treated with 1 μM of staurosporine for 4 h, (g) treated with 2.5 μM of gambogic acid for 4 h, (h) treated with 5 μM of gambogic acid for 4 h. Fig. 5D, PC3 human prostate cancer cells: (a) DMSO control, (b) treated with 1 μM of staurosporine for 2 h, (c) treated with 2.5 μM of gambogic acid for 2 h, (d) treated with 5 μM of gambogic acid for 2 h, (e) DMSO control, (f) treated with 1 μM of staurosporine for 4 h, (g) treated with 2.5 μM of gambogic acid for 4 h, (h) treated with 5 μM of gambogic acid for 4 h.

***Detailed Description of the Invention***

The present invention arises out of the discovery that gambogic acid is a potent and highly efficaceous activator of the caspase cascade and inducer of apoptosis. Therefore gambogic acid is useful for treating disorders responsive to induction of apoptosis.

There are many functional groups in the structure of gambogic acid which can be modified. These include, but are not limited to, the carboxyl group, which can be converted to an ester, amide, ketone or alcohol and other functional groups; the ester and amide, in turn, may also contain other functional groups, such as the carboxyl of an amino acid, which can be further modified; the hydroxy group, may be converted to an ether, ester or other functional groups; the carbon-carbon double bond between C-9 and C-10 is part of an α,β-unsaturated ketone, which can react with a nucleophile, be reduced to a carbon-carbon single bond, or may be converted to an epoxide, which in turn may undergo further reaction; the carbon-carbon double bond between C-27 and C-28 is part of an α,β-unsaturated carboxyl, that may also react with a nucleophile, be reduced to a carbon-carbon single bond, or may be converted to a cyclopropane ring, which in turn may undergo further reaction; the two isoprene carbon-carbon double bonds at C-37/C-38 and C-32/C-33, may also be reduced to a carbon-carbon single bond, be cleaved to form an aldehyde group or a carboxyl group, both of which may be modified to other functional groups, or be converted to an epoxide, which in turn may undergo further reaction; the carbon-carbon double bond between C-3 and C-4 may also be reduced to a carbon-carbon single bond, or be converted to an epoxide that may undergo further reaction; the ketone group at C-12 may be reduced to an alcohol, or may be converted to an oxime, a semicarbazone, or an amino group; the other ketone group may also be reduced, or may be converted to other functional groups. In short, many derivatives of gambogic acid can be prepared.

In addition, analogs of gambogic acid, including isomorellin, morellic acid, desoxymorellin, gambogin, morelline dimethyl acetal, isomoreollin B Moreollic acid, gambogenic acid, gambogenin, isogambogenin, desoxygambogenin, gambogenin dimethyl acetal, gambogellic acid, hanburin (Asano, J., et al., Phytochemistry 41:815-820 (1996)), isogambogic acid, isomorellinol (Lin, L.-J., et al., Magn. Reson. Chem. 31:340-347 (1993)) and neo-gambogic acid (Lu, G.B., et al., Yao Hsueh Hsueh Pao 19:636-639 (1984)) can be isolated from gamboge. Other analogs of gambogic acid, including morellin, desoxymorellin, dihydroisomorellin (Bhat et al. Indian J. Chem. 2:405-409 (1964)) and moreollin (Rao

et al. Proc. Indian Acad Sci. 87A:75-86 (1978)), can be isolated from the seed of *Garcinia morella.* Morellinol can be isolated from the bark of *Garcinia morella* (Adawadkar et al. Indian J. Chem. 14B:19-21 (1976)). Gaudichaudiones (A-H) and gaudichaudiic acids A-E can be isolated from the leaves of *Garcinia gaudichaudii* (Guttiferae) (Cao, S.-G., et al., Tetrahedron 54(36):10915-10924 (1998) and Cao, S.-G., et al., Tetrahedron Lett. 39(20):3353-3356 (1998)), and forbesione can be isolated from *Garcinia forbesii* (Leong, Y.-W., et al., J Chem. Res., Synop. 392-393 (1996)).

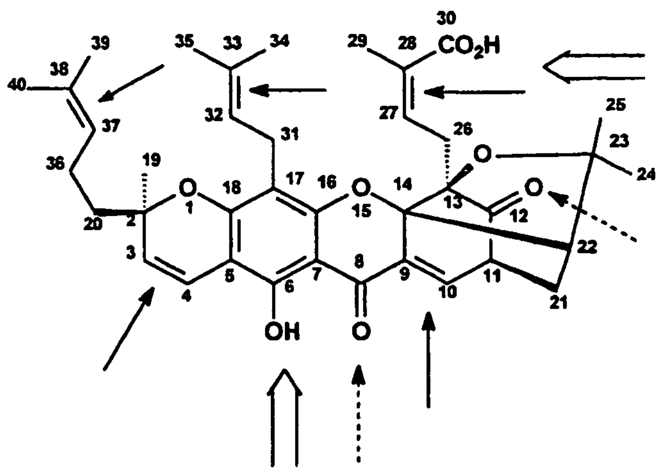

The present invention, therefore, also arises out of the discovery that novel derivatives and analogs of gambogic acid are also activators of the caspase cascade and inducers of apoptosis. Therefore these derivatives and analogs of gambogic acid are useful for treating disorders responsive to the induction of apoptosis.

The compounds useful in the present invention are gambogic acid analogs and derivatives as represented by Formulae I-III:

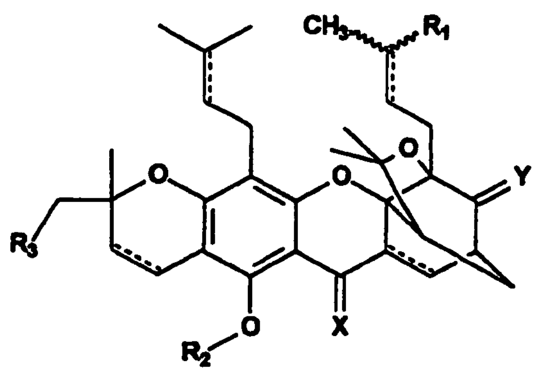

(I)

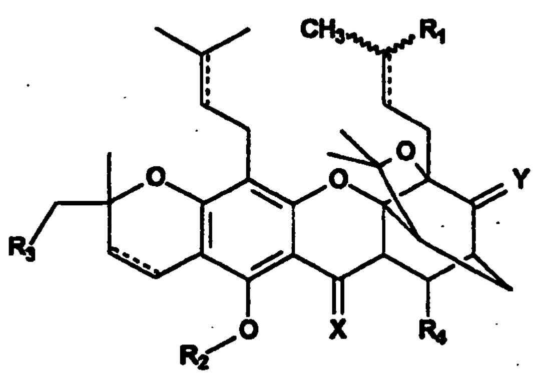

(II)

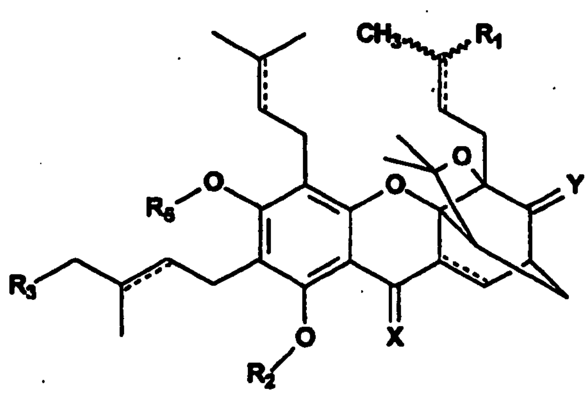

(III)

or pharmaceutically acceptable salts or prodrugs thereof, wherein:

the dotted lines are single bonds, double bonds or epoxy groups;
X together with the attached carbon is a carbonyl;
Y together with the attached carbon is a carbonyl;
$R_1$ is acyl ($R_aCO$), optionally substituted alkoxycarbonyl ($R_aOCO$), optionally substituted alkylthiocarbonyl, optionally substituted aminocarbonyl (carbamyl, $R_bR_cNCO$) or hydroxyaminocarbonyl, where $R_8$ is optionally substituted $C_{1-10}$ alkyl, optionally substituted aryl, N-succinimidyl or optionally substituted $C_{6-14}$ aryl($C_{1-10}$)alkyl group; $R_b$ and $R_c$ are independently hydrogen, optionally substituted heteroalkyl, optionally substituted $C_{1-10}$ alkyl, optionally substituted aryl, optionally substituted heteroaryl or optionally substituted $C_{6-14}$ aryl($C_{1-10}$)alkyl groups; or $R_b$ and $R_c$ may be taken together with the attached N to form an optionally substituted, saturated or partially saturated 5-7 membered heterocyclo group, including piperidine, morpholine and piperazine;
$R_2$ is hydrogen, optionally substituted alkyl, acyl ($R_aCO$), carbamyl ($R_bR_cNCO$) or sulfonyl ($R_dSO_2$), where $R_a$, $R_b$, and $R_c$ are defined above; $R_d$ is hydrogen, optionally substituted $C_{1-10}$ alkyl, optionally substituted aryl, or optionally substituted $C_{6-14}$ aryl($C_{1-10}$)alkyl groups;
$R_3$ is hydrogen or prenyl;
$R_4$ is hydrogen, halogen, hydroxy, optionally substituted alkyl, cycloalkyl, alkoxy, alkylthio or amino; and
$R_5$ is hydrogen, optionally substituted alkyl or acyl ($R_aCO$), carbamyl ($R_bR_cNCO$) or sulfonyl ($R_dSO_2$), where $R_a$, $R_b$, $R_c$ and $R_d$ are defined above;
for the manufacture of a medicament for treating cancer in an animal;

wherein said prodrug is:

a) an ester of a hydroxy containing compound obtained by condensation with a $C_{1-4}$ carboxylic acid, $C_{3-6}$ dioic acid, or anhydride thereof; or
b) an imine of an amino containing compound obtained by condensation with a $C_{1-4}$ aldehyde or ketone.

Preferred compounds falling within the scope of Formula I include compounds wherein $R_1$ is acetyl, propionyl, methoxycarbonyl, ethoxycarbonyl, methylthiocarbonyl, ethylthiocarbonyl, butylthiocarbonyl, dimethylcarbamyl, diethylcarbamoyl, 1-piperidinylcarbonyl, N-methyl-N'-piperazinylcarbonyl, 2-(dimethylamino)-ethylcarbamyl or N-morpholinylcatbonyl; $R_2$ is hydrogen, formyl, acetyl, dimethylcarbamyl, diethylcarbamyl, 2-(dimethylamino)ethylcarbamyl, 1-piperidinylcarbonyl, N-methyl-N'-piperazinylcarbonyl, N-morpholinyl-carbonyl, methylsulfonyl, ethylsulfonyl, phenylsulfonyl, methyl, ethyl, 2-piperidinylethyl, 2-morpholinylethyl, 2-(dimethylamino)ethyl, or 2-(diethylamino)ethyl; X and Y is O; $R_3$ is prenyl; and the dotted lines are double bonds or an epoxy group. If the double bond is present at C27-28, it is preferred that it has the Z configuration.

Preferred compounds falling within the scope of Formula II include compounds wherein $R_1$ is acetyl, propionyl, methoxycarbonyl, ethoxycarbonyl, methylthiocarbonyl, ethylthiocarbonyl, butylthiocarbonyl, dimethylcarbamyl, diethylcarbamyl, N-piperidinylcarbonyl, N-methyl-N'-piperazinylcarbonyl, 2-(dimethylamino)ethylcarboxy or N-morpholinylcarbonyl; $R_2$ is hydrogen, formyl, acetyl, dimethylcarbamyl, diethylcarbamyl, 2-(dimethylamino)ethylcarbamyl, 1-piperidinylcarbonyl, N-methyl-N'-piperazinylcarbonyl, N-morpholinylcarbonyl, methylsulfonyl, ethylsulfonyl, phenylsulfonyl, methyl, ethyl, 2-piperidinylethyl, 2-morpholinylethyl, 2-(dimethylamino)ethyl, or 2-(diethylamino)ethyl; and $R_4$ is methyl, ethyl, phenyl, chloro, bromo, hydroxy, hydrogen, methoxy, ethoxy, methylthio, ethylthio, butylthio, dimethylamino, diethylamino, piperidinyl, pyrrolidinyl, imidazolyl, pyrazolyl, N-methylpiperazinyl, 2-(dimethylamino)ethylamino or morpholinyl; X and Y is O; $R_3$ is prenyl; and the dotted lines are double bonds. If the double bond is present at C27-28, it is preferred that it has the Z configuration.

Preferred compounds falling within the scope of Formula III include compounds wherein $R_1$ is acetyl, propionyl, methoxycarbonyl, ethoxycarbonyl, methylthiocarbonyl, ethylthiocarbonyl, butylthiocarbonyl, dimethylcarbamyl, diethylcarbamyl, N-piperidinylcarbonyl, N-methyl-N'-piperazinylcarbonyl, 2-(dimethylamino)ethylcarbamyl or N-morpholinylcarbonyl; $R_2$ is hydrogen, formyl, acetyl, dimethylcarbamyl, diethylcarbamyl, 2-(dimethylamino)ethylcarbamyl, 1-piperidinylcarbonyl, N-methyl-N'-piperazinylcarbonyl, N-morpholinylcarbonyl, methylsulfonyl, ethylsulfonyl, phenylsulfonyl, methyl, ethyl, 2-piperidinylethyl, 2-morpholinylethyl, 2-(dimethylamino)ethyl, or 2-(diethyhvnino)ethyl; $R_5$ is hydrogen, formyl, acetyl, dimethylcarbamyl, diethylcarbamyl, 2-(dimethylamino)ethylcarbamyl, 1-piperidinylcarbonyl, N-methyl-N'-piperazinylcarbonyl, N-morpholinylcarbonyl, methylsulfonyl, ethylsulfonyl, phenylsulfonyl, methyl, ethyl, 2-piperidinylethyl, 2-morpholinylethyl, 2-(dimethylamino)ethyl, or 2-(diethylamino)ethyl; X and Y is O; R, is prenyl; and the dotted lines are double bonds. If the double bond is present at C27-28, it is preferred that it has the Z configuration.

Exemplary preferred compounds that may be employed for use according to the invention include, without limitation:

Methyl gambogate;
9,10-Dihydrogambogyl (4-methylpiperazine);
9,10-Dihydrogambogyl (2-dimethylaminoethylamine);
Gambogyl diethylamine;
Gambogyl dimethylamine;
Gambogyl amine;
Gambogyl hydroxyamine;
Gambogyl piperidine;
6-Methoxy-gambogyl piperidine;
Gambogyl morpholine;
Gambogyl (2-dimethylaminoethylamine);
10-Morpholinyl-gambogyl morpholine;
10-Morpholinyl-gambogyl piperidine;
10-(4-Methylpiperazinyl)-gambogyl piperidine;
10-(4-Methylpiperazinyl)-gambogyl morpholine;
10-Piperidinyl-gambogyl piperidine;
10-(4-Methylpiperazinyl)-gambogyl (4-methylpiperazine);
Gambogyl (4-methylpiperazine);
Methyl-6-methoxy-gambogate;
Methyl-10-Morpholinyl-gambogate;
N-(2-Gambogylamidoethyl)biotinamide;
Gambogyl (2-morpholinylethylamine);
Gambogyl (4-(2-pyridyl)piperazine);
10-(4-(2-Pyridyl)piperazinyl)gambogyl (4-(2-pyridyl)piperazine);
N-Hydroxysuccinimidyl gambogate;
8-(Gambogylamido)octanoic acid;
6-(Gambogylanido)hexanoic acid;
12-(Gambogylamido)dodecanoic acid;
N-Hydroxysuccinimidyl-8-(gambogylamido)octanoate;
N-Hydroxysuccinimidyl-6-(gambogylamido)hexanoate;
N-Hydroxysuccinimidyl-12-(gambogylamido)dodecanoate;
10-Methoxy-gambogyl piperidine;
Gambogyl (4-(2-pyrimidyl)piperazine);
Gambogyl (bis(2-pyridylmethyl)amine);
Gambogyl (N-(3-pyridyl)-N-(2-hydroxybenzyl)amine);
Gambogyl (4-benzylpiperazine);
Gambogyl (4-(3,4-methylenedioxybenzyl)piperazine);
Gambogyl (N-methyl-5-(methylamino)-3-oxapentylamine);
Gambogyl (N-methyl-8-(methylamino)-3,6-dioxaoctylamine);
Gambogyl (N-ethyl-2-(ethylamino)ethylamine);
Gambogyl (4-isopropylpiperazine);
Gambogyl (4-cyclopentylpiperazine);
Gambogyl (N-(2-oxo-2-ethoxyethyl)-(2-pyridyl)methylamine);

Gambogyl (2,5-dimethylpiperazine);
Gambogyl (3,5-dimethylpiperazine);
Gambogyl (4-(4-acetylphenyl)piperazine);
Gambogyl (4-ethoxycarbonylpiperazine);
Gambogyl (4-(2-oxo-2-pyrrolidylethyl)piperazine);
Gambogyl (4-(2-hydroxyethyl)piperazine);
Gambogyl (N-methyl-2-(methylamino)ethylamine);
Gambogyl (N-methyl-2-(benzylamino)ethylamine);
Gambogyl (N-methyl-(6-methyl-2-pyridyl)methylamine);
Gambogyl (N-ethyl-2-(2-pyridyl)ethylamine);
Gambogyl (N-methyl-(2-pyridyl)methylamine);
Gambogyl (N-methyl-4-(3-pyridyl)butylamine);
Gambogyl (bis(3-pyridylmethyl)amine);
Gambogyl (2,4-dimethyl-2-imidazoline);
Gambogyl (4-methyl-homopiperazine);
Gambogyl (4-(5-hydroxy-3-oxapentyl)piperazine);
Gambogyl (3-dimethylaminopyrrolidine);
Gambogyl ((2-furanyl)methylamine);
Gambogyl (2-hydroxy-1-methyl-2-phenylethylamine);
Gambogyl (3,4,5-trimethoxybenzylamine);
Gambogyl (2-(2-methoxyphenyl)ethylamine);
Gambogyl (2-methoxybenzylamine);
Gambogyl (3,4-methylenedioxybenzylamine);
Gambogyl (2-(2,5-dimethoxyphenyl)ethylamine);
Gambogyl (2-(3-methoxyphenyl)ethylamine);
Gambogyl (3-(piperidinyl)propylamine);
Gambogyl (2-(Piperidinyl)ethylamine);
Gambogyl (3,4-dimethoxybenzylamine);
Gambogyl ((2-tetrahydrofuranyl)methylamine);
Gambogyl ((N-ethyl-2-pyrrolidinyl)methylamine);
Gambogyl (2-diethylaminoethylamine);
Gambogyl (2,2-dimethyl-3-dimethylaminopropylamine);
Gambogyl ((N-ethoxycarbonyl-4-piperidinyl)amine);
Gambogyl (2-carbamylpyrrolidine);
Gambogyl (3-(homopiperidinyl)propylamine);
Gambogyl ((N-benzyl-4-piperidinyl)amine);
Gambogyl (2-(4-methoxyphenyl)ethylamine);
Gambogyl (4-oxa-hex-5-enylamine);
Ganbogyl (6-hydroxyhexylamine);
Gambogyl (2-(3,5-dimethoxyphenyl)ethylamine);
Gambogyl (3,5-dimethoxybenzylamine); and
Gambogyl (2-carbamyl-2-(4-hydroxyphenyl)ethylamine).

The positions in gambogic acid are numbered according to Asano, J., et al., Phytochemistry 41:815-820 (1996), and Lin, L.-J., et al., Magn. Reson. Chem. 31:340-347 (1993).

The present invention is also directed to novel compounds of Formulae I and II. Exemplary preferred compounds of Formulae I and II include, without limitation:

9,10-Dihydrogambogyl (4-methylpiperazine);
9,10-Dihydrogambogyl (2-(dimethylamino)ethylamine);
Gambogyl diethylamine;
Gambogyl dimethylamine;
Gambogyl amine;
Gambogyl hydroxyamine;
Gambogyl piperidine;
6-Methoxy-gambogyl piperidine;
Gambogyl morpholine;
Gambogyl 2-(dimethylamino)ethylamine;

10-Moipholinyl-gambogyl morpholine;
10-Morpholinyl-gambogyl piperidine;
10-(4-Methylpiperazinyl)-gambogyl piperidine;
10-(4-Methylpiperazinyl)-gambogyl morpholine;
10-Piperidinyl-gambogyl piperidine;
10-(4-Methylpiperazinyl)-gambogyl (4-methylpiperazine);
Gambogyl (4-methylpiperazine);
Methyl-10-Morpholinyl-gambogate;
N-(2-Gambogylamido-ethyl)biotinamide;
Gambogyl (2-(4-morpholinyl)ethylamine);
Gambogyl (4-(2-pyridyl)piperazine);
10-(4-(2-Pyridyl)piperazinyl)gambogyl (4-(2-pyridyl)piperazine);
N-Hydroxysuccinimidyl gambogate;
8-(Gambogylamido)octanoic acid;
6-(Gambogylamido)hexanoic acid;
12-(Gambogylamido)dodecanoic acid;
N-Hydroxysuccinimidyl-8-(gambogylamido)ctanoate;
N-Hydroxysuccinimidyl-6-(gambogylamido)hexanoate;
N-Hydroxysuccinimidyl-12-(gambogylamido)dodecanoate;
10-Methoxy-gambogyl piperidine;
Gambogyl (4-(2-pyrimidyl)piperazine);
Gambogyl (bis(2-pyridylmethyl)amine);
Gambogyl (N-(3-pyridyl)-N-(2-hydroxybenzyl)amine);
Gambogyl (4-benzylpiperazine);
Gambogyl (4-(3,4-methylenedioxybenzyl)piperazine);
Gambogyl (N-methyl-5-(methylamino)-3-oxapentylamine);
Gambogyl (N-methyl-8-(methylamino)-3,6-dioxaoctylamine);
Gambogyl (N-ethyl-2-(ethylamino)ethylamine);
Gambogyl (4-isopropylpiperazine);
Gambogyl (4-cyclopentylpiperazine);
Gambogyl (N-(2-oxo-2-ethoxyethyl)-(2-pyridyl)methylamme);
Gambogyl (2,5-dimethylpiperazine);
Gambogyl (3,5-dimethylpiperazine);
Gambogyl (4-(4-acetylphenyl)piperazine);
Gambogyl (4-ethoxycarbonylpiperazine);
Gambogyl (4-(2-oxo-2-pyrrolidylethyl)piperazine);
Gambogyl (4-(2-hydroxyethyl)piperazine);
Gambogyl (N-methyl-2-(methylamino)ethylamine);
Gambogyl (N-methyl-2-(benzylamino)ethylamine);
Gambogyl (N-methyl-(6-methyl-2-pyridyl)methylamine);
Gambogyl (N-ethyl-2-(2-pyridyl)ethylamine);
Gambogyl (N-methyl-(2-pyridyl)methylamine);
Gambogyl (N-methyl-4-(3-pyridyl)butylamine);
Gambogyl (bis(3-pyridylmethyl)amine);
Gambogyl (2,4-dimethyl-2-imidazoline);
Gambogyl (4-methyl-homopiperazine);
Gambogyl (4-(5-hydroxy-3-oxapentyl)piperazine);
Gambogyl (3-dimethylaminopyrrolidine);
Gambogyl ((2-furanyl)methylamine);
Gambogyl (2-hydroxy-1-methyl-2-phenylethylamine);
Gambogyl (3,4,5-trimethoxybenzylamine);
Gambogyl (2-(2-methoxyphenyl)ethylamine);
Gambogyl (2-methoxybenzylamine);
Gambogyl (3,4-methylenedioxybenzylamine);
Gambogyl (2-(2,5-dimethoxyphenyl)ethylamine);
Gambogyl (2-(3-methoxyphenyl)ethylamine);
Gambogyl (3-(piperidinyl)propylamine);
Gambogyl (2-(piperidinyl)ethylamine);

Gambogyl (3,4-dimethoxybenzylamine);
Gambogyl ((2-tetrahydrofuranyl)methylamine);
Gambogyl ((N-ethyl-2-pyrrolidinyl)methylamine);
Gambogyl (2-diethylaminoethylamine);
Gambogyl (2,2-dimethyl-3-dimethylaminopropylamine);
Gambogyl ((N-ethoxycarbonyl-4-piperidinyl)amine);
Gambogyl (2-carbamylpyrrolidine);
Gambogyl (3-(homopiperidinyl)propylamine);
Gambogyl ((N-benzyl-4-piperidinyl)amine);
Gambogyl (2-(4-methoxyphenyl)ethylamine);
Gambogyl (4-oxa-hex-5-enylamine);
Ganbogyl (6-hydroxyhexylamine);
Gambogyl (2-(3,5-dimethoxyphenyl)ethylamine);
Gambogyl (3,5-dimethoxybenzylamine); and
Gambogyl (2-carbamyl-2-(4-hydroxyphenyl)ethylamine).

Useful alkyl groups include straight-chained and branched $C_{1-10}$ alkyl groups, more preferably $C_{1-}6$ alkyl groups. Typical $C_{1-10}$ alkyl groups include methyl, ethyl, propyl, isopropyl, butyl, *sec*-butyl, *tert*-butyl, 3-pentyl, hexyl and octyl groups, which may be optionally substituted.

Useful alkoxy groups include oxygen substituted by one of the $C_{1-10}$ alkyl groups mentioned above, which may be optionally substituted.

Useful alkylthio groups include sulphur substituted by one of the $C_{1-10}$ alkyl groups mentioned above, which may be optionally substituted. Also included are the sulfoxides and sulfones of such alkylthio groups.

Useful amino groups include $-NH_2$, $-NHR_{11}$, and $-NR_{11}R_{12}$, wherein $R_{11}$ and $R_{12}$ are $C_{1-10}$ alkyl or cycloalkyl groups, or $R_{11}$ and $R_{12}$ are combined with the N to form a ring structure, such as a piperidine, or $R_{11}$ and $R_{12}$ are combined with the N and another heteroatom to form an optionally substituted, saturated or partially saturated 5-7 membered heterocyclo group, such as a piperazine. The alkyl group may be optionally substituted.

Useful heteroatoms include N, O or S.

Optional substituents on the alkyl groups include one or more halo, hydroxy, carboxyl, alkoxycarbonyl, amino, nitro, cyano, $C_1$-$C_6$ acylamino, $C_1$-$C_6$ aminoacyl, $C_1$-$C_6$ acyloxy, $C_1$-$C_6$ alkoxy, aryloxy, alkylthio, $C_6$-$C_{10}$ aryl, $C_4$-$C_7$ cycloalkyl, $C_2$-$C_6$ alkenyl, $C_2$-$C_6$ alkynyl, $C_6$-$C_{10}$ aryl($C_2$-$C_6$)alkenyl, $C_6$-$C_{10}$ aryl($C_2$-$C_6$)alkynyl, saturated or partially saturated 5-7 membered heterocyclo group, or heteroaryl.

Optional substituents on the aryl, aralkyl and heteroaryl groups include one or more acyl, alkylenedioxy ($-OCH_2O-$), halo, $C_1$-$C_6$ haloalkyl, $C_6$-$C_{10}$ aryl, $C_4$-$C_7$ cycloalkyl, $C_1$-$C_6$ alkyl, $C_2$-$C_6$ alkenyl, $C_2$-$C_6$ alkynyl, $C_6$-$C_{10}$ aryl ($C_1$-$C_6$)alkyl, $C_6$-$C_{10}$ aryl($C_2$-$C_6$)alkenyl, $C_6$-$C_{10}$ aryl($C_2$-$C_6$)alkynyl, $C_1$-$C_6$ hydroxyalkyl, nitro, amino, ureido, cyano, $C_1$-$C_6$ acylamino, hydroxy, thiol, $C_1$-$C_6$ acyloxy, azido, $C_1$-$C_6$ alkoxy, or carboxy.

Useful heteroalkyl groups contain 1-10 carbon atoms and 1, 2 or 3 heteroatoms. Examples of heteroalkyl groups include $-CH_2CH_2OCH_2CH_3$, $-CH_2CH_2OCH_2CH_2OCH_2CH_3$, $-CH_2CH_2NHCH_3$, $-CH_2CH_2N(CH_2CH_3)_2$, $-CH_2CH_2OCH_2CH_2NHCH_3$, $-CH_2CH_2OCH_2CH_2OCH_2CH_2NHCH_3$, $-CH_2CH_2NHCH_2CH_3$, $-CH_2C(CH_3)_2CH_2N(CH_3)_2$ or $-CH_2$(N-ethylpyrrolidine), which may be optionally substituted.

Optional substituents on heteroalkyl groups include one or more halo, hydroxy, carboxyl, amino, nitro, cyano, alkyl, $C_1$-$C_6$ acylamino, $C_1$-$C_6$ aminoacyl, $C_1$-$C_6$ acyloxy, $C_1$-$C_6$ alkoxy, aryloxy, alkylthio, $C_6$-$C_{10}$ aryl, $C_4$-$C_7$ cycloalkyl, $C_2$-$C_6$ alkenyl, alkenoxy, $C_2$-$C_6$ alkynyl, $C_6$-$C_{10}$ aryl($C_2$-$C_6$)alkenyl, $C_6$-$C_{10}$ aryl($C_2$-$C_6$)alkynyl, saturated and unsaturated heterocyclic, or heteroaryl.

Useful aryl groups are $C_{6-14}$ aryl, especially $C_{6-10}$ aryl. Typical $C_{6-14}$ aryl groups include phenyl, naphthyl, phenanthrenyl, anthracenyl, indenyl, azulenyl, biphenyl, biphenylenyl and fluorenyl groups.

Useful cycloalkyl groups are $C_{3-8}$ cycloalkyl. Typical cycloalkyl groups include cyclopropyl, cyclobutyl, cyclopentyl, cyclohexyl and cycloheptyl.

Useful saturated or partially saturated carbocyclic groups are cycloalkyl groups as defined above, as well as cycloalkenyl groups, such as cyclopentenyl, cycloheptenyl and cyclooctenyl.

Useful halo or halogen groups include fluorine, chlorine, bromine and iodine.

Useful aralkyl groups include any of the above-mentioned $C_{1-10}$ alkyl groups substituted by any of the above-mentioned $C_6$-$_{14}$ aryl groups. Useful values include benzyl, phenethyl and naphthylmethyl.

Useful haloalkyl groups include $C_{1-10}$ alkyl groups substituted by one or more fluorine, chlorine, bromine or iodine atoms, e.g. fluoromethyl, difluoromethyl, trifluoromethyl, pentafluoroethyl, 1,1-difluoroethyl, chloromethyl, chlorofluoromethyl and trichloromethyl groups.

Useful acylamino groups are any $C_{1-6}$ acyl (alkanoyl) attached to an amino nitrogen, e.g. acetamido, propionamido, butanoylamido, pentanoylamido, hexanoylamido as well as aryl-substituted $C_{2-6}$ substituted acyl groups.

Useful acyloxy groups are any $C_{1-6}$ acyl (alkanoyl) attached to an oxy (-O-) group, e.g. formyloxy, acetoxy, propionoyloxy, butanoyloxy, pentanoyloxy, hexanoyloxy and the like.

Useful saturated or partially saturated 5-7 membered heterocyclo groups include tetrahydrofuranyl, pyranyl, piperidinyl, piperazinyl, pyrrolidinyl, imidazolidinyl, imidazolinyl, indolinyl, isoindolinyl, quinuclidinyl, morpholinyl, isochromanyl, chromanyl, pyrazolidinyl pyrazolinyl, tetronoyl and tetramoyl groups.

Optional substitutents on the 5-7 membered heterocyclo groups include one or more heteroaryl, heterocyclo, alkyl, aralkyl, cycloalkyl, alkoxycarbonyl, carbamyl, aryl or $C_1$-$C_6$ aminoacyl.

Useful heteroaryl groups include any one of the following: thienyl, benzo[b]thienyl, naphtho[2,3-b]thienyl, thianthrenyl, furanyl, pyranyl, isobenzofuranyl, chromenyl, xanthenyl, phenoxanthiinyl, 2*H*-pyrrolyl, pyrrolyl, imidazolyl, pyrazolyl, pyridyl, pyrazinyl, pyrimidinyl, pyridazinyl, indolizinyl, isoindolyl, 3*H*-indolyl, indolyl, indazolyl, purinyl, 4*H*-quinolizinyl, isoquinolyl, quinolyl, phthalzinyl, naphthyridinyl, quinozalinyl, cinnolinyl, pteridinyl, carbazolyl, β-carbolinyl, phenanthridinyl, acrindinyl, perimidinyl, phenanthrolinyl, phenazinyl, isothiazolyl, phenothiazinyl, isoxazolyl, furazanyl, phenoxazinyl, 1,4-dihydroquinoxaline-2,3-dione, 7-aminoisocoumarin, pyrido[1,2-a]pyrimidin-4-one, 1,2-benzoisoxazol-3-yl, benzimidazolyl, 2-oxindolyl and 2-oxobenzimidazolyl. Where the heteroaryl group contains a nitrogen atom in a ring, such nitrogen atom may be in the form of an N-oxide, e.g. a pyridyl N-oxide, pyrazinyl N-oxide, pyrimidinyl N-oxide and the like.

Optional substituents on the heteroaryl groups include one or more heteroaryl, heterocyclo, alkyl, aralkyl, cycloalkyl, alkoxycarbonyl, carbamyl, aryl and $C_1$-$C_6$ aminoacyl.

Certain compounds of the present invention may exist as stereoisomers including optical isomers. The invention includes all stereoisomers and both the racemic mixtures of such stereoisomers as well as the individual enantiomers that may be separated according to methods that are well known to those of ordinary skill in the art.

Examples of pharmaceutically acceptable addition salts include inorganic and organic acid addition salts such as hydrochloride, hydrobromide, phosphate, sulphate, citrate, lactate, tartrate, maleate, fumarate, mandelate and oxalate; and inorganic and organic base addition salts with bases such as sodium hydroxy, Tris(hydroxymethyl)aminomethane (TRIS, tromethane) and N-methyl-glucamine

The prodrugs of the compounds of the invention are: a) esters of hydroxy containing compounds obtained by condensation with a $C_{1-4}$ carboxylic acid, $C_{3-6}$ dioic acid or anhydride thereof, such as succinic and fumaric anhydrides according to methods known in the art; or b) imines of amino containing compounds obtained by condensation with a $C_{1-4}$ aldehyde or ketone according to methods known in the art.

The compounds of this invention may be prepared and purified using methods known to those skilled in the art, or the novel methods of this invention. Specifically, gambogic acid can be purified by 1) preparation of the pyridine salt of the crude extract from gamboge (resin from *Garcinia hanburyi* Hook) followed by repeated recrystallization of the salt in ethanol or 2) converting the salt to the free acid. Using this procedure, about 10% by weight of gambogic acid with purity >99% (HPLC) can be obtained from the crude extract. Gambogic acid and analogs of gambogic acid with Formula I-III also can be separated and purified from gamboge by repeated chromatography ($SiO_2$, hexane-EtOAc gradient) using a Combi Flash SG 100 separation system.

Derivatives of gambogic acid with Formula I can be prepared as illustrated by exemplary reactions in Schemes 1 and 2. Reaction of gambogic acid with methanol in the presence of DMAP and EDC produced the methyl ester of gambogic acid (Scheme 1). Reaction of gambogic acid with piperidine in the presence of DMAP and EDC produced the piperidinyl amide of gambogic acid (Scheme 2).

**Scheme 1**

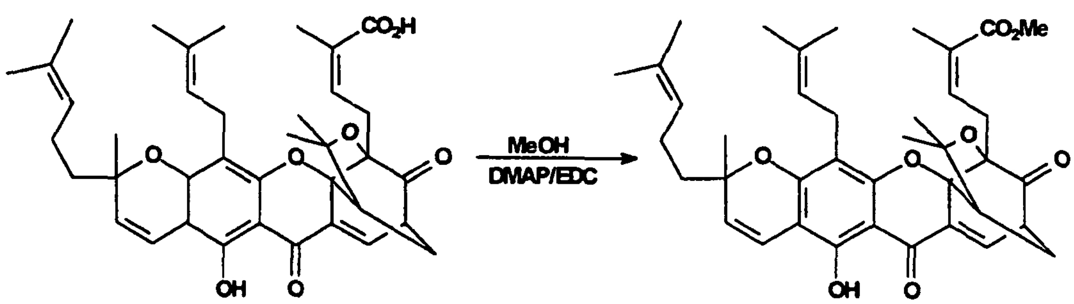

**Scheme 2**

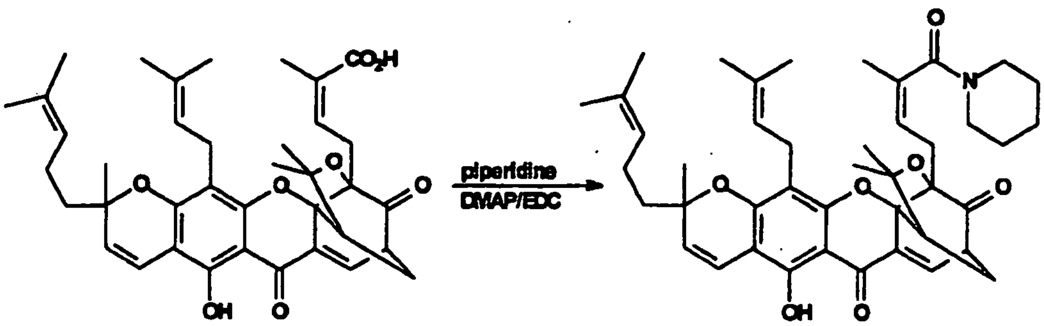


Derivatives of gambogic acid can also be prepared as illustrated by exemplary reactions in Schemes 3-5. Reaction of methyl gambogate with methyl iodide in the presence of a base, such as $K_2CO_3$, produced the methyl-6-methoxy-gambogate of Formula I (Scheme 3). Reaction of gambogic acid with acetic anhydride in pyridine produced 6-acetyl gambogic acid (Scheme 4). Reaction of gambogic acid with $H_2O_2$ under basic conditions produced 9,10-epoxygambogic acid (Scheme 5).

**Scheme 3**

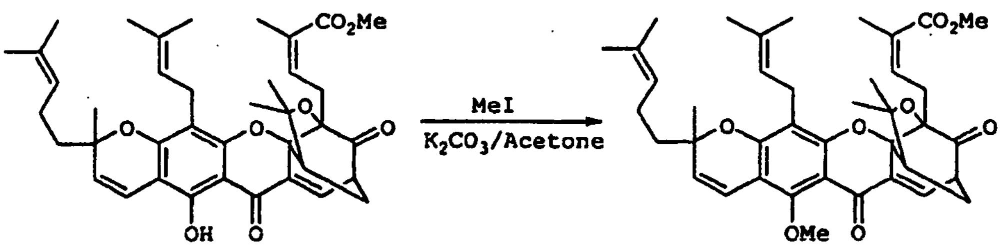


**Scheme 4**

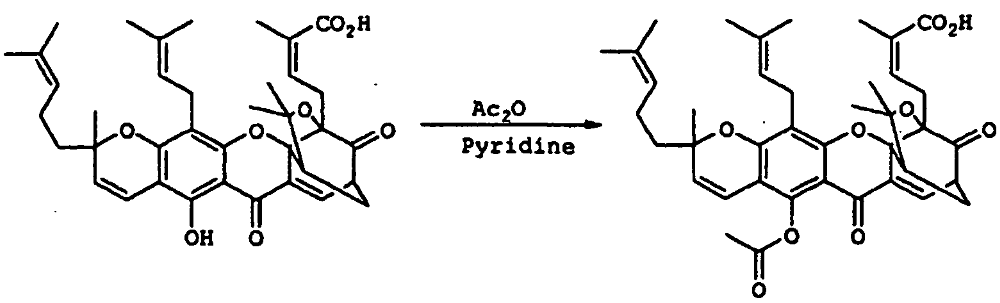

**Scheme 5**

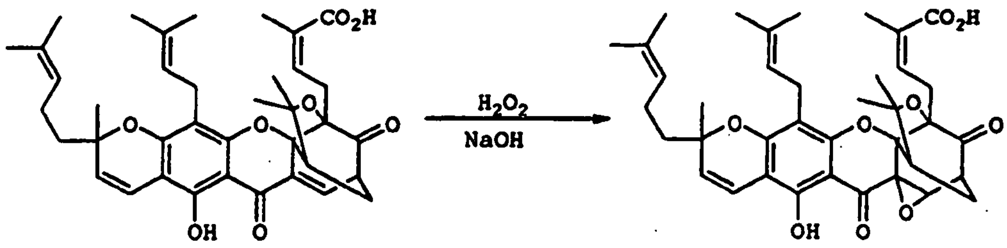


Derivatives of gambogic acid can also be prepared as illustrated by exemplary reactions in Schemes 6-10. Reaction of gambogyl piperidine with sodium methoxide produced the methoxy addition product of the amide (Scheme 6), which is a compound of Formula II. Similarly, reaction of gambogic acid with an amine, such as morpholine, with or without the presence of a base, such as $Et_3N$, produced the morpholine addition product of gambogic acid (Scheme 7). Reaction of the piperidine amide of gambogic acid with N-methylpiperazine produced the N-methylpiperazine addition product of the amide, (Scheme 8), which is a compound of Formula II. Reduction of gambogic acid by $NaBH_4$ gave 9,10-dihydro-12-hydroxygambogic acid, which may be oxidized by Dess-Martin reagent to give 9,10-dihydro-gambogic acid (Scheme 9). Alternatively, selective reduction of gambogic acid by L-selectride also produced 9,10-dihydro-gambogic acid (Scheme 9). Reaction of gambogic acid with an alkylcuprate, such as cyclohexylcuprate, resulted in the addition of the alkyl group to the 10-position, thereby producing 10-cyclohexyl-gambogic acid (Scheme 10).

**Scheme 6**

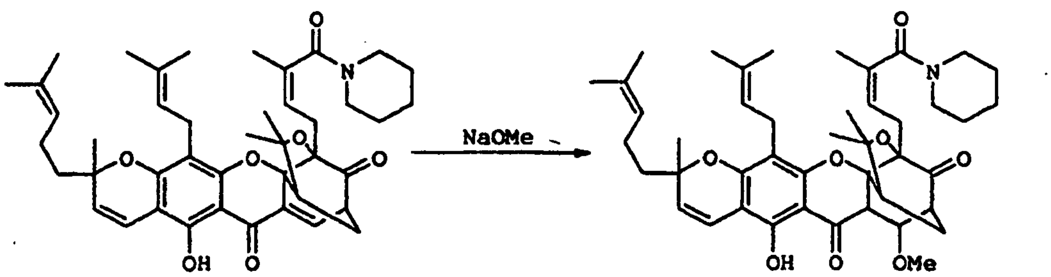


**Scheme 7**

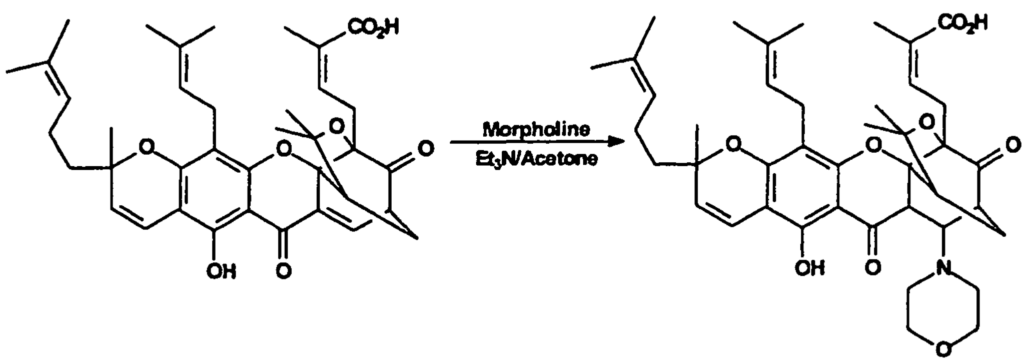

## Scheme 8

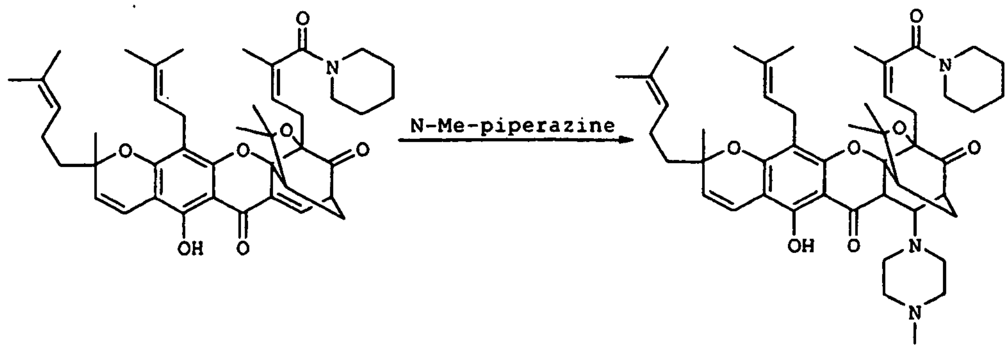


## Scheme 9

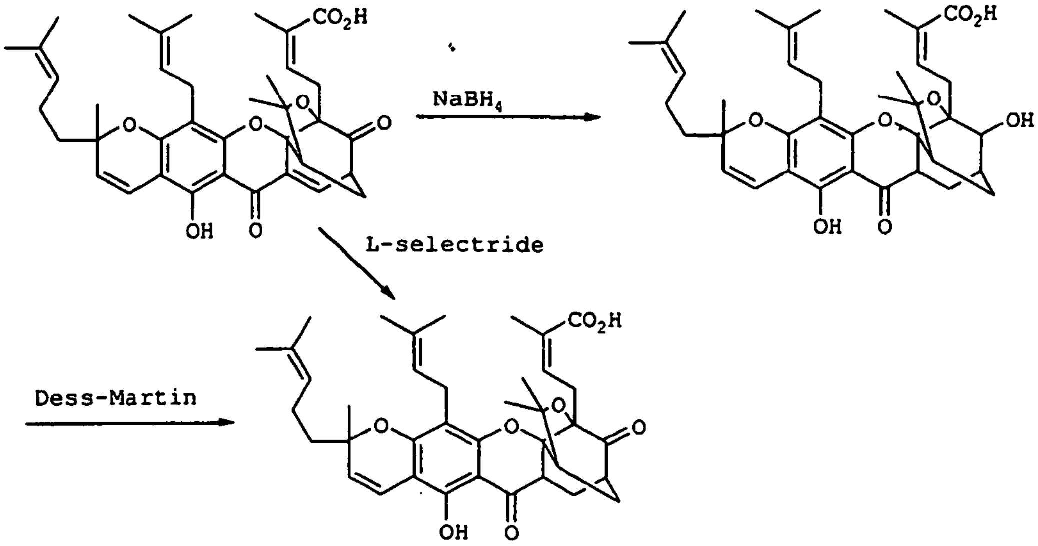


## Scheme 10

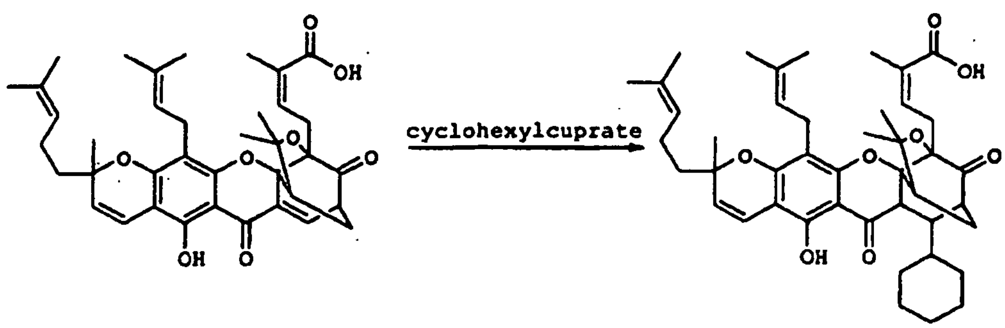


An important aspect of the present invention is the discovery that compounds having Formula I-III are activators of caspases and inducers of apoptosis. Therefore, these compounds are expected to be useful in a variety of clinical conditions in which there is uncontrolled cell growth and spread of abnormal cells, such as in the case of cancer.

Another important aspect of the present invention is the discovery that compounds having Formula I-III are potent and highly efficacious activators of caspases and inducers of apoptosis in drug resistant cancer cells, such as breast and prostate cancer cells, which enables these compounds to kill these drug resistant cancer cells. In comparison, most standard anti-cancer drugs are not effective in killing drug resistant cancer cells under the same conditions. Therefore, gambogic acid, its derivatives and analogs are expected to be useful for the treatment of drug resistant cancer in animals.

Disclosed is a therapeutic method useful to modulate in vivo apoptosis or *in vivo* neoplastic disease, comprising administering to a subject in need of such treatment an effective amount of a compound, or a pharmaceutically acceptable salt or prodrug of a compound of Formulae I-III, which functions as a caspase cascade activator and inducer of apoptosis.

Also disclosed is a therapeutic method comprising administering to an animal an effective amount of a compound, or a pharmaceutically acceptable salt or prodrug of said compound of Formulae I-III, wherein said therapeutic method is useful to treat cancer, which is a group of diseases characterized by the uncontrolled growth and spread of abnormal cells. Such diseases include, but are not limited to, Hodgkin's disease, non-Hodgkin's lymphomas, acute and chronic lymphocytic leukemias, multiple myeloma, neuroblastoma, breast carcinomas, ovarian carcinomas, lung carcinomas, Wilms' tumor, cervical carcinomas, testicular carcinomas, soft-tissue sarcomas, chronic lymphocytic leukemia, primary macroglobulinemia, bladder carcinomas, chronic granulocytic leukemia, primary brain carcinomas, malignant melanoma, small-cell lung carcinomas, stomach carcinomas, colon carcinomas, malignant pancreatic insulinoma, malignant carcinoid carcinomas, malignant melanomas, choriocarcinomas, mycosis fungoides, head and neck carcinomas, osteogenic sarcoma, pancreatic carcinomas, acute granulocytic leukemia, hairy cell leukemia, neuroblastoma, rhabdomyosarcoma, Kaposi's sarcoma, genitourinary carcinomas, thyroid carcinomas, esophageal carcinomas, malignant hypercalcemia, cervical hyperplasia, renal cell carcinomas, endometrial carcinomas, polycythemia vera, essential thrombocytosis, adrenal cortex carcinomas, skin cancer, and prostatic carcinomas.

In practicing the therapeutic methods, effective amounts of compositions containing therapeutically effective concentrations of the compounds formulated for oral, intravenous, local and topical application, for the treatment of neoplastic diseases and other diseases in which caspase cascade mediated physiological responses are implicated, are administered to an individual exhibiting the symptoms of one or more of these disorders. The amounts are effective to ameliorate or eliminate one or more symptoms of the disorders. An effective amount of a compound for treating a particular disease is an amount that is sufficient to ameliorate, or in some manner reduce, the symptoms associated with the disease. Such amount may be administered as a single dosage or may be administered according to a regimen, whereby it is effective. The amount may cure the disease but, typically, is administered in order to ameliorate the disease. Typically, repeated administration is required to achieve the desired amelioration of symptoms.

In another embodiment, a pharmaceutical composition comprising a compound, or a pharmaceutically acceptable salt of said compound of Formula I or II, which functions as a caspase cascade activator and inducer of apoptosis in combination with a pharmaceutically acceptable vehicle is provided.

Another embodiment of the present invention is directed to a composition effective to inhibit neoplasia comprising a compound, or a pharmaceutically acceptable salt or prodrug of said compound of Formula I or II, which functions as a caspase cascade activator and inducer of apoptosis, in combination with at least one known cancer chemotherapeutic agent, or a pharmaceutically acceptable salt of said agent. Examples of known anti-cancer agents which can be used for combination therapy include, but are not limited to, alkylating agents such as busulfan, cis-platin, mitomycin C, and carboplatin; antimitotic agents such as colchicine, vinblastine, paclitaxel, and docetaxel; topo I inhibitors such as camptothecin and topotecan; topo II inhibitors such as doxorubicin and etoposide; RNA/DNA antimetabolites such as 5-azacytidine, 5-fluorouracil and methotrexate; DNA antimetabolites such as 5-fluoro-2'-deoxy-uridine, ara-C, hydroxyurea and thioguanine; antibodies such as Herceptin and Rituxan. Other known anti-cancer agents which can be used for combination therapy include melphalan, chlorambucil, cyclophosamide, ifosfamide, vincristine, mitoguazone, epirubicin, aclarubicin, bleomycin, mitoxantrone, elliptinium, fludarabine, octreotide, retinoic acid, tamoxifen and alanosine.

In practicing the methods disclosed herein, the compound of the invention may be administered together with at least one known chemotherapeutic agent as part of a unitary pharmaceutical composition. Alternatively, the compound of the invention may be administered apart from the at least one known cancer chemotherapeutic agent. In this embodiment, the compound of the invention and the at least one known cancer chemotherapeutic agent are administered substantially simultaneously, i.e. the compounds are administered at the same time or one after the other, so long as the compounds reach therapeutic levels in the blood.

Another embodiment of the present invention is directed to a composition effective to inhibit neoplasia comprising a bioconjugate of said compound of Formula I or II, which functions as a caspase cascade activator and inducer of apoptosis, in bioconjugation with at least one known therapeutically useful antibody, such as Herceptin or Rituxan, growth factors such as DGF, NGF, cytokines such as IL-2, IL-4, or any molecule that binds to the cell surface. The antibodies and other molecules will deliver the compound of Formula I or II to its target and make it an effective anticancer agent. The bioconjugate could also enhance the anticancer effect of therapeutically useful antibodies, such as Herceptin or Rituxan.

Similarly, another embodiment of the present invention is directed to a composition effective to inhibit neoplasia comprising a compound, or a pharmaceutically acceptable salt or prodrug of said compound of Formula I or II, which functions as a caspase cascade activator and inducer of apoptosis, in combination with radiation therapy. In this embodiment, the compound of the invention may be administered at the same time as the radiation therapy is administered or at a different time.

Yet another embodiment of the present invention is directed to a composition effective for post-surgical treatment of cancer, comprising a compound, or a pharmaceutically acceptable salt or prodrug of said compound of Formula I or II, which functions as a caspase cascade activator and inducer of apoptosis. Also disclosed is a method of treating cancer by surgically removing the cancer and then treating the animal with one of the pharmaceutical compositions described herein.

A wide range of immune mechanisms operate rapidly following exposure to an infectious agent. Depending on the type of infection, rapid clonal expansion of the T and B lymphocytes occurs to combat the infection. The elimination of the effector cells following an infection is one of the major mechanisms maintaining immune homeostasis. This deletion of reactive cells has been shown to be regulated by a phenomenon known as apoptosis. Autoimmune diseases have been recently identified to occur as a consequence of deregulated cell death. In certain autoimmune diseases, the immune system directs its powerful cytotoxic effector mechanisms against specialized cells such as oligodendrocytes in multiple sclerosis, the beta cells of the pancreas in diabetes mellitus, and thyrocytes in Hashimoto's thyroiditis (Ohsako, S. & Elkon, K.B., Cell Death Differ 6(1):13-21 (1999)). Mutations of the gene encoding the lymphocyte apoptosis receptor Fas/APO-1/CD95 are reportedly associated with defective lymphocyte apoptosis and autoimmune lymphoproliferative syndrome (ALPS), which is characterized by chronic, histologically benign splenomegaly and generalized lymphadenopathy, hypergammaglobulinemia, and autoantibody formation. (Infante, A.J., et al., J Pediatr. 133(5):629-633 (1998) and Vaishnaw, A.K., et al., J Clin. Invest. 103(3):355-363 (1999)). It was reported that overexpression of Bcl-2, which is a member of the Bcl-2 gene family of programmed cell death regulators with anti-apoptotic activity in developing B cells of transgenic mice, in the presence of T cell dependent costimulatory signals, results in the generation of a modified B cell repertoire and in the production of pathogenic autoantibodies (Lopez-Hoyos, M., et al., Int. J. Mol. Med. 1(2): 475-483 (1998)). It is therefore evident that many types of autoimmune diseases are caused by defects of the apoptotic process. One treatment strategy for autoimmune diseases is to turn on apoptosis in the lymphocytes that are causing the autoimmune disease (O'Reilly, L.A. & Strasser, A., Inflamm Res 48(1):5-21 (1999)).

Fas-Fas ligand (FasL) interaction is known to be required for the maintenance of immune homeostasis. Experimental autoimmune thyroiditis (EAT), characterized by autoreactive T and B cell responses and a marked lymphocytic infiltration of the thyroid, is a good model for the study of the therapeutic effects of FasL. Batteux, F., et al., J Immunol. 162(1):603-608 (1999)) reported that the direct injection of DNA expression vectors encoding FasL into the inflammed thyroid inhibited the development of lymphocytic infiltration of the thyroid. In addition, the death of infiltrating T cells was observed. These results show that FasL expression on thyrocytes may have a curative effect on ongoing EAT by inducing death of pathogenic autoreactive infiltrating T lymphocytes.

Bisindolylmaleimide VIII is known to potentiate Fas-mediated apoptosis in human astrocytoma 1321N1 cells and in Molt-4T cells, and both of which were resistant to apoptosis induced by anti-Fas antibody in the absence of bisindolylmaleimide VIII. Potentiation of Fas-mediated apoptosis by bisindolylmaleimide VIII was reported to be selective for activated, rather than non-activated, T cells, and was Fas-dependent. Zhou T. et al. (Nat Med 5(1):42-8 (1999)) reported that administration of bisindolylmaleimide VIII to rats during autoantigen stimulation prevented the development of symptoms of T cell-mediated autoimmune diseases in two models: the Lewis rat model of experimental allergic encephalitis and the Lewis adjuvant arthritis model. Therefore, the application of a Fas-dependent apoptosis enhancer such as bisindolylmaleimide VIII may be therapeutically useful for the more effective elimination of detrimental cells and inhibition of T cell-mediated autoimmune diseases. Therefore, an effective amount of a compound, or a pharmaceutically acceptable salt or prodrug of the compound of Formulae I-III, which functions as a caspase cascade activator and inducer of apoptosis, should be an effective treatment for autoimmune disease.

Psoriasis is a chronic skin disease which is characterized by scaly red patches. Psoralen plus ultraviolet A (PUVA) is a widely used and effective treatment for psoriasis vulgaris. Coven, et al., Photodermatol Photoimmunol Photomed 15(1):22-7 (1999), reported that lymphocytes treated with psoralen 8-MOP or TMP plus UVA displayed DNA degradation patterns typical of apoptotic cell death. Ozawa, et al., J. Exp. Med 189(4):711-718 (1999) reported that induction of T cell apoptosis could be the main mechanism by which 312-nm UVB resolves psoriasis skin lesions. Low doses of methotrexate may be used to treat psoriasis to restore a clinically normal skin. Heenen, et al., Arch. Dermatol. Res. 290(5):240-245 (1998), reported that low doses of methotrexate may induce apoptosis and this mode of action could explain the reduction in epidermal hyperplasia during treatment of psoriasis with methotrexate. Therefore, an effective amount of a compound, or a pharmaceutically acceptable salt or prodrug of the compound of Formulae I-III, which functions as a caspase cascade activator and inducer of apoptosis, should be an effective treatment for psoriasis.

Synovial cell hyperplasia is a characteristic of patients with rheumatoid arthritis (RA). Excessive proliferation of RA synovial cells as well as defects in synovial cell death may be responsible for synovial cell hyperplasia. Wakisaka,

et al., Clin. Exp. Immunol. 114(1):119-28 (1998), found that although RA synovial cells could die via apoptosis through Fas/FasL pathway, apoptosis of synovial cells was inhibited by proinflammatory cytokines present within the synovium. This suggested that inhibition of apoptosis by the proinflammatory cytokines may contribute to the outgrowth of synovial cells, and lead to pannus formation and the destruction of joints in patients with RA. Therefore, an effective amount of a compound, or a pharmaceutically acceptable salt or prodrug of the compound of Formulae I-III, which functions as a caspase cascade activator and inducer of apoptosis, should be an effective treatment for RA.

There has been an accumulation of convincing evidence that apoptosis plays a major role in promoting resolution of the acute inflammatory response. Neutrophils are constitutively programmed to undergo apoptosis, thus limiting their pro-inflammatory potential and leading to rapid, specific, and non-phlogistic recognition by macrophages and semi-professional phagocytes (Savill, J., J. Leukoc. Biol. 61(4):375-80 (1997)). Boirivant, et al., Gastroenterology 116(3): 557-65 (1999), reported that lamina propria T cells isolated from areas of inflammation in Crohn's disease, ulcerative colitis, and other inflammatory states manifest decreased CD2 pathway-induced apoptosis. Moreover, studies of cells from inflamed Crohn's disease tissue indicate that this defect is accompanied by elevated Bcl-2 levels. Therefore, an effective amount of a compound, or a pharmaceutically acceptable salt or prodrug of the compound of Formulae I-III, which functions as a caspase cascade activator and inducer of apoptosis, should be an effective treatment for inflammation.

Compositions within the scope of this invention include all compositions wherein the compounds of the present invention are contained in an amount which is effective to achieve its intended purpose. While individual needs vary, determination of optimal ranges of effective amounts of each component is within the skill of the art. Typically, the compounds may be administered to mammals, e.g. humans, orally at a dose of 0.0025 to 50 mg/kg, or an equivalent amount of the pharmaceutically acceptable salt thereof, per day, per kg of body weight of the mammal being treated for apoptosis-mediated disorders. Preferably, about 0.01 to about 10 mg/kg is orally administered to treat or prevent such disorders. For intramuscular injection, the dose is generally about one-half of the oral dose. For example, a suitable intramuscular dose would be about 0.0025 to about 25 mg/kg, and most preferably, from about 0.01 to about 5 mg/kg. If a known cancer chemotherapeutic agent is also administered, it is administered in an amount with is effective to achieve its intended purpose. The amounts of such known cancer chemotherapeutic agents effective for cancer are well known to those of ordinary skill in the art.

The unit oral dose may comprise from about 0.01 to about 50 mg, preferably about 0.1 to about 10 mg of the compound of the invention. The unit dose may be administered one or more times daily as one or more tablets each containing from about 0.1 to about 10, conveniently about 0.25 to 50 mg of the compound or its solvates.

In a topical formulation, the compound may be present at a concentration of about 0.01 to 100 mg per gram of carrier.

In addition to administering the compound as a raw chemical, the compounds of the invention may be administered as part of a pharmaceutical preparation containing suitable pharmaceutically acceptable carriers comprising excipients and auxiliaries which facilitate processing of the compounds into preparations which can be used pharmaceutically. Preferably, the preparations, particularly those preparations which can be administered orally and which can be used for the preferred type of administration, such as tablets, dragees, and capsules, and also preparations which can be administered rectally, such as suppositories, as well as suitable solutions for administration by injection or orally, contain from about 0.01 to 99 percent, preferably from about 0.25 to 75 percent of active compound(s), together with the excipient.

Also included within the scope of the present invention are the non-toxic pharmaceutically acceptable salts of the compounds of the present invention. Acid addition salts are formed by mixing a solution of the particular apoptosis inducers of the present invention with a solution of a pharmaceutically acceptable non-toxic acid such as hydrochloric acid, fumaric acid, maleic acid, succinic acid, acetic acid, citric acid, tartaric acid, carbonic acid, phosphoric acid, oxalic acid, and the like. Basic salts are formed by mixing a solution of the particular apoptosis inducers of the present invention with a solution of a pharmaceutically acceptable non-toxic base such as sodium hydroxide, potassium hydroxide, choline hydroxide, sodium carbonate, Tris, N-methyl-glucamine and the like.

The pharmaceutical compositions of the invention may be administered to any animal which may experience the beneficial effects of the compounds of the invention. Foremost among such animals are mammals, e.g., humans and veterinary animals, although the invention is not intended to be so limited.

The pharmaceutical compositions of the present invention may be administered by any means that achieve their intended purpose. For example, administration may be by parenteral, subcutaneous, intravenous, intramuscular, intraperitoneal, transdermal, buccal, intrathecal, intracranial, intranasal or topical routes. Alternatively, or concurrently, administration may be by the oral route. The dosage administered will be dependent upon the age, health, and weight of the recipient, kind of concurrent treatment, if any, frequency of treatment, and the nature of the effect desired.

The pharmaceutical preparations of the present invention are manufactured in a manner which is itself known, for example, by means of conventional mixing, granulating, dragee-making, dissolving, or lyophilizing processes. Thus, pharmaceutical preparations for oral use may be obtained by combining the active compounds with solid excipients,

optionally grinding the resulting mixture and processing the mixture of granules, after adding suitable auxiliaries, if desired or necessary, to obtain tablets or dragee cores.

Suitable excipients are, in particular, fillers such as saccharides, for example lactose or sucrose, mannitol or sorbitol, cellulose preparations and/or calcium phosphates, for example tricalcium phosphate or calcium hydrogen phosphate, as well as binders such as starch paste, using, for example, maize starch, wheat starch, rice starch, potato starch, gelatin, tragacanth, methyl cellulose, hydroxypropylmethylcellulose, sodium carboxymethylcellulose, and/or polyvinyl pyrrolidone. If desired, disintegrating agents may be added such as the above-mentioned starches and also carboxymethyl-starch, crosslinked polyvinyl pyrrolidone, agar, or alginic acid or a salt thereof, such as sodium alginate. Auxiliaries are, above all, flow-regulating agents and lubricants, for example, silica, talc, stearic acid or salts thereof, such as magnesium stearate or calcium stearate, and/or polyethylene glycol. Dragee cores are provided with suitable coatings which, if desired, are resistant to gastric juices. For this purpose, concentrated saccharide solutions may be used, which may optionally contain gum arabic, talc, polyvinyl pyrrolidone, polyethylene glycol and/or titanium dioxide, lacquer solutions and suitable organic solvents or solvent mixtures. In order to produce coatings resistant to gastric juices, solutions of suitable cellulose preparations such as acetylcellulose phthalate or hydroxypropymethyl-cellulose phthalate, are used. Dye stuffs or pigments may be added to the tablets or dragee coatings, for example, for identification or in order to characterize combinations of active compound doses.

Other pharmaceutical preparations which may be used orally include push-fit capsules made of gelatin, as well as soft, sealed capsules made of gelatin and a plasticizer such as glycerol or sorbitol. The push-fit capsules may contain the active compounds in the form of granules which may be mixed with fillers such as lactose, binders such as starches, and/or lubricants such as talc or magnesium stearate and, optionally, stabilizers. In soft capsules, the active compounds are preferably dissolved or suspended in suitable liquids, such as fatty oils, or liquid paraffin. In addition, stabilizers may be added.

Possible pharmaceutical preparations which may be used rectally include, for example, suppositories, which consist of a combination of one or more of the active compounds with a suppository base. Suitable suppository bases are, for example, natural or synthetic triglycerides, or paraffin hydrocarbons. In addition, it is also possible to use gelatin rectal capsules which consist of a combination of the active compounds with a base. Possible base materials include, for example, liquid triglycerides, polyethylene glycols, or paraffin hydrocarbons.

Suitable formulations for parenteral administration include aqueous solutions of the active compounds in water-soluble form, for example, water-soluble salts and alkaline solutions. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides or polyethylene glycol-400 (the compounds are soluble in PEG-400). Aqueous injection suspensions may contain substances which increase the viscosity of the suspension include, for example, sodium carboxymethyl cellulose, sorbitol, and/or dextran. Optionally, the suspension may also contain stabilizers.

In accordance with one aspect of the present invention, compounds of the invention are employed in topical and parenteral formulations and are used for the treatment of skin cancer.

The topical compositions of this invention are formulated preferably as oils, creams, lotions, ointments and the like, by choice of appropriate carriers. Suitable carriers include vegetable or mineral oils, white petrolatum (white soft paraffin), branched chain fats or oils, animal fats and high molecular weight alcohol (greater than $C_{12}$). The preferred carriers are those in which the active ingredient is soluble. Emulsifiers, stabilizers, humectants and antioxidants may also be included as well as agents imparting color or fragrance, if desired. Additionally, transdermal penetration enhancers may be employed in these topical formulations. Examples of such enhancers are found in U.S. Patent Nos. 3,989,816 and 4,444,762.

Creams are preferably formulated from a mixture of mineral oil, self-emulsifying beeswax and water in which mixture the active ingredient, dissolved in a small amount of an oil, such as almond oil, is admixed. A typical example of such a cream is one which includes about 40 parts water, about 20 parts beeswax, about 40 parts mineral oil and about 1 part almond oil.

Ointments may be formulated by mixing a solution of the active ingredient in a vegetable oil, such as almond oil, with warm soft paraffin and allowing the mixture to cool. A typical example of such an ointment is one which includes about 30% almond oil and about 70% white soft paraffin by weight.

The following examples are illustrative, but not limiting, of the method and compositions of the present invention.

***Example 1*** (Reference Example)

***Gambogic Acid***

**Procedure 1:**

**Step A.** The dry gamboge powder (140 g) was extracted with MeOH (3 x 600 mL) at room temperature for 1 week, after filtration, the solvent was removed under reduced pressure, gave crude extract (122 g) as yellow powder.

**Step B. Gambogic acid pyridine salt.** The above crude extract (120 g) was dissolved in pyridine (120 mL), then warm water (30 mL) was added to the stirred solution. After cooling to r.t., some precipitate was observed. Hexane (120 mL) was added to the mixture and the mixture was filtered and the solid was washed with hexane and dried. The salt was purified by repeated recrystallization from ethanol and gave gambogic acid pyridine salt (7.5 g); HPLC: 99%.

**Step C. Gambogic acid.** The gambogic acid pyridine salt (0.4 g) was dissolved in ether (25 mL) and shaken with aqeuous HCl (1N, 25 mL) for 1 h. The ether solution was then washed with water (2 x 10 mL), dried and evaporated to give the title compound (345 mg); HPLC: 99%. $^1$H NMR ($CDCl_3$): 12.66 (s, 1H), 7.43 (d, J = 6.9 Hz, 1H), 6.48 (d, J = 10.2 Hz, 1H), 5.97 (t, J = 7.5 Hz, 1H), 5.26 (d, J = 9.9 Hz, 1H), 4.91 (m, 2H), 3.37 (m, 1H), 3.24-2.98 (m, 2H), 2.81 (d, J = 6.6 Hz, 1H), 2.41 (d, J = 9Hz, 1H), 2.20 (m, $J_1$ = 8.4 Hz, $J_2$ = 5.1 HZ, 1H), 1.88 (m, 1H), 1.63 (s, 3H), 1.60 (s, 3H), 1.58 (s, 3H), 1.53 (s, 3H), 1.51 (s, 3H), 1.43 (s, 3H), 1.26 (s, 3H), 1.18 (s, 3H). MS: 627 (M-H).

**Procedure 2:**

The crude extract of gamboge (300 mg) was purified by repeated column chromatography ($SiO_2$, hexane-EtOAc gradient) using a Combi Flash SG 100 separation system, gave 18 mg of gambogic acid; HPLC: 94%, MS. 627 (M-H).

***Example 2*** (Reference Example)

***Gambogenic Acid***

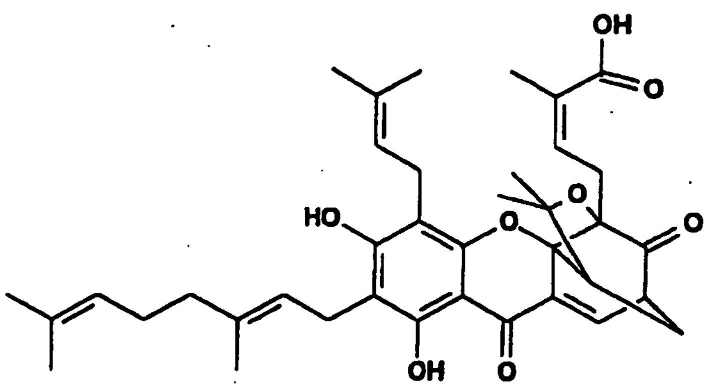

The crude extract of gamboge (300 mg) was purified as described in Example 1, procedure 2, to give 3 mg of gambogenic acid; HPLC: 84%, MS. 629 (M-H).

***Example 3*** (Reference Example)

***Gambogenin***

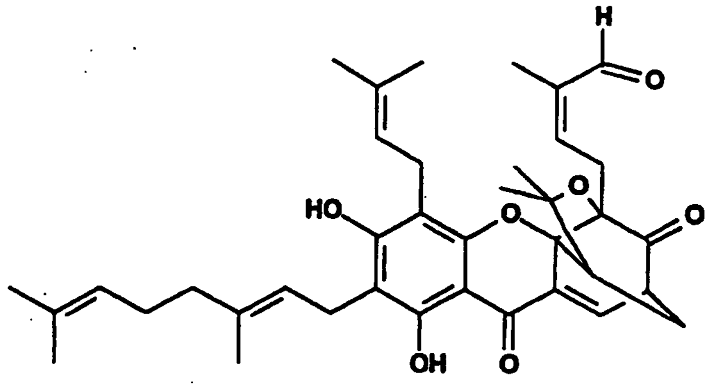

The crude extract of gamboge (300 mg) was purified as described in Example 1, procedure 2, to give 2 mg of gambogenin, HPLC: 71%. MS. 613 (M-H).

***Example 4***

***Methyl Gambogate***

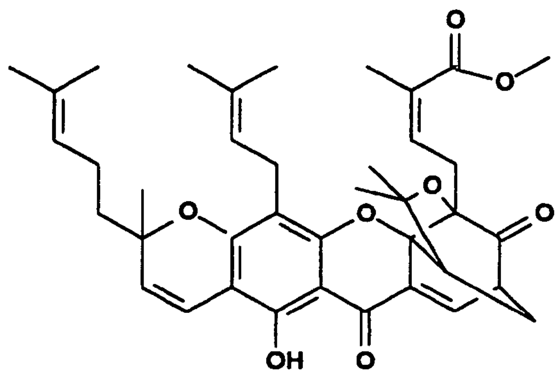

A mixture of gambogic acid (200 mg, 0.32 mmol), DMAP (78 mg, 0.64 mmol), EDC (123 mg, 0.64 mmol) and methanol (102 mg, 3.2 mmol) in THF (5 mL) was stirred at room temperature for 3 h. The solution was poured into water (10 mL) and was extracted with ethyl acetate (3 x 10 mL). The combined organic layer was dried and concentrated to give the crude product, which was purified by chromatography ($SiO_2$, EtOAc/Hexane 1:5) to give the title compound (196 mg, 95%). $^1H$ NMR ($CDCl_3$): 12.85 (s, 1H), 7.54 (d, J = 6.9 Hz, 1H), 6.67 (d, J = 10.5 Hz, 1H), 5.94 (t, J = 6 Hz, 1H), 5.43 (d, J = 10.2 Hz, 1H), 5.05 (m, 2H), 3.49 (m, 1H), 3.43 (s, 3H), 3.35-3.10 (s, 2H), 3.00 (t, J = 7.2 Hz, 1H), 2.52 (d, J = 10.2 Hz, 1H), 2.32 (quar, J1 = 4.8 Hz, 1H), 2.02 (m, 1H), 1.74 (s, 3H), 1.69 (s, 3H), 1.67-1.64 (m, 9H), 1.55 (s, 3H), 1.44 (s, 3H), 1.29 (s, 3H).

***Example 5***

***Gambogyl Piperidine***

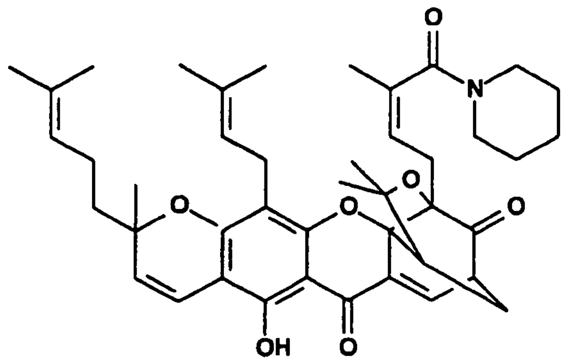

A mixture of gambogic acid (200 mg, 0.32 mmol), DMAP (39 mg, 0.32 mmol), EDC (123 mg, 0.64 mmol) and piperidine (54.2 mg, 0.64 mmol) in THF (3 mL) was stirred at room temperature for 6 h. The solution was poured into water (10 mL) and was extracted with ethyl acetate (3 x 10 mL). The combined organic layer was dried and concentrated to give the crude product, which was purified by chromatography ($SiO_2$, EtOAc/$CH_2Cl_2$ 1:8) to give the title compound (187 mg, 84%). $^1$H NMR ($CDCl_3$): 12.87 (s, 1H), 7.53 (d, J = 6.9 Hz, 1H), 6.68 (d, J = 10.2 Hz, 1H), 5. 43 (d, J = 10.5 Hz, 1H), 5.40 (t, J = 6 Hz, 1H), 5.05 (m, 2H), 3.54-3.33 (m, 2H), 3.28 (d, J = 6.9 Hz, 1H), 3.11 (t, J = 8.4 Hz, 1H), 2.50 (d, J = 9.6 Hz, 1H), 2.46-2.17 (m, 3H), 2.00 (m, 1H), 1.75-1.72 (m, 5H), 1.68 (s, 2H), 1.65 (bs, 6H), 1.58 (s, 3H), 1.56 (s, 3H), 1.43 (s, 3H), 1.25 (s, 3H).

***Example 6***

***Methyl-6-methoxy-gambogate***

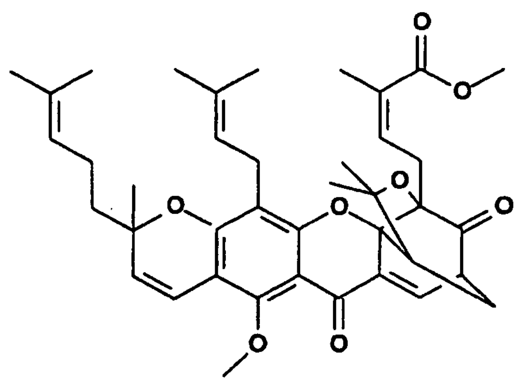

A mixture of methyl gambogate (70 mg, 0.11 mmol), anhydrous $K_2CO_3$ (0.5 g), methyl iodide (1 mL) in acetone (5 mL) was stirred at room temperature for 70 h. After evaporation to near dryness, water (30mL) was added into the mixture and it was extracted with ethyl acetate (3 x 10 mL). The combined organic layer was dried and concentrated to give the crude product, which was purified by chromatography ($SiO_2$, EtOAc/Hexane 1:4) to give the title compound (69 mg, 96%). MS. 657(M+H), 679 ($M+Na^+$). $^1$H NMR ($CDCl_3$): 7.41 (d, J = 6.9 Hz, 1H), 6.64 (d, J = 9.9 Hz, 1H), 5.93 (m, J1 = 6.9Hz, J2 = 0.9 Hz, 1H), 5.52 (d, J = 10.2 Hz, 1H), 5.05 (m, 2H), 3.79 (s, 3H), 3.40 (s, 3H), 3.45-3.18 (m, 3H), 2.95 (d, J = 9.6 Hz, 1H), 2.26 (m, 1H), 2.02 (m, 1H), 1.73 (s, 3H), 1.66 (d, 3H), 1.63 (bs, 6H), 1.52 (s, 3H), 1.42 (s, 3H), 1.27(s, 3H).

***Example 7***

***6-Methoxy-gambogyl Piperidine***

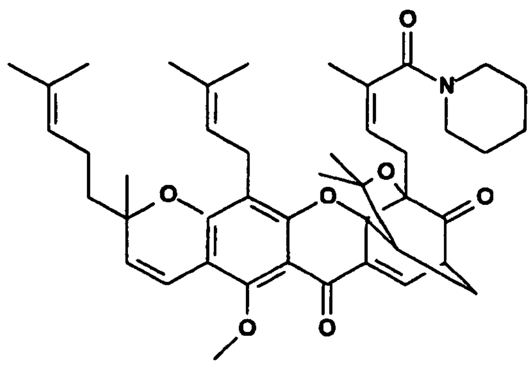

The title compound was prepared by a procedure similar to that of Example 6 from gambogyl piperidine and methyl iodide. MS: 710 (M+H), 732 (M+Na$^+$). $^1$H NMR ($CDCl_3$): 7.40 (d, J = 6.9 Hz, 1H), 6.64 (d, J = 10.2 Hz, 1H), 5.53 (d, J = 10.2 Hz, 1H), 5.34 (m, 1H), 5.09 (t, 1H), 5.04 (t, 1H), 3.80 (s, 3H), 3.52(m, 3H), 3.38-3.31 (m, 3H), 3.11 (t, 2H), 2.50- 1.98 (m, 5H), 1.73 (s, 3H), 1.70 (d, 3H), 1.65 (s, 6H), 1.63 (bs, 6H), 1.53 (s, 3H), 1.42 (s, 3H), 1.22 (s, 3H).

***Example 8***

***10-Morpholinyl-gambogyl Morpholine***

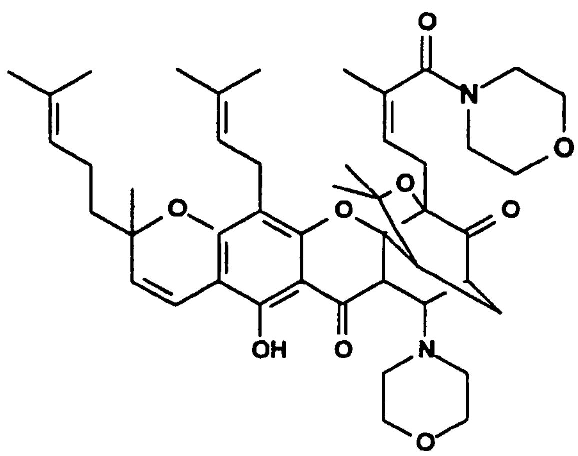

A mixture of gambogic acid (100 mg, 0.16 mmol), DMAP (20 mg, 0.16 mmol), EDC (67.4 mg, 0.35 mmol) and morpholine (30.6 mg, 0.35 mmol) in THF (3 mL) was stirred at room temperature for 6 h. The solution was poured into water (10 mL) and was extracted with ethyl acetate (3 x 10 mL). The combined organic layer was dried and concentrated to give the crude product, which was purified by chromatography ($SiO_2$, EtOAc/$CH_2Cl_2$ 1: 1) to give the title compound (87 mg, 69%). MS: 785 (M+H), 807 (M+Na$^+$). $^1$H NMR ($CDCl_3$): 11.94 (s, 1H), 6.63 (d, J = 7.5 Hz, 1H), 5.96 (m, 1H), 5.43 (d, J = 10.2 Hz, 1H), 5.08 (m, 2H), 3.80 (m, 1H), 3.70-3.12 (m, 12H), 2.80-2.36 (m, 7H), 2.05 (m, 1H), 1.95 (m, 1H), 1.87 (s, 3H), 1.73 (bs, 3H), 1.64 (bs, 6H), 1.55 (s, 3H), 1.45 (m, 1H), 1.32 (s, 3H), 1.28 (s, 3H), 1.24 (3H), 1.07 (s, 3H).

***Example 9***

***Gambogyl (4-Methylpiperazine)***

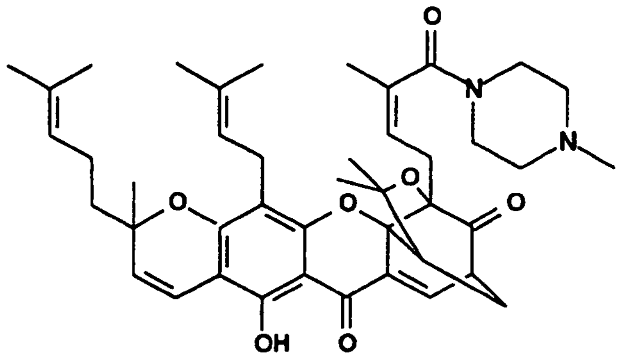

A mixture of gambogic acid (93 mg, 0.15 mmol), DMAP (22 mg, 0.18 mmol), EDC (34 mg, 0.18 mmol) and N-methyl piperazine (15 mg, 0.15 mmol) in THF (5 mL) was stirred at room temperature for 5 h. The solution was poured into water (50 mL) and was extracted with ethyl acetate (3 x 10 mL). The combined organic layer was dried and concentrated to give crude product, which was purified by chromatography ($SiO_2$, EtOAc/MeOH 12:1) to give the title compound (35 mg, 33%). MS: 709 (M-H), 711 (M+H), 733 ($M+Na^+$). $^1H$ NMR ($CDCl_3$): 12.85 (s, 1H), 7.52 (d, J = 6.6 Hz, 1H), 6.66 (d, J = 9.9 Hz, 1H), 5.42 (t, J = 10.5 Hz, 1H), 5.05 (m, 2H), 3.62 (m, 1H), 3.40 (m, 2H), 3.28-3.17 (m, 4H), 2.50- 1.98 (m, 7H), 2.23 (s, 3H), 1.72 (bs, 6H), 1.63 (bs, 6H), 1.53 (bs, 6H), 1.41 (s, 3H), 1.23 (s, 3H).

***Example 10***

***10-Morpholinyl-gambogyl Piperidine***

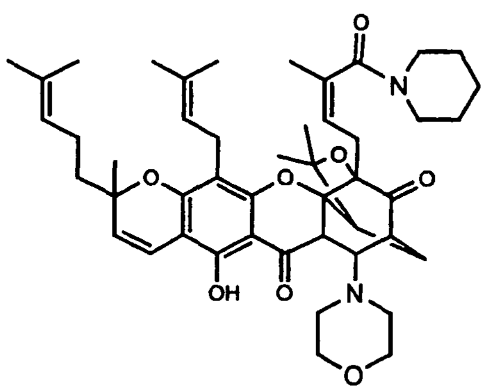

A solution of gambogyl piperidine (50 mg, 0.071 mmol) and morpholine (0.3 mL) in THF (3 mL) was stirred for 48 h. It was evaporated and the crude product was purified through chromatography to yield the title compound (48 mg, 86%). $^1H$ NMR ($CDCl_3$) 11.94 (s, 1H), 6.66 (d, J=9.9 Hz, 1H), 5.92 (t, 1H), 5.44 (d, J= 10.2 Hz, 1H), 5.06 (m, 1H), 3.72-3.12 (m, 12H), 2.80 (m, 3H), 2.60-2.40 (m, 4H), 2.06 (m, 2H), 1.88 (s, 3H), 1.75 (s, 3H), 1.66 (s, 3H), 1.65 (s, 3H), 1.57 (s, 3H), 1.55 (m, 2H), 1.34 (s, 3H), 1.32 (s, 3H), 1.22 (s, 2H), 1.11 (s, 3H).

***Example 11***

***10-(4-Methylpiperazinyl)-gambogyl Piperidine***

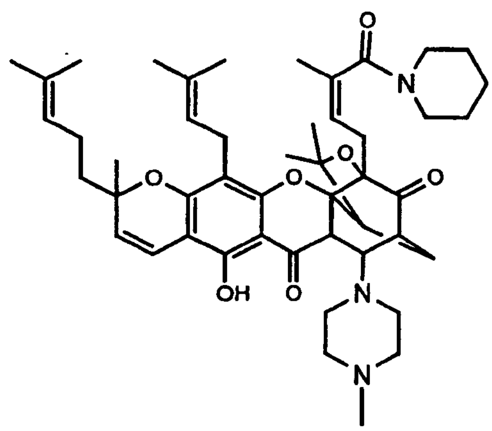

The title compound was prepared from gambogyl piperidine and N-methylpiperazine by a procedure similar to that of Example 10. MS. 797 (M+H), 819 (M+Na), 835 (M+K), 795 (M-H). $^{1}H$ NMR ($CDCl_3$) 12.01 (s, 1H), 6.66 (d, J=9.9 Hz, 1H), 5.94 (t, 1H), 5.44 (d, J= 10.2 Hz, 1H), 5.12-5.10 (m, 2H), 3.80 (d, 1H), 3.52 (d, 1H), 3.38-3.12 (m, 5H), 2.78-2.26 (m, 6H), 2.24 (s, 3H), 2.12-2.04 (m, 2H),), 1.89 (s, 3H), 1.75 (s, 3H), 1.66 (s, 6H), 1.57 (s, 3H), 1.55 (m, 2H), 1.34 (s, 3H), 1.32 (s, 3H), 1.11 (s, 3H).

***Example 12***

***N-(2-Gambogylamidoethyl)biotinamide***

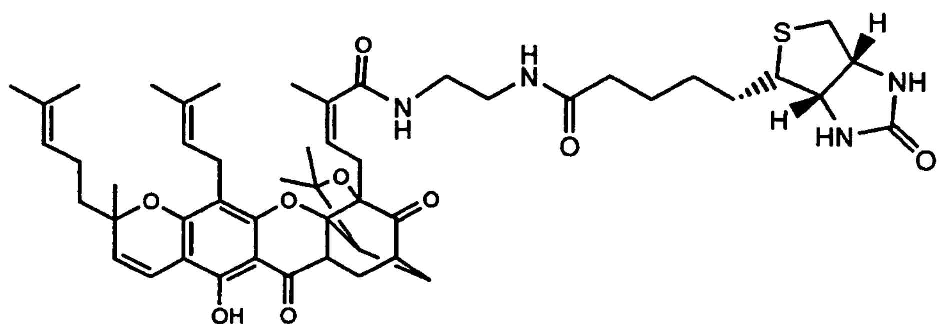

The title compound was prepared by a procedure similar to that of Example 9 from gambogic acid and N-(2-aminoethyl)biotinamide. MS: 919 (M+Na), 897 (M+H), 895 (M-H). $^{1}H$ NMR ($CDCl_3$): 12.9, 12.78 (1H), 7.60-7.57 (m, 1H), 6.90 (m, 1H), 6.78 (m, 1H), 6.70-6.62 (m, 1H), 5.48(d, J = 9.9Hz, 1H), 5.42 (m, 1H), 5.30 (m, 1H), 5.08 (m, 2H), 4.66 (s,1H), 4.49 (m, 1H), 4.33 (m,1H), 3.58-3.40 (m, 2H), 3.38-3.10 (m, 5H), 3.16-2.88 (m, 1H), 2.80- 2.52 (m, 2H), 2,40-1.92 (m, 6H), 1.78 (bs, 3H), 1.74 (bs, 2H), 1.73 (bs, 3H), 1.69 (bs, 3H), 1.65 (bs, 6H), 1.55 (bs, 3H), 1.50-1.20 (m, 13H), 1.2-0.88 (m, 4H).

***Example 13*** (Reference Example)

***10-(4-Methylpiperazinyl)gambogic Acid***

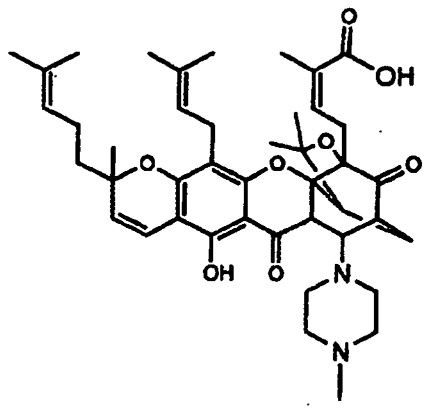

A solution of gambogic acid (35 mg, 0.056 mmol) and N-methylpiperazine (0.5 mL) in THF (4 mL) was stirred for 24 h, then another portion of N-methylpiperazine (0.5 mL) was added and stirred for 48 h. The solution was diluted with EtOAc (30 mL) and washed with aqueous $NH_4Cl$ ( 3 x 30 mL). After concentration, the mixture was dissolved in ethyl ether (15 mL) and washed with 0.1 N HCl. After concentration, the residue was washed with hexane four times to gave the title compound (9 mg, 20 %). $^1H$ NMR ($CDCl_3$) 11.8 (s, 1H), 6.64 (d, J=9.9 Hz, 1H), 6.57(t, 1H), 5.45 (d, J= 10.5 Hz, 1H), 5.09 (t, 1H), 3.51 (bs, 1H), 3.30-2.70 (m, 11H), 2.81 (s, 3H), 2.51 (d, J=8.4Hz, 2H), 2.12-2.02 (m, 2H), 1.96 (s, 3H), 1.73 (s, 3H), 1.66 (s, 3H), 1.63 (s, 3H), 1.56 (s, 3H), 1.35 (s, 6H), 1.26 (m, 2H), 1.11 (s, 3H), 0.88 (m, 2H).

***Example 14***

***10-Piperidyl-gambogyl Piperidine***

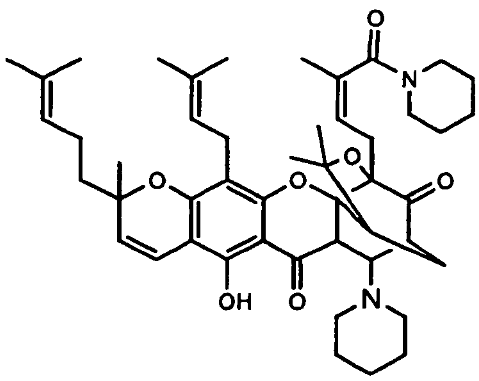

A mixture of gambogic acid (460 mg, 0.73 mmol), EDCI (166 mg, 0.87 mmol), DMAP (47 mg, 0.38 mmol) and piperidine (75 μL, 0.76 mmol) in THF (5 mL) was stirred at room temperature for 40 h. It was diluted with 1:1 hexane/EtOAc (80 mL), washed with water and brine, dried over $Na_2SO_4$ and concentrated in vacuo. The residue was purified by chromatography (3:2 hexane/EtOAc) to yield the title compound as a pale yellow solid (40 mg, 0.051 mmol, 7%) and gambogyl 1'-piperidine (220 mg, 0.32 mmol). $^1H$ NMR ($CDCl_3$): 12.05 (s, 1H), 6.66 (d, J = 10.0 Hz, 1H), 5.95 (t, J = 6.3 Hz, 1H), 5.44 (d, J = 10.0 Hz, 1H), 5.12-5.03 (m, 2H), 3.75-3.10 (m, 9H), 2.76-2.66 (m, 3H), 2.49 (d, J = 8.4, 2H), 2.34 (m, 2H), 2.08 (m, 3H), 1.89 (s, 3H), 1.75 (s, 3H), 1.66 (d, 3H), 1.61-1.23 (m, 15H), 1.33 (s, 3H), 1.31 (s, 3H), 1.27(s, 3H), 1.26 (s, 3H).

***Example 15*** (Reference Example)

***10-Piperidinyl-gambogic Acid***

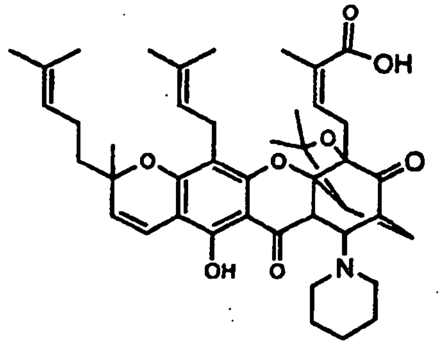

Gambogic acid (10 mg, 0.016 mmol) in piperidine (0.5 mL) was stirred at room temperature for 40 h. The solvent was removed in vacuo. The residue was diluted with 1:2 hexane/EtOAc (50 mL), washed with saturated ammonium chloride aqueous solution followed by brine, dried over $Na_2SO_4$ and concentrated in vacuo. The residue was purified by chromatography (3:2 hexane/EtOAc) to yield one of the diasteromers of the title compound (A, 3 mg, 0.004 mmol, 26%) and the other diastereomer (B, 1 mg, 0.001 mmol, 6%). $^1H$ NMR ($CDCl_3$): diastereomer A: 12.00 (s, 1H), 6.66 (d, J = 9.9 Hz, 1H), 6.52 (t, J = 6.9 Hz, 1H), 5.46 (d, J = 9.9 Hz, 1H), 5.12-5.05 (m, 2H), 3.32-3.04 (m, 6H), 2.81 (t, J = 4.5, 1H), 2.55-2.43 (m, 3H), 2.33(m, 2H), 2.12-1.91 (m, 3H), 1.98 (s, 3H), 1.74 (s, 3H), 1.66 (s, 3H), 1.63 (s, 3H), 1.35 (s, 6H), 1.50-1.28 (m, 6H), 1.14 (s, 3H); diastereomer B: 12.00 (s, 1H), 7.37 (t, J = 6.3 Hz, 1H), 6.66 (d, J = 10.0 Hz, 1H), 5.46 (d, J = 10.0 Hz, 1H), 5.12-5.02 (m, 2H), 3.35-3.18 (m, 3H), 3.11 (s, 1H), 2.91-2.79 (m, 3H), 2.56-2.48 (m, 3H), 2.33(m, 2H), 2.12-1.94 (m, 5H), 1.87 (s, 3H), 1.74 (s, 3H), 1.66 (s, 3H), 1.63 (s, 3H), 1.40 (s, 3H), 1.34 (s, 3H), 1.50-1.28 (m, 6H), 1.13 (s, 3H).

***Example 16*** (Reference Example)

***9,10-Dihydro-12-hydroxygambogic Acid***

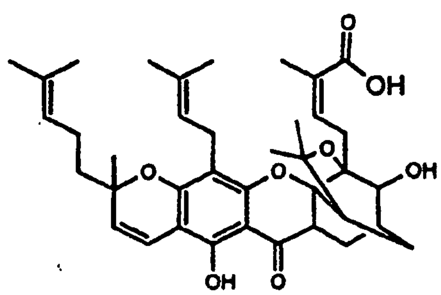

To a solution of gambogic acid (14 mg, 0.022 mmol) in methanol (2 mL) was added $NaBH_4$ (22 mg, 0.58 mmol) at 0° C. The mixture was stirred for 3 h and the cooling bath was allowed to slowly warm to room temperature. Acetone (0.5 mL) was added to the mixture and it was stirred for 30 min., acidified with 2 N HCl to pH 6, diluted with EtOAc (40 mL), washed with water (3 times) and brine, dried over $Na_2SO_4$ and concentrated in vacuo. The residue was purified by chromatography (9:1 EtOAc/MeOH) to give the title compound as an oil (9 mg, 0.014 mmol, 66%). $^1H$ NMR ($CDCl_3$): 12.02 (s, 1H), 6.66 (d, J = 10.2 Hz, 1H), 6.33 (t, J = 7.2, 1H), 5. 45 (d, J = 10.2 Hz, 1H), 5.12-5.05 (m, 2H), 3.71 (s, 1H), 3.26-3.11 (m, 3H), 3.02-2.94 (m, 2H), 2.56-2.49 (m, 1H), 2.36 (d, J = 9.6, 1H), 2.12-2.04 (m, 3H), 1.99 (s, 3H), 1.76 (m, 1H), 1.73 (s, 3H), 1.56 (s, 3H), 1.63 (s, 3H), 1.60-1.30 (m, 5H), 1.42 (s, 3H), 1.39 (s, 3H), 1.35 (s, 3H).

***Example 17***

***Methyl-10-Morpholinyl-gambogate***

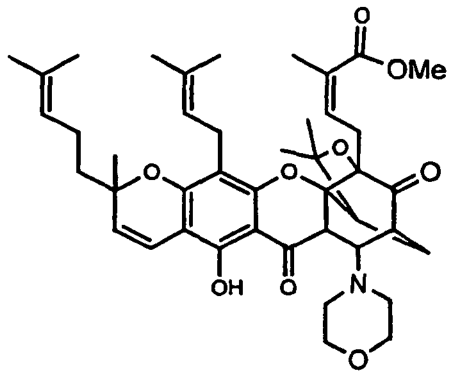

To a solution of methyl gambogate (50 mg, 0.078 mmol) in THF (2 mL) was added morpholine (70 L, 0.80 mmol). The mixture was stirred at room temperature for 17 h, diluted with 1:1 hexane/EtOAc (100 mL), washed with water (3 times) and brine, dried over $Na_2SO_4$ and concentrated in vacuo to yield the title compound as light yellow solid (52 mg, 0.071 mmol, 91%). $^1H$ NMR ($CDCl_3$): 11.98 (s, 1H), 6.66 (d, J = 10.2 Hz, 1H), 6.62 (t, J = 6.6 Hz, 1H), 5.46 (d, J = 10.2 Hz, 1H), 5.12-5.00 (m, 2H), 3.68 (s, 3H), 3.74-3.55 (m, 4H), 3.43-3.14 (m, 6H), 2.77 (m, 1H), 2.59-2.40 (m, 5H), 2.07(m, 1H), 1.95 (s, 3H), 1.74 (s, 3H), 1.66 (s, 3H), 1.63 (s, 3H), 1.36 (s, 3H), 1.35 (s, 3H), 1.14 (s, 3H).

***Example 18*** (Reference Example)

***Isogambogic Acid***

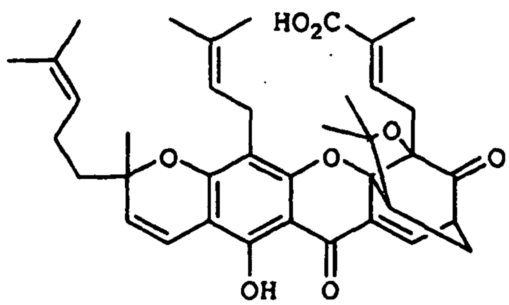

The crude extract of gamboge (300 mg) was purified as described in Example 1, procedure 2, to give 2 mg of isogambogic acid; MS. 627 (M-H).

***Example 19*** (Reference Example)

***Morellic Acid***

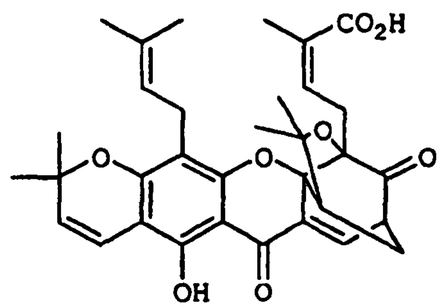

The crude extract of gamboge (300 mg) was purified as described in Example 1, procedure 2, to give 2 mg of morellic acid; MS. 559 (M-H).

***Example 20*** (Reference Example)

***10-Cyclohexylgambogic Acid***

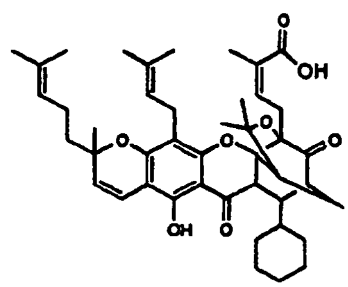

To a solution of gambogic acid (80 mg, 0.13 mmol) in THF (5 mL) was added a solution of cyclohexylcuprate (1.2 mmol) in THF prepared from cyclohexylmagnesium chloride and CuI at 0˚ C. The mixture was stirred for 2 h and the cooling bath was allowed to slowly warm to room temperature. The reaction was quenched with 2 N HCl and diluted with 1:1 hexane/EtOAc (80 mL). The resulting mixture was washed with water and brine, dried over $Na_2SO_4$ and concentrated *in vacuo*. The residue was purified by chromatography (3:1 hexane/EtOAc) to give the title compound as an oily solid (9 mg, 0.013 mmol, 10%). $^1H$ NMR ($CDCl_3$): 6.61 (d, J = 10.2, 1H), 6.14 (t, J = 6.0, 1H), 5.39 (d, J = 10.2, 1H), 5.20 (t, J = 6.6,1H), 5.06 (t, J = 7.2, 1H), 3.64 (m, 1H), 3.35-3.10 (m, 3H), 2.82 (br s, 2H), 2.67-2.61 (m, 2H), 1.76 (s, 3H), 1.72 (s, 3H), 1.68 (s, 3H), 1.66 (s, 3H), 1.56 (s, 3H), 1.44 (s, 3H), 1.94-1.25 (m, 15H).

***Example 21*** (Reference Example)

***10-Methylgambogic Acid***

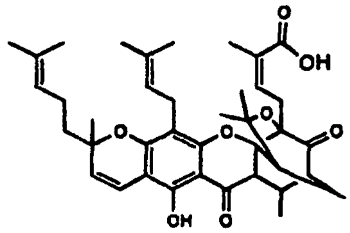

The title compound was prepared by a procedure similar to that of Example 20 from gambogic acid and methylcuprate and was isolated as an oil. $^1H$ NMR ($CDCl_3$): 6.58 (d, J = 10.2, 1H), 6.12 (t, J = 6.6, 1H), 5.36 (d, J = 9.9, 1H), 5.15 (t, J = 6.6, 1H), 5.04 (t, J = 7.2, 1H), 3.32-3.10 (m, 2H), 2.93 (d, J = 8.4, 1H), 2.84 (d, J = 6.3, 2H), 2.62 (d, J = 7.6, 1H), 2.40 (t, J = 7.2, 1H), 2.25 (d, J = 4.8, 1H), 2.08-1.92 (m, 4H), 1.73(s, 3H), 1.68 (s, 3H), 1.66 (s, 6H), 1.64 (s, 3H), 1.54 (s, 3H), 1.41 (s, 3H), 1.35 (d, J = 6.9, 3H), 1.23 (s, 1H).

***Example 22*** (Reference Example)

***9,10-Dihydrogambogic Acid***

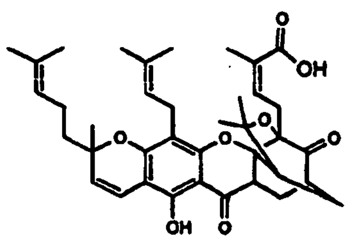

To a solution of gambogic acid (17 mg, 0.027 mmol) in methylenechloride (2 mL) was added L-selectride solution in THF (1.0 mL, 0.5 mmol) dropwise at -78 ˚C. After 30 min of stirring, the reaction was quenched with 1 mL of 2 N HCl. The mixture was then allowed to warm to room temperature and was diluted with 1:1 hexane/EtOAc (50 mL). The resulting mixture was washed with water and brine, dried over $Na_2SO_4$ and concentrated *in vacuo.* The residue was purified by chromatography (2:3 hexane/EtOAc/) to give the title compound as an oil (0.6 mg, 0.001 mmol, 4%). $^1H$ NMR ($CDCl_3$): 11.96 (s, 1H), 6.67 (d, J = 10.2, 1H), 6.54 (t, J = 6.6, 1H), 5.46 (d, J = 10.2, 1H), 5.13-5.05 (m, 2H), 3.33-3.16 (m, 3H), 2.85 (d, J = 13.8, 1H), 2.60 (d, J = 8.7, 1H), 2.43 (s, 1H), 2.08 (m, 1H), 1.97 (s, 3H), 1.74 (s, 3H), 1.67 (s, 6H), 1.64 (s, 3H), 1.57 (s, 3H), 1.37 (s, 3H), 1.36 (d, J = 6.9, 3H), 1.31-1.22 (m, 5H), 1.14 (s, 3H).

***Example 23***

***Gambogyl (2-(4-Morpholinyl)ethylamine)***

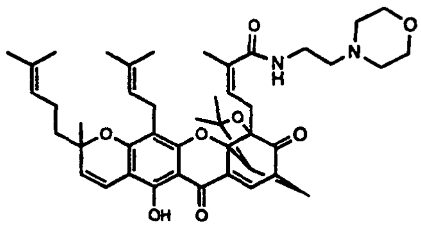

The title compound was prepared as described in Example 5 from gambogic acid and 2-(4-morpholinyl)ethylamine and isolated as a yellow solid (75 mg, 0.10 mmol, 56%). $^1$H NMR ($CDCl_3$): 12.86 (s, 1H), 7.54 (d, J = 6.6, 1H), 6.68 (d, J = 10.2, 1H), 6.56 (t, J = 5.1, 1H), 5.46 (d, J = 10.2, 1H), 5.28 (d, J = 7.5, 1H), 5.05 (br s, 1H), 3.68 (t, J = 4.2, 4H), 3.47 (m, 1H), 3.71-3.17 (m, 4H), 2.68 (t, J = 6.6, 2H), 2.54 (d, J = 9.6, 1H), 2.48-2.44 (m, 6H), 2.36-2.30 (m, 1H), 2.01-2.00 (m, 3H), 1.74 (s, 6H), 1.67 (s, 3H), 1.65 (s, 6H), 1.61 (s, 3H), 1.44 (s, 3H), 1.28 (s, 3H).

***Example 24*** (Reference Example)

***9,10-Epoxygambogic Acid***

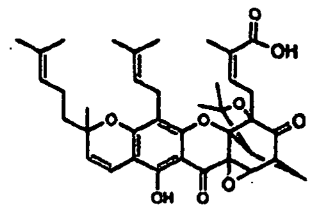

To a solution of gambogic acid (52 mg, 0.08 mmol) in methanol (2 mL) was added 2 N NaOH (0.5 mL, 1.0 mmol), followed by 35% $H_2O_2$ (0.2 mL, 2.1 mmol) at room temperature. The mixture was stirred at room temperature for 10 min, diluted with 1:1 hexane/EtOAc (50 mL), washed with water, 2 N HCl and brine, dried over $Na_2SO_4$ and concentrated *in vacuo*. The residue was purified by chromatography (1:2 hexane/EtOAc) to yield the title compound as an oil (2.2 mg, 0.003 mmol, 4%). $^1$H NMR ($CDCl_3$): 11.92 (s, 1H), 6.66 (d, J = 10.2, 1H), 6.51 (t, J = 6.9, 1H), 5.46 (d, J = 9.9, 1H), 5.09-5.04 (m, 2H), 4.35 (d, J = 3.9, 1H), 3.32 (s, 2H), 3.27-2.99 (m, 4H), 2.85 (t, J = 4.8, 1H), 2.51 (d, J = 8.7, 1H), 2.07 (m, 1H), 1.97 (s, 3H), 1.74 (s, 3H), 1.66 (s, 3H), 1.63 (s, 3H), 1.56 (s, 3H), 1.36 (s, 3H), 1.15 (s, 3H).

***Example 25***

***Gambogyl (4-(2-Pyridyl)piperazine) and 10-[4-(2-Pyridyl)piperazinyl]gambogyl(4-(2-Pyridyl)piperazine)***

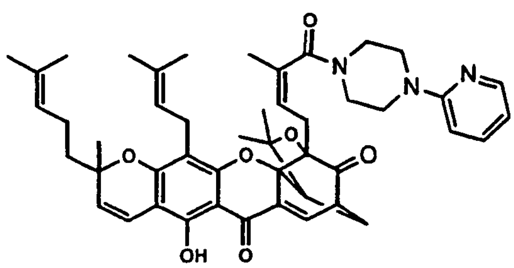

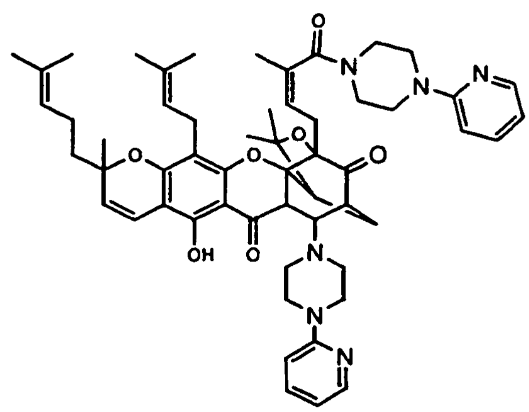

A mixture of gambogic acid (230 mg, 0.37 mmol), 1-(2-pyridyl)piperazine (75 μL, 0.46 mmol), and EDC (77 mg, 0.40 mmol) in DMF (3 mL) was stirred at room temperature, overnight. The mixture was diluted with 1:1 hexane/EtOAc (90 mL), washed with water and brine, dried over $Na_2SO_4$ and concentrated *in vacuo.* The residue was purified by chromatography (3:2 hexane/EtOAc) to yield 10 mg of gambogyl (4-(2-pyridyl)piperazine) as a yellow solid. $^1$H NMR ($CDCl_3$): 12.87 (s, 1H), 8.19 (m, 1H), 7.52 (d, J = 6.9, 1H), 6.68-6.62 (m, 2H), 6.43 (d, J = 9.9, 1H), 5.06 (br s, 2H),

3.76-3.27 (m, 11H), 2.52-2.00 (m, 6H), 1.76 (s, 3H), 1.73 (s, 3H), 1.68 (s, 3H), 1.65 (s, 6H), 1.56 (s, 3H), 1.42 (s, 3H), 1.26 (s, 3H); and 31 mg of 10-[4-(2-pyridyl)piperazinyl]gambogyl(4-(2-pyridyl)piperazine) as a yellow solid. $^1$H NMR ($CDCl_3$): 11.99 (s, 1H), 8.17 (d, J = 4.8, 2H), 7.45 (t, J = 7.5, 2H), 6.68-6.52 (m, 5H), 6.00 (t, J = 6.6, 1H), 5.44 (d, J = 10.2, 1H), 5.11-5.07 (m, 2H), 3.90-3.13 (m, 15H), 2.83-2.51 (m, 8H), 2.07 (m, 2H), 1.90 (s, 3H), 1.73 (s, 3H), 1.65 (s, 6H), 1.57 (s, 3H), 1.35 (s, 3H), 1.32 (s, 3H), 1.12 (s, 3H);

*Example **26*** (Reference Example)

***6-Acetyl-gambogic Acid***

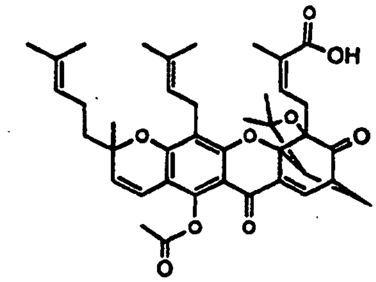

A mixture of gambogic acid (154 mg, 0.24 mmol) and $Ac_2O$ (0.3 mL, 3.2 mmol) in pyridine (3 mL) was stirred at room temperature for four days. The mixture was diluted with 1:1 hcxane/EtOAc (80 mL), washed with water, 2 N HCl and brine, dried over $Na_2SO_4$ and concentrated *in vacuo*. The residue was purified by chromatography (1:2 hexane/EtOAc) to yield the title compound as a yellow solid (47 mg, 0.07 mmol, 29%). $^1$H NMR ($CDCl_3$): 7.44 (d, J = 6.9, 1H), 6.66 (t, J = 6.6, 1H), 6.40 (d, J = 10.2, 1H), 5.60 (d, J = 10.5, 1H), 5.13 (t, J = 6.9, 1H), 5.04 (t, J = 6.9, 1H), 3.46 (m, 1H), 3.34 (d, J = 6.9, 1H), 2.67-2.50 (m, 3H), 2.39 (s, 3H), 2.33-2.27 (m, 1H), 2.08-1.98 (m, 2H), 1.73 (s, 3H), 1.71 (s, 3H), 1.65 (s, 6H), 1.54 (s, 3H), 1.40 (s, 3H), 1.6 (s, 3H), 1.29 (s, 3H).

***Example 27*** (Reference Example)

***10-[4-(2-Pyridyl)piperazinyl]gambogic Acid***

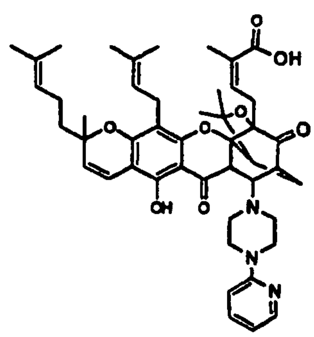

A mixture of pyridinium gambogate (228 mg, 0.32 mmol) and 1-(2-pyridyl)piperazine (289 mg, 1.8 mmol) in THF (3 mL) was stirred at room temperature, overnight. The mixture was diluted with 1:1 hexane/EtOAc (80 mL), washed with water, 2 N HCl and brine, dried over $Na_2SO_4$ and concentrated in vacuo to yield the title compound as a yellow solid (143 mg, 0.16 mmol, 50%). $^1$H NMR ($CDCl_3$): 11.97 (s, 1H), 8.15 (m, 1H), 7.45 (m, 1H), 6.68-6.54 (m, 4H), 5.46 (d, J = 9.9, 1H), 4.12-5.02 (m, 2H), 3.40-3.08 (m, 10H), 2.83 (t, J = 4.5, 1H), 2.71-2.67 (m, 2H), 2.57-2.52 (m, 3H), 1.95 (s, 3H), 1.74 (s, 3H), 1.66(s, 3H), 1.63 (s, 3H), 1.57 (s, 3H), 1.37 (s, 6H), 1.16 (s, 3H).

***Example 28***

***N-Hydroxysuccinimidyl Gambogate***

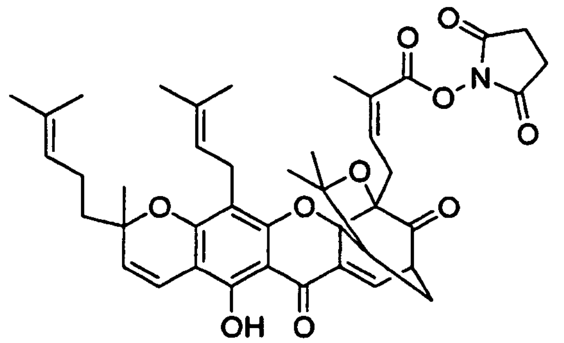

A mixture of gambogic acid (600 mg, 0.96 mmol), N-hydroxysuccinimide (221 mg, 1.92 mmol), DCC (296.6 mg, 1.44 mmol) in dichloromethane (20 mL) was stirred for 2 h. It was evaporated to dryness and the residue was dissolved in ethyl acetate (50 mL) and washed with water (50 mL x 3). The organic layer was dried and concentrated to give crude product, which was purified by flash column chromatography ($SiO_2$, EtOAc/hexane 1:3) to give the title compound (530 mg, 76%). $^1$H NMR ($CDCl_3$): 12.85 (s, 1H), 7.55 (d, J = 6.9 Hz, 1H), 6.67 (d, J = 10.2 Hz, 1H), 6.62 (t, J = 6.9 Hz, 1H), 5.44 (d, J = 9.9 Hz, 1H), 5.06 (m, 2H), 3.46 (m, 1H), 3.38-3.12 (m, 2H), 2.84-2.76 (m, 4H), 2.54 (d, 1H), 2.30 (m, 1H), 2.04 (m, 1H), 1.94 (s, 3H), 1.74(s, 3H), 1.72 (s, 3H), 1.66 (s, 3H), 1.63 (s, 3H), 1.56 (s, 3H), (bs, 6H), 1.43 (s, 3H), 1.29 (s, 3H).

***Example 29***

***8-(Gambogylamido)octanoic Acid***

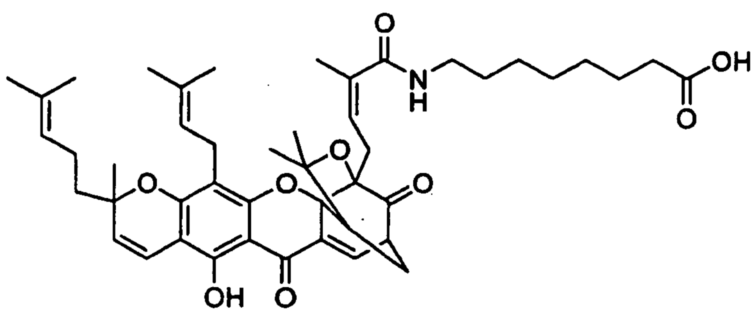

A solution of 8-aminooctanoic acid (3.07 mg, 0.019 mmol), N-hydroxysuccinimidyl gambogate (14 mg, 0.019 mmol), triethylamine (0.15 mL) in anhydrous DMSO (3 mL) was stirred overnight. It was diluted with water and extracted with ethyl acetate (3 x10 mL). The combined organic layer was dried and concentrated to give crude product, which was purified by column chromatography ($SiO_2$, EtOAc/MeOH 10:1) to give the title compound (11 mg, 67%). $^1$H NMR ($CDCl_3$): 12.80 (bs, 1H), 7.58 (bs, 1H), 6.64 (d, J = 9.3 Hz, 1H), 5.50-5.00 (m, 4H), 3.54 (bs, 1H), 3.32-3.00 (m, 3H), 3.50-2.42 (m, 4H), 1.75 (s, 3H), 1.72 (s, 3H), 1.70 (s, 3H). MS. 792 (M+$Na^+$), 768 (M-H).

***Example 30***

***6-(Gambogylamido)hexanoic Acid***

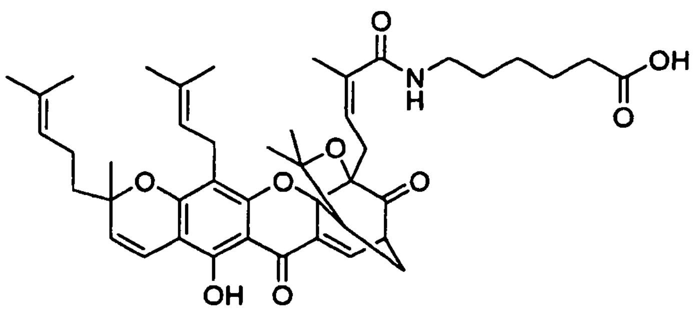

The title compound was prepared by a procedure similar to that of Example 29. $^1H$ NMR ($CDCl_3$): 12.70 (bs, 1H), 7.58 (bs, 1H), 6.62 (bs, 1H), 5.40 (bs, 1H), 5.20 (bs, 1H), 5.00 (bs, 2H), 3.60-3.00 (m, 4H), 3.50-2.42 (m, 4H), 1.74 (s, 3H), 1.72 (s, 3H), 1.69 (s, 3H). MS. 764 ($M+Na^+$), 740 (M-H).

***Example 31***

***12-(Gambogylamido)dodecanoic Acid***

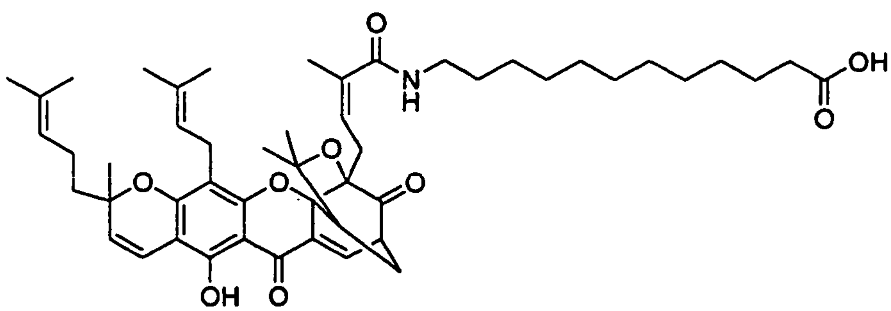

The title compound was prepared by a procedure similar to that of Example 29. $^1H$ NMR ($CDCl_3$): 12.7 (bs, 1H), 7.48 (d, 1H), 6.64 (d, J = 10.5 Hz, 1H), 5.50-5.00 (m, 6H), 3.50 (bs, 1H), 3.40-3.00 (m, 3H), 2.80-1.92 (m, 6H). 1.75 (s, 3H), 1.73 (s, 3H), 1.71 (s, 3H), 1.56 (s, 3H). MS. 849 ($M+Na^+$), 825 (M-1).

***Example 32***

***N-Hydroxysuccinimidyl-8-(Gambogylamido)octanoate***

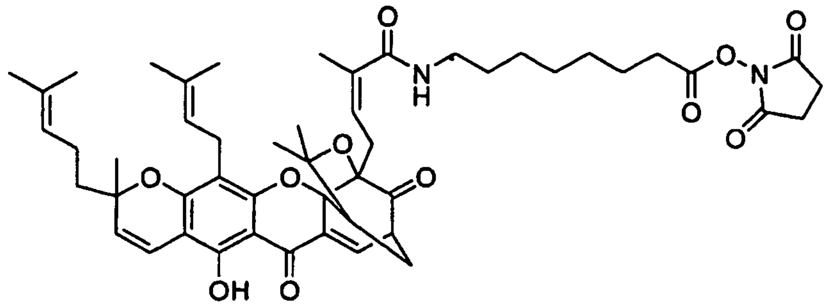

The title compound was prepared by a procedure similar to that of Example 28. $^1$H NMR ($CDCl_3$): 12.70 (s, 1H), 7.55 and 7.51 (d, J = 6.9 Hz, 1H), 6.65 and 6.64 (d, J = 10.2 Hz, 1H), 5.50-5.00 (m, 4H), 4.12 (d, 2H), 3.49 (m, 2H), 3.30 (t, J = 6.6 Hz, 1H), 3.19 (m, 3H), 2.85 (s, 4H), 2.70-2.50 (m, 3H), 2.04 (m, 1H), 1.75 (bs, 3H), 1.74(s, 3H), 1.72 (s, 3H), 1.70 (s, 3H), 1.56 (s, 3H), 1.42 (s, 3H), 1.33 (bs, 3H), 1.30 (bs, 3H). MS. 889 ($M+Na^+$), 865 (M-H).

***Example 33***

***N-Hydroxysuccinimidyl-6-(Gambogylamido)hexanoate***

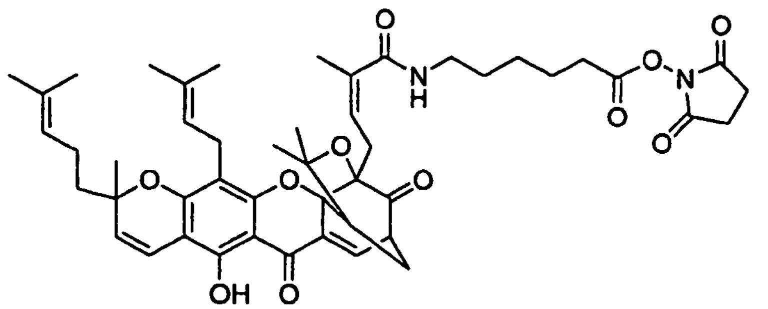

The title compound was prepared by a procedure similar to that of Example 28. $^1$H NMR ($CDCl_3$): 12.70 (s, 1H), 7.56 and 7.52 (d, J = 6.9 Hz, 1H), 6.69 and 6.65(d, J = 10.2 Hz, 1H), 6.59 (t, 1H), 5.60-5.00 (m, 4H), 4.10 (m, 2H), 3.60-3.12 (m, 6H), 2.85 (s, 4H), 2.70-2.50 (m, 3H), 2.35 (m, 1H), 2.04 (m, 1H), 1.90 (m, 4H), 1.73(s, 3H), 1.72 (s, 3H), 1.69 (s, 3H), 1.56 (bs, 6H), 1.44(s, 3H), 1.33(s, 3H), 1.29(s, 3H). MS. 861 ($M+Na^+$), 837 (M-H).

***Example 34***

***N-Hydroxysuccinimidyl-12-(Gambogylamido)dodecanoate***

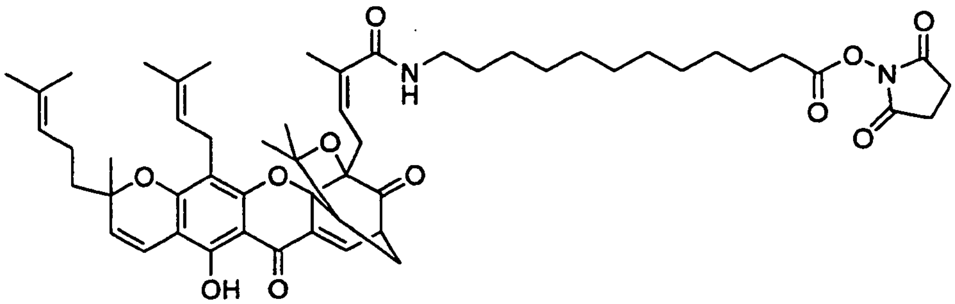

The title compound was prepared by a procedure similar to that of Example 28. $^1$H NMR ($CDCl_3$): 12.70 (s, 1H), 7.55 and 7.51 (d, J = 7.2 Hz, 1H), 6.66 and 6.64(d, J = 9.9 Hz, 1H), 5.47(d, J = 10.5 Hz, 1H), 5.46-5.10 (m, 3H), 4.08 (m, 4H), 3.56-3.40 (m, 4H), 3.18 (m, 2H), 2.60 (t, 1H), 2.83 (s, 4H). MS. 946 (M+Na$^+$), 922 (M-H).

***Example 35***

***10-Methoxy-gambogyl Piperidine***

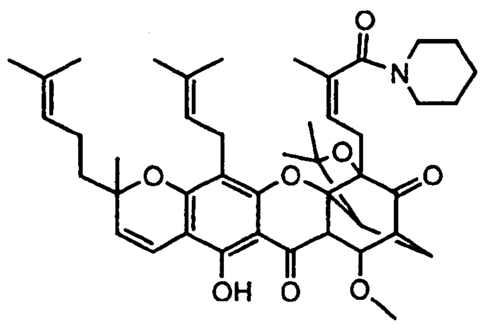

To a solution of gambogyl piperidine (30 mg, 0.043 mmol) in methanol (4 mL) was added sodium methoxide (4.6 mg, 0.086 mmol) and it was stirred at room temperature for 3 h. The reaction was poured into ice water (20 mL), and extracted with ethyl acetate (3x10 mL). The organic extract was dried and concentrated to give crude product, which was purified by chromatography to give the title compound (18 mg, 58%). MS. 726 (M-H$^+$), 750 (M+Na$^+$). $^1$H NMR ($CDCl_3$): 11.98 (s, 1H), 6.65 (d, J = 10.2 Hz, 1H), 5.77 (t, J = 6.6 Hz, 1H), 5.43 (d, J = 10.2 Hz, 1H), 5.07 (m, 2H), 4.33 (d, 1H), 3.60-3.15 (m, 3H), 3.31 (s, 3H), 2.80-2.40 (m, 3H), 1.87 (s, 3H), 1.66 (s, 3H), 1.60 (s, 3H), 1.36 (s, 3H), 1.31 (s, 3H), 1.28 (s, 3H), 1.23 (s, 3H), 1.11 (s, 3H).

***Example 36***

***Gambogyl (2-Dimethylaminoethylamine)***

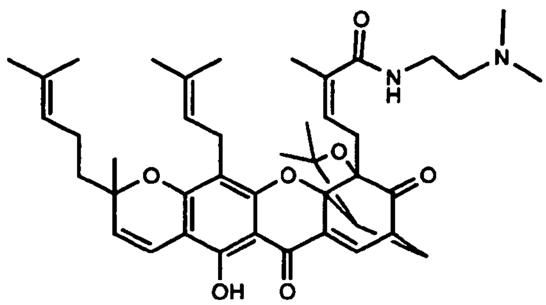

The title compound was prepared by a procedure similar to that of Example 29. MS. 697 (M-H$^-$), 699 (M+H$^+$). $^1$H NMR ($CDCl_3$): 12.90 (bs, 1H), 7.54 (d, J = 6.9 Hz, 1H), 6.68 (d, J = 9.6 Hz, 1H), 6.52 (t, 1H), 5.45 (d, J = 10.2 Hz, 1H), 5.37 (dt, $J_1$ = 8.4 Hz, $J_2$ = 1.5 Hz, 1H), 5.05 (m, 2H), 3.50-3.10 (m,3H), 2.21 (s, 6H), 1.76 (s, 3H), 1.75 (s, 3H), 1.69 (s, 3H), 1.65 (s, 3H), 1.64 (s, 3H), 1.56 (s, 3H), 1.44 (s, 3H), 1.29 (s, 3H).

The following compounds (Examples 37-89) were prepared by a procedure similar to that of Example 9.

**Table I. Examples 37-89**

| Example # | STRUCTURE | MF | MW |
|---|---|---|---|
| 37 | 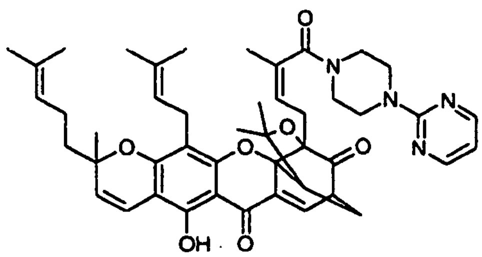 | $C_{46}H_{54}N_4O_7$ | 774.954 |
| 38 | 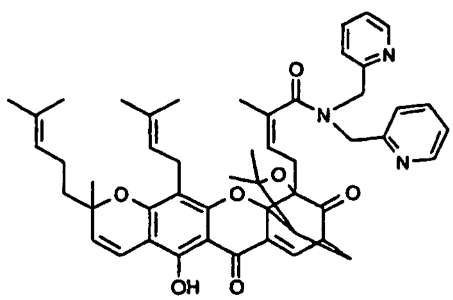 | $C_{50}H_{55}N_3O_7$ | 809.998 |
| 39 | 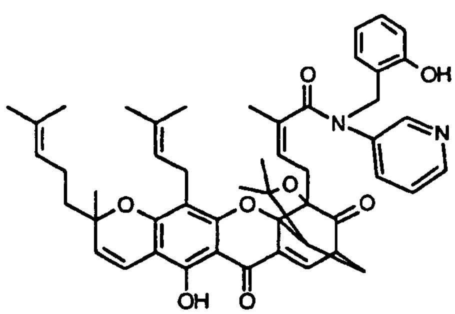 | $C_{50}H_{54}N_2O_8$ | 810.983 |

(continued)

| Example # | STRUCTURE | MF | MW |
|---|---|---|---|
| 40 | 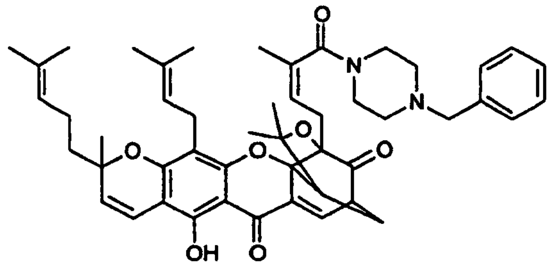 | $C_{49}H_{58}N_2O_7$ | 787.004 |
| 41 | 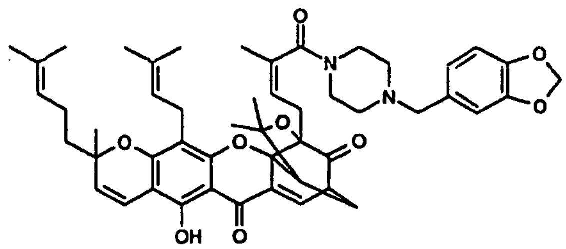 | $C_{50}H_{58}N_2O_9$ | 831.013 |
| 42 | 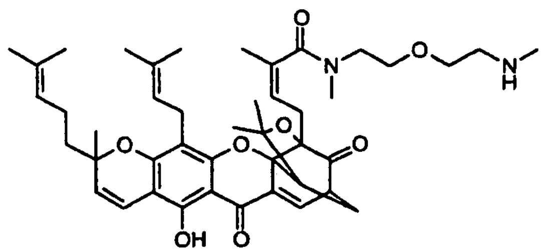 | $C_{44}H_{58}N_2O_8$ | 742.948 |
| 43 | 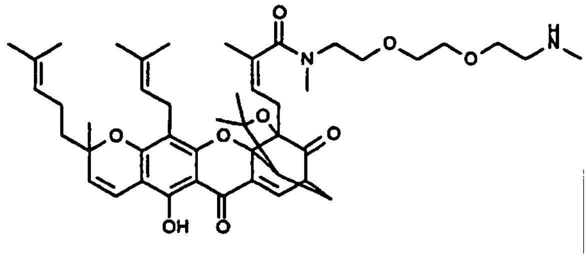 | $C_{46}H_{62}N_2O_9$ | 787.001 |
| 44 | 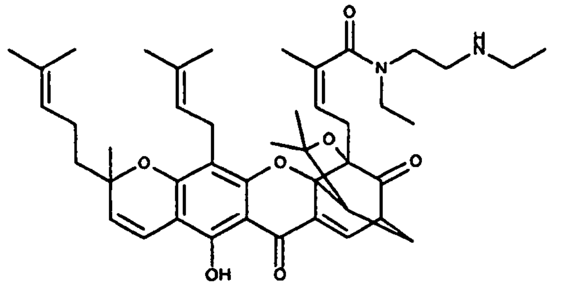 | $C_{44}H_{58}N_2O_7$ | 726.949 |

(continued)

| Example # | STRUCTURE | MF | MW |
|---|---|---|---|
| 45 | 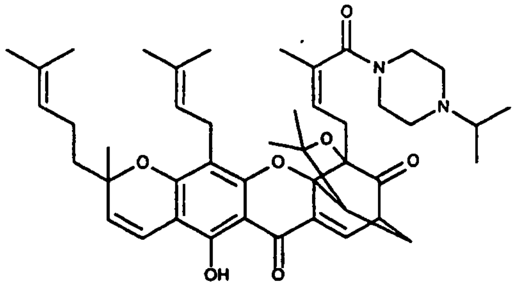 | $C_{45}H_{58}N_2O_7$ | 738.96 |
| 46 | 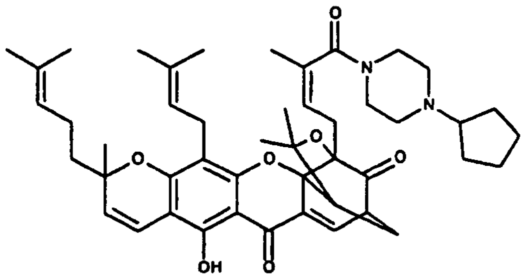 | $C_{47}H_{60}N_2O_7$ | 764.998 |
| 47 | 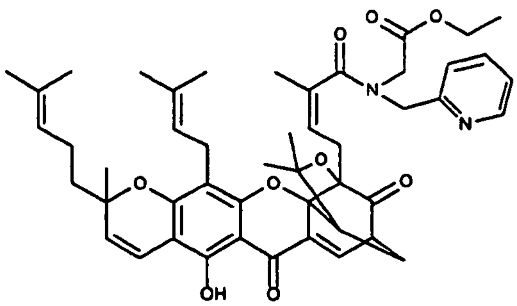 | $C_{48}H_{56}N_2O_9$ | 804.975 |
| 48 | 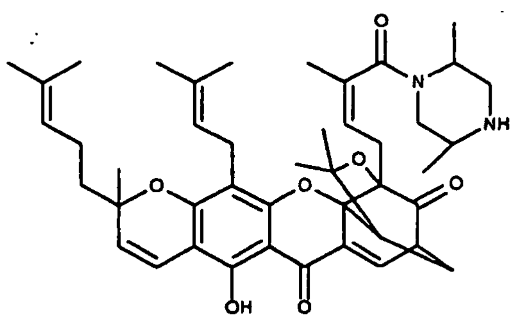 | $C_{44}H_{56}N_2O_7$ | 724.933 |
| 49 | 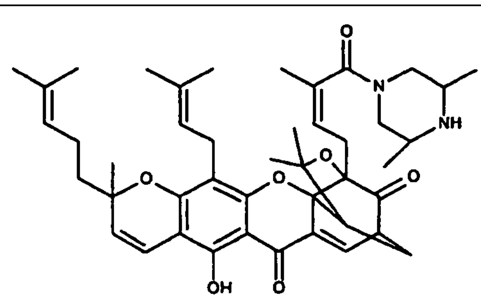 | $C_{44}H_{56}N_2O_7$ | 724.933 |

(continued)

| Example # | STRUCTURE | MF | MW |
|---|---|---|---|
| 50 | 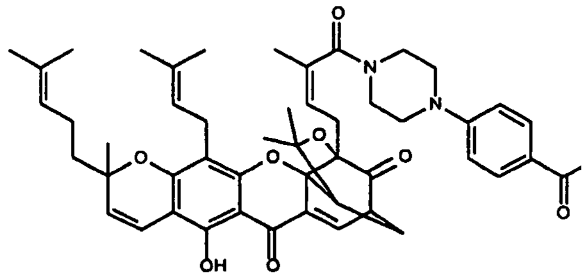 | $C_{50}H_{58}N_2O_8$ | 815.014 |
| 51 | 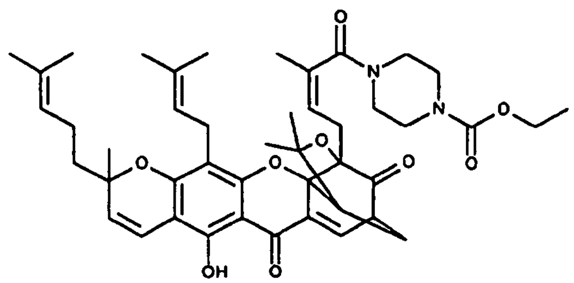 | $C_{45}H_{56}N_2O_9$ | 768.942 |
| 52 | 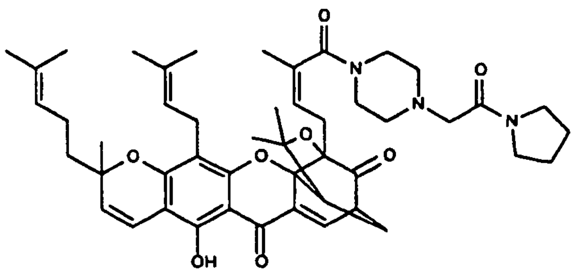 | $C_{48}H_{61}N_3O_8$ | 808.023 |
| 53 | 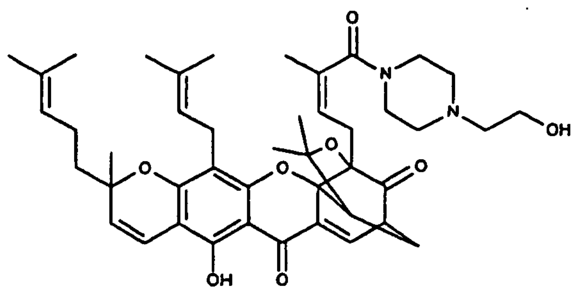 | $C_{44}H_{56}N_2O_8$ | 740.932 |
| 54 | 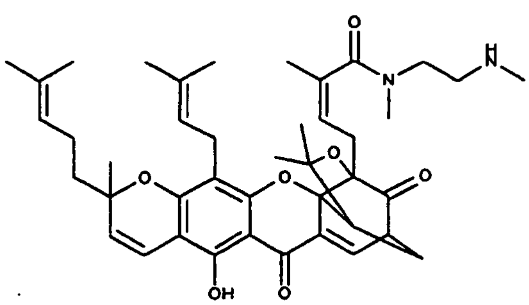 | $C_{42}H_{54}N_2O_7$ | 698.896 |

(continued)

| Example # | STRUCTURE | MF | MW |
|---|---|---|---|
| 55 | 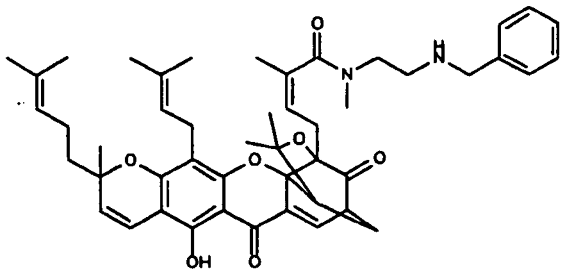 | $C_{48}H_{58}N_2O_7$ | 774.993 |
| 56 | 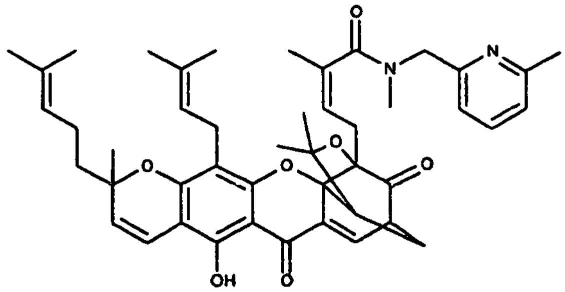 | $C_{46}H_{54}N_2O_7$ | 746.94 |
| 57 | 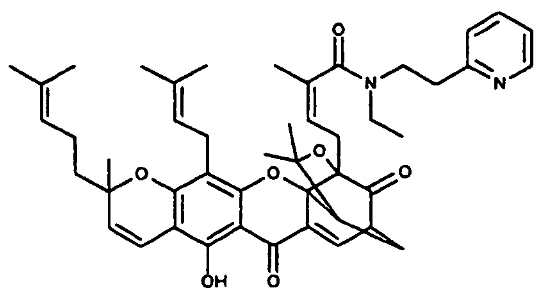 | $C_{47}H_{56}N_2O_7$ | 760.966 |
| 58 | 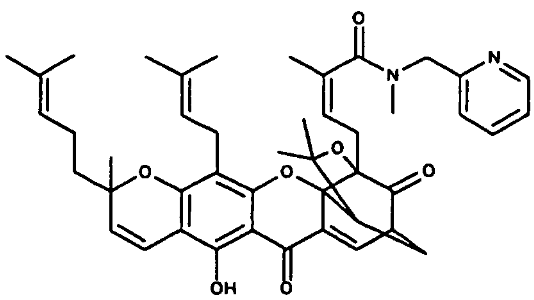 | $C_{45}H_{52}N_2O_7$ | 732.913 |
| 59 | 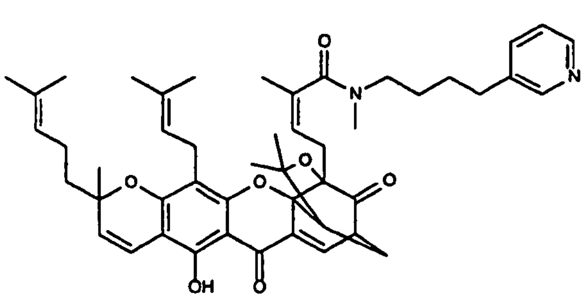 | $C_{48}H_{58}N_2O_7$ | 774.993 |

(continued)

| Example # | STRUCTURE | MF | MW |
|---|---|---|---|
| 60 | 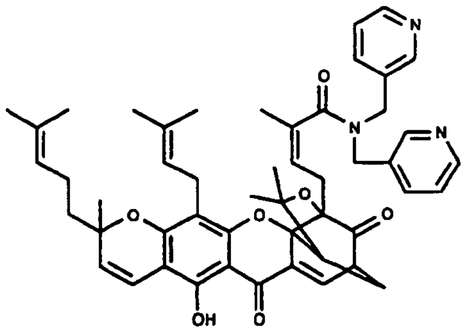 | $C_{50}H_{55}N_3O_7$ | 809.998 |
| 61 | 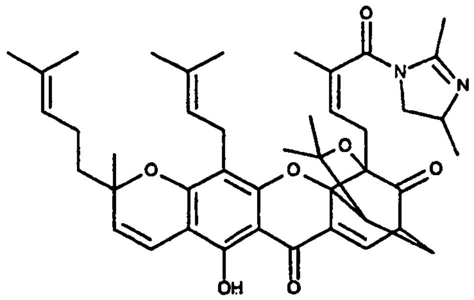 | $C_{43}H_{52}N_2O_7$ | 708.891 |
| 62 | 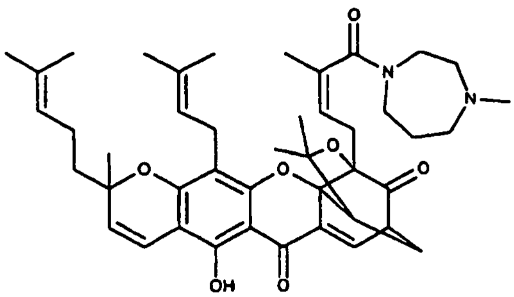 | $C_{44}H_{56}N_2O_7$ | 724.933 |
| 63 | 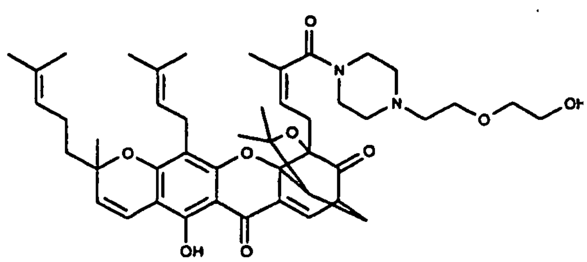 | $C_{46}H_{60}N_2O_9$ | 784.985 |
| 64 | 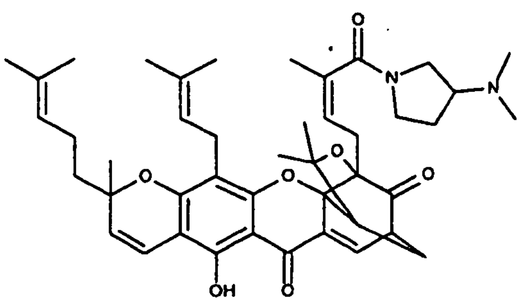 | $C_{44}H_{56}N_2O_7$ | 724.93 |

(continued)

| Example # | STRUCTURE | MF | MW |
|---|---|---|---|
| 65 | 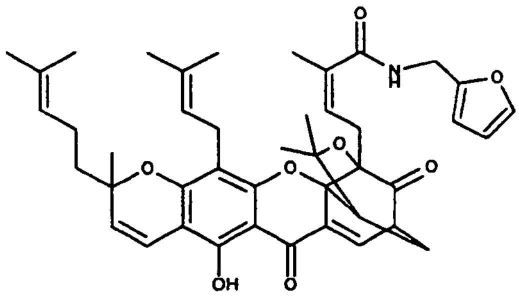 | $C_{43}H_{49}NO_8$ | 707.859 |
| 66 | 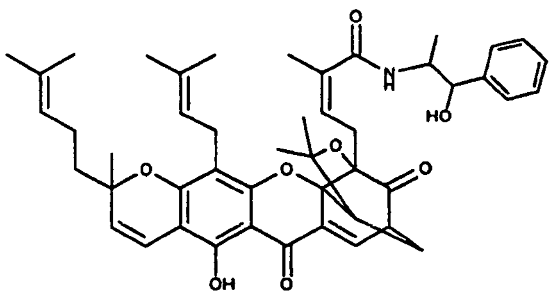 | $C_{47}H_{55}NO_8$ | 761.951 |
| 67 | 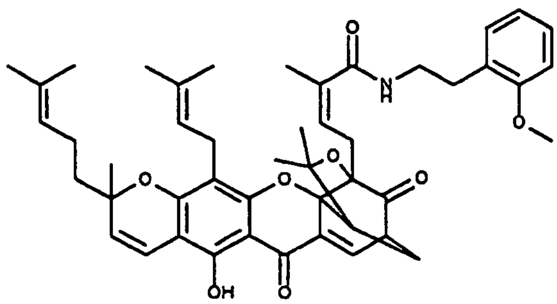 | $C_{47}H_{55}NO_8$ | 761.951 |
| 68 | 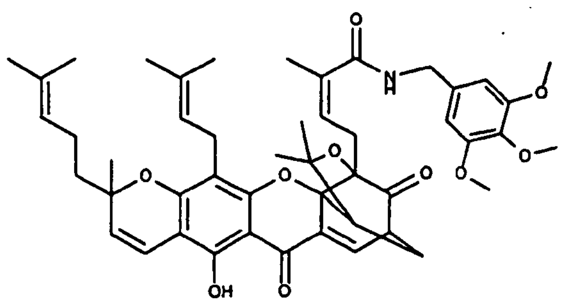 | $C_{48}H_{57}NO_{10}$ | 807.975 |
| 69 | 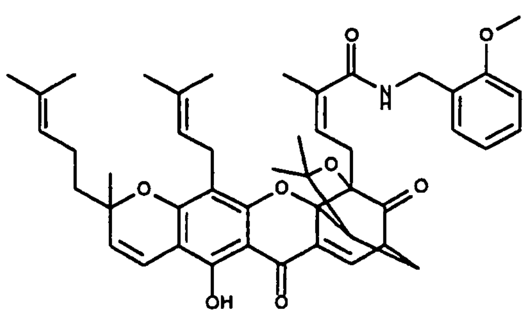 | $C_{46}H_{53}NO_8$ | 747.924 |

(continued)

| Example # | STRUCTURE | MF | MW |
|---|---|---|---|
| 70 | 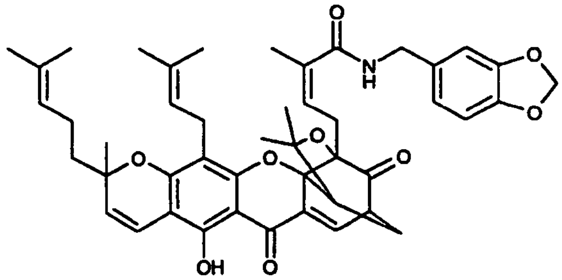 | $C_{46}H_{51}NO_9$ | 761.907 |
| 71 | 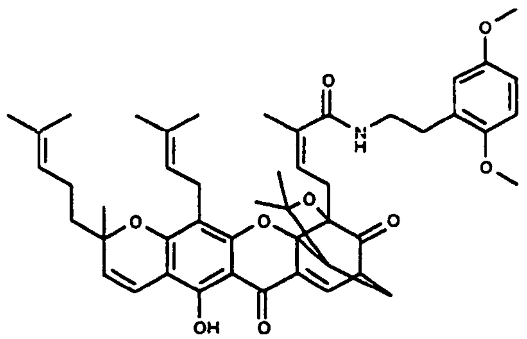 | $C_{48}H_{57}NO_9$ | 791.976 |
| 72 | 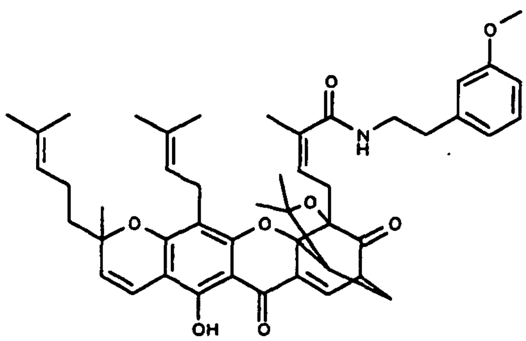 | $C_{47}H_{55}NO_8$ | 761.951 |
| 73 | 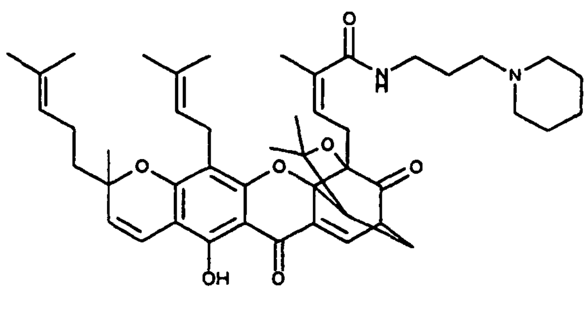 | $C_{46}H_{60}N_2O_7$ | 752.987 |
| 74 | 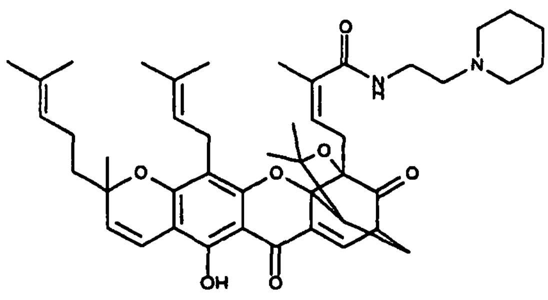 | $C_{45}H_{58}N_2O_7$ | 738.96 |

(continued)

| Example # | STRUCTURE | MF | MW |
|---|---|---|---|
| 75 | 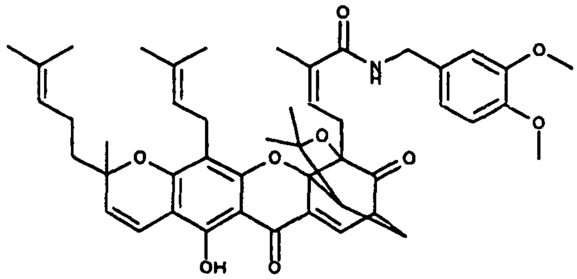 | $C_{47}H_{55}NO_9$ | 777.95 |
| 76 | 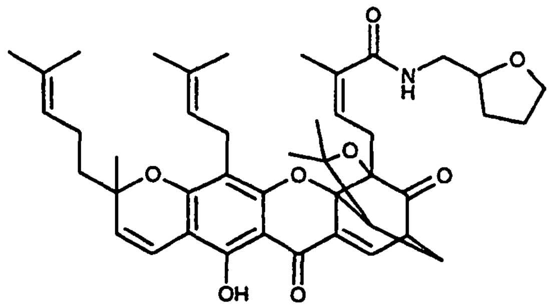 | $C_{43}H_{53}NO_8$ | 711.891 |
| 77 | 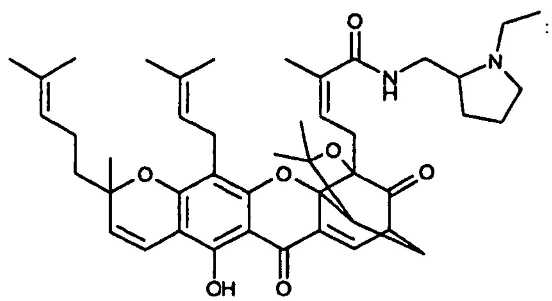 | $C_{45}H_{58}N_2O_7$ | 738.96 |
| 78 | 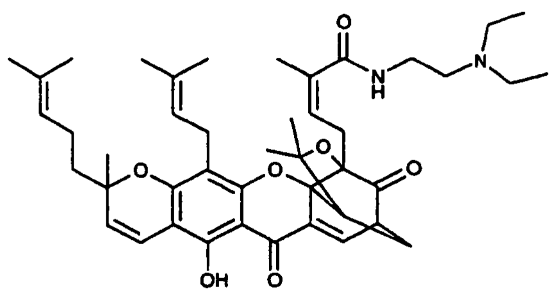 | $C_{44}H_{58}N_2O_7$ | 726.949 |
| 79 | 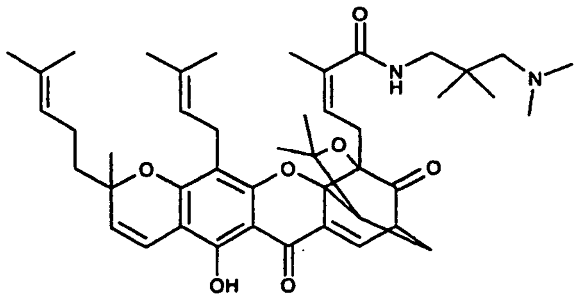 | $C_{45}H_{60}N_2O_7$ | 740.976 |

(continued)

| Example # | STRUCTURE | MF | MW |
|---|---|---|---|
| 80 | 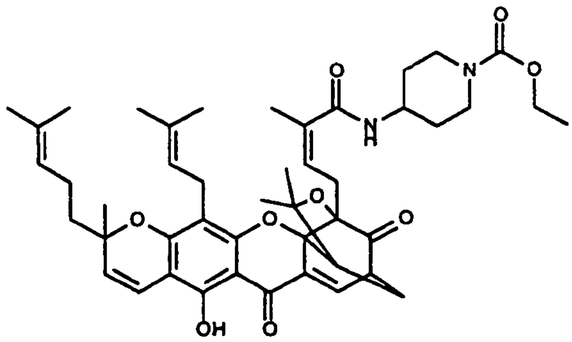 | $C_{46}H_{58}N_2O_9$ | 782.969 |
| 81 | 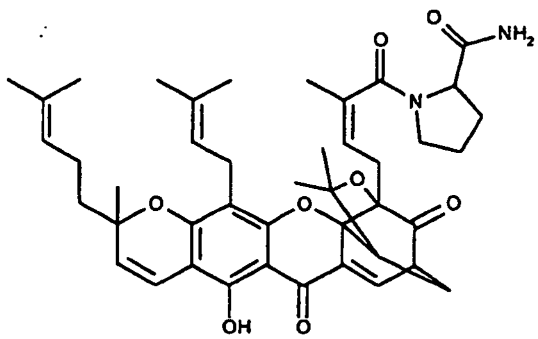 | $C_{43}H_{52}N_2O_8$ | 724.89 |
| 82 | 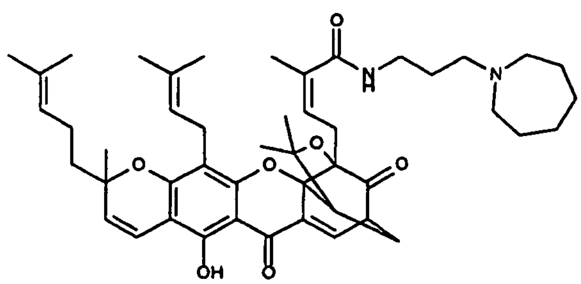 | $C_{47}H_{62}N_2O_7$ | 767.014 |
| 83 | 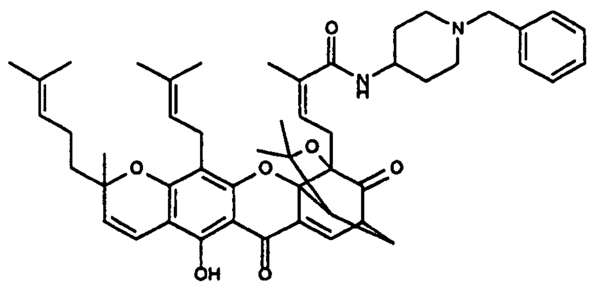 | $C_{50}H_{60}N_2O_7$ | 801.031 |
| 84 | 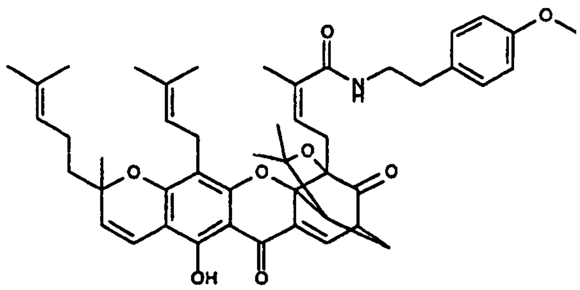 | $C_{47}H_{55}NO_8$ | 761.951 |

(continued)

| Example # | STRUCTURE | MF | MW |
|---|---|---|---|
| 85 | 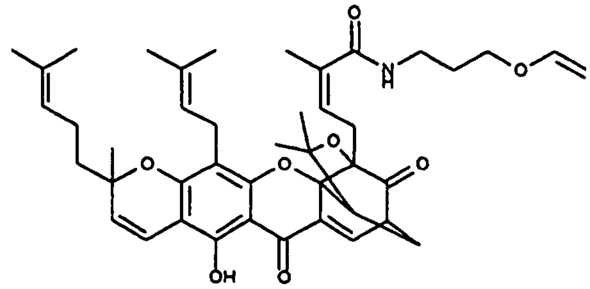 | $C_{43}H_{53}NO_8$ | 711.891 |
| 86 | 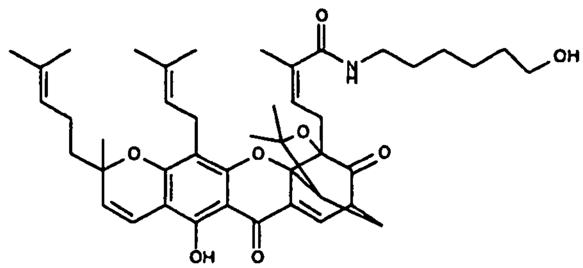 | $C_{44}H_{57}NO_8$ | 727.933 |
| 87 | 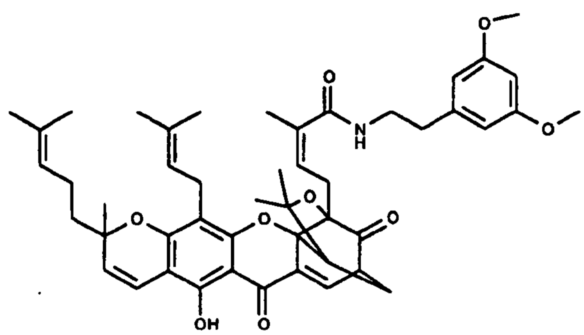 | $C_{48}H_{57}NO_9$ | 791.976 |
| 88 | 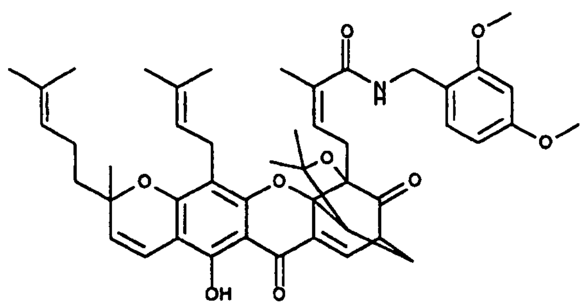 | $C_{47}H_{55}NO_9$ | 777.95 |
| 89 | 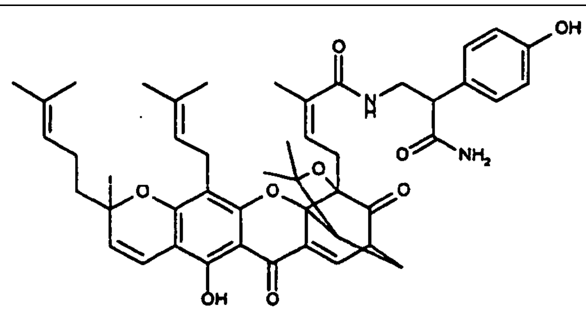 | $C_{47}H_{54}N_2O_9$ | 790.949 |

***Example 90***

***Identification Of Gambogic Acid And Analogs As Antineoplastic Compounds That Are Caspase Cascade Activators***

Human breast cancer cell lines T-47D and ZR-75-1, human prostate cancer cell line PC-3, human leukemia cancer cell line HL-60 and human non-transformed fibroblast cell line MRC-5 cells were grown according to media component mixtures designated by The American Type Culture Collection + 10% FCS (Life Technologies, Inc.), in a 5% $CO_2$ -95% humidity incubator at 37° C. T-47D, ZR-75-1 and PC-3 cells were maintained at a cell density between 30 and 80% confluency and for HL-60 at a cell density of 0.1 to 0.6 x $10^6$ cells/ml. Cells were harvested at 600xg and resuspended at 0.65 x $10^6$ cells/ml into appropriate media + 10% FCS. An aliquot of 45 μl of cells was added to a well of a 96-well microtiter plate containing 5 μl of a 10% DMSO in RPMI-1640 media solution containing 1.6 to 100 μM gambogic acid or other test compound (0.16 to 10 μM final). An aliquot of 45 μl of cells was added to a well of a 96-well microtiter plate containing 5 μl of a 10% DMSO in RPMI-1640 media solution without test compound as the control sample. The samples were mixed by agitation and then incubated at 37° C for 24 h in a 5% $CO_2$-95% humidity incubator. After incubation, the samples were removed from the incubator and 50 μl of a solution containing 20 μM of *N*-(Ac-DEVD)-*N'*-ethoxycarbonyl-R110 fluorogenic substrate (SEQ ID NO:1) (Cytovia, Inc.; WO99/18856), 20% sucrose (Sigma), 20 mM DTT (Sigma), 200 mM NaCl (Sigma), 40 mM Na PIPES buffer pH 7.2 (Sigma), and 500 μg/ml lysolecithin (Calbiochem) was added. The samples were mixed by agitation and incubated at room temperature. Using a fluorescent plate reader (Model 1420 Wallac Instruments), an initial reading (T = 0) was made approximately 1- 2 min after addition of the substrate solution, employing excitation at 485 nm and emission at 530 nm, to determine the background fluorescence of the control sample. After the 3 h incubation, the samples were read for fluorescence as above (T = 3 h).

Calculation:

The Relative Fluorescence Unit values (RFU) were used to calculate the sample readings as follows:

$$\text{RFU}_{(T=3hr)} - \text{Control RFU}_{(T=0)} = \text{Net RFU}_{(T=3hr)}$$

The activity of caspase cascade activation was determined by the ratio of the net RFU value for gambogic acid or other test compound to that of control samples. The $EC_{50}$ (nM) was determined by a sigmoidal dose-response calculation (Prism 2.0, GraphPad Software Inc.). The caspase activity (Ratio) and potency ($EC_{50}$) are summarized in Table II:

**Table II. Caspase Activity and Potency**

| Example # | T-47D Ratio EC50 (nM) | | ZR-75-1 Ratio EC50 (nM) | | PC-3 Ratio EC50 (nM) | | HL-60 Ratio EC50 (nM) | | MRC-5 Ratio EC50 (nM) | |
|---|---|---|---|---|---|---|---|---|---|---|
| **1** | 13.6 | 560 | 11.9 | 1400 | 2.1 | 1500 | 6.3 | 400 | 17.8 | 1410 |
| **4** | 16.8 | 484 | 14.9 | 1640 | 3.9 | 1330 | 7.7 | 339 | 27.8 | 501 |
| **5** | 13.9 | 210 | 14.3 | 783 | 2.7 | 900 | 5.2 | 200 | 12.6 | 631 |
| **6** | 12.0 | 2800 | ND | ND | 4.5 | 5000 | ND | ND | ND | ND |
| **2** | 16.9 | 310 | 14.2 | 1160 | 2.9 | 1350 | 5.6 | 340 | 22.4 | 1260 |
| **3** | 7.0 | 1000 | ND | ND | 2.9 | 1700 | ND | ND | ND | ND |
| **7** | 14.4 | 830 | 13.4 | 1650 | 2.3 | 1700 | ND | ND | 9.4 | 1200 |
| **9** | 11.7 | 990 | 12.8 | 2050 | 3.1 | 5900 | ND | ND | 11.4 | 1900 |
| ND = not determined | | | | | | | | | | |

Thus, gambogic acid and its derivatives and analogs are identified as potent caspase cascade activators and antineoplastic compounds in this assay.

***Example 91***

***Identification Of Gambogic Acid And Analogs As Antineoplastic Compounds That Exhibit Inhibition Of Cell Proliferation ($GI_{50}$) And Cell Death ($LC_{50}$)***

T-47D, ZR-75-1, PC-3, human prostate cancer cell line DU-145, human non-small cell lung cancer cell line A-549, human small cell lung cancer cell line SHP-77, HL-60 and MRC-5 cells were grown and harvested as in Example 90. An aliquot of 90 μl of cells (2. 2 x $10^4$ cells/ml) was added to a well of a 96-well microtiter plate containing 10 μl of a 10% DMSO in RPMI-1640 media solution containing 1 nM to 100 μM gambogic acid or other test compound (0.1 nM to 10 μM final). An aliquot of 90 μl of cells was added to a well of a 96-well microtiter plate containing 10 μl of a 10% DMSO in RPMI-1640 media solution without compound as the control sample for maximal cell proliferation ($A_{max}$). The samples were mixed by agitation and then incubated at 37° C for 48 h in a 5% $CO_2$-95% humidity incubator. After incubation, the samples were removed from the incubator and 20 μl of CellTiter 96 $AQ_{UEOUS}$ One Solution Cell Proliferation™ reagent (Promega) was added. The samples were mixed by agitation and incubated at 37° C for 2-4 h in a 5% $CO_2$-95% humidity incubator. Using an absorbance plate reader (Model 1420 Wallac Instruments), an initial reading (T=0) was made approximately 1- 2 min. after addition of the solution, employing absorbance at 490 nm. This determines the possible background absorbance of the test compounds. No absorbance for gambogic acid or its analogs or derivatives was found at 490 nm. After the 2-4 h incubation, the samples were read for absorbance as above ($A_{Test}$).

Baseline for $GI_{50}$ (dose for 50% inhibition of cell proliferation) and $LC_{50}$ (dose for 50% cell death) of initial cell numbers was determined by adding an aliquot of 90 μl of cells or 90 μl of media, respectively, to wells of a 96-well microtiter plate containing 10 μl of a 10% DMSO in RPMI-1640 media solution. The samples were mixed by agitation and then incubated at 37° C for 0.5 h in a 5% $CO_2$-95% humidity incubator. After incubation, the samples were removed from the incubator and 20 μl of CellTiter 96 $A_{QUEOUS}$ One Solution Cell Proliferation™ reagent (Promega) was added. The samples were mixed by agitation and incubated at 37° C for 2-4 h in a 5% $CO_2$-95% humidity incubator. Absorbance was read as above, ($A_{T=0}$) defining absorbance for initial cell number used as baseline in $GI_{50}$ determinations and ($A_{min}$) defining absorbance for media alone used as baseline in $LC_{50}$ determinations.

Calculation:

$GI_{50}$ (dose for 50% inhibition of cell proliferation)

$$50 = 100 \times [(A_{Test} - A_{T=0}) / (A_{max} - A_{T=0})]$$

$LC_{50}$ (dose for 50% cell death)

$$50 = 100 \times [(A_{Test} - A_{min}) / (A_{T=0} - A_{min})]$$

The $GI_{50}$ (nM) and $LC_{50}$ (nM) are summarized in Table III:

**Table III. $GI_{50}$ and $LC_{50}$ in Cancer Cells**

| Cell lines | Gambogic acid $GI_{50}$ $LC_{50}$ (nM) | | Methyl Gambogate $GI_{50}$ $LC_{50}$ (nM) | | Gambogyl Piperidine $GI_{50}$ $LC_{50}$ (nM) | | Methyl-6-Methoxy-gambogate $GI_{50}$ $LC_{50}$ (nM) | | Gambogenic Acid $GI_{50}$ $LC_{50}$ (nM) | | Gambogenin $GI_{50}$ $LC_{50}$ (nM) | |
|---|---|---|---|---|---|---|---|---|---|---|---|---|
| **T-47D** | 65 | 450 | 40 | 50 | 50 | 50 | 50 | 80 | 500 | 500 | 500 | 500 |
| **ZR-75-1** | 400 | 500 | 300 | 500 | 300 | 500 | 400 | 500 | ND | ND | ND | ND |
| **PC-3** | 500 | 700 | 500 | 500 | 500 | 500 | 500 | 500 | 3000 | 5000 | 5000 | 5000 |
| **DU-145** | 500 | 800 | 500 | 500 | 500 | 500 | 500 | 900 | 600 | 5000 | 2000 | 5000 |
| **A-549** | 800 | 5000 | 500 | 2000 | 800 | 5000 | 500 | 900 | ND | ND | ND | ND |
| **SHP-77** | 500 | 500 | 500 | 500 | 500 | 500 | 500 | 500 | ND | ND | ND | ND |
| **HL-60** | 500 | 700 | 50 | 500 | 100 | 800 | 100 | 800 | ND | ND | ND | ND |
| **MRC-5** | 400 | 500 | 200 | 500 | 800 | 800 | 500 | 500 | 500 | 5000 | 3000 | 5000 |
| ND = not determined | | | | | | | | | | | | |

Thus, gambogic acid and its analogs and derivatives are identified as potent antineoplastic compounds that both inhibit cell proliferation ($GI_{50}$) and elicit cell death ($LC_{50}$).

***Example 92*** (Comparative Example**)**

***Identification Of Vinblastine, Cisplatin, 5-Fluorouracil, Taxol, Camptothecin, Doxorubicin, Etoposide And Methobexate As Conventional Antineoplastic Agents That Are Not Efficient Caspase Cascade Activators In Solid Tumors***

T-47D, ZR-75-1, PC-3 and HL-60 cells were grown and harvested as in Example 90. An aliquot of 45 μl of cells was added to a well of a 96-well microtiter plate containing 5 μl of a 10% DMSO in RPMI-1640 media solution containing 100 μM of test compounds (10 μM final). An aliquot of 45 μl of cells was added to a well of a 96-well microtiter plate containing 5 μl of a 10% DMSO in RPMI-1640 media solution without test compound as the control sample. The samples were mixed by agitation and then incubated at 37° C for 24 h in a 5% $CO_2$-95% humidity incubator. After incubation, the samples were removed from the incubator and 50 μl of a solution containing 20 μM of *N*-(Ac-DEVD)-*N*'-ethoxycarbonyl-R110 fluorogenic substrate (SEQ ID NO:1) (Cytovia, Inc.), 20% sucrose (Sigma), 20 mM DTT (Sigma), 200 mM NaCl (Sigma), 40 mM Na PIPES buffer pH 7.2 (Sigma), and 500 μg/ml lysolecithin (Calbiochem) was added. The samples were mixed by agitation and incubated for 3 h at room temperature. Using a fluorescent plate reader (Model 1420 Wallac Instruments), an initial reading (T=0) was made approximately 1-2 min. after addition of the substrate solution, employing excitation at 485 nm and emission at 530 nm, to determine the background fluorescence of the control sample. After the 3 h incubation, the samples were read for fluorescence as above (T = 3 h).

Calculation:

The Relative Fluorescence Unit values (RFU) were used to calculate the sample readings as follows:

$$\text{RFU}_{(T=3hr)} - \text{Control RFU}_{(T=0)} = \text{Net RFU}_{(T=3hr)}$$

The activity in caspase cascade activation was determined by the ratio of the net RFU value for test compounds to that of control samples. A ratio around 1 indicates that the compound is not an efficient caspase cascade activator. The ratios are summarized in Table IV.

**Table IV. Activity of Known Antineoplastic Compound as Caspase Cascade Activators**

| | *Cell lines* | |
|---|---|---|
| | **T-47D** | **PC-3** |
| Vinblastine | 0.9 | 0.8 |
| Cisplatin | 1.1 | 0.9 |
| 5-fluorouracil | 0.8 | 0.7 |
| Taxol | 0.9 | 0.7 |
| Camptothecin | 0.7 | 0.6 |
| Doxorubicin | 1.3 | 1.1 |
| Etoposide | 1.0 | 0.8 |
| Methotrexate | 0.8 | 0.7 |

Thus, vinblastine, cisplatin, 5-fluorouracil, taxol, camptothecin, doxorubicin, etoposide and methotrexate are identified as known antineoplastic compounds that are not caspase cascade activators in this assay.

***Example 93*** (Comparative Example)

***Morphological Change of T47D Cells Treated with Gambogic Acid***

Cells undergoing apoptosis typically demonstrate several characteristic morphological changes, including rounding and blebbing. In addition, apoptotic adherent cells in culture lose their ability to remain attached to the culture dish. The ability of gambogic acid to trigger these morphological changes in T47D cells was investigated.

60 mm culture dishes were seeded with 750,000. T47D cells and the cultures were incubated under normal growth conditions (complete medium with 10% FBS) for 24 h. The cells were then treated with 2.5 μM of gambogic acid and further incubated under normal growth conditions for 2 or 6 h. Morphological changes were documented by photographing the cells under phase contrast illumination.

As shown in Figs. 1A-C, T47D cells incubated with vehicle (Control) are phase-dark and show a normal, flat morphology (Fig. 1A). After 2 h of treatment with gambogic acid, many of the cells have taken on a rounded, phase-bright morphology (Fig. 1B). By 6 h of treatment with gambogic acid, most of the cells in the culture are rounded up and are beginning to detach from the dish (Fig. 1C). At this timepoint, many of the cells also show evidence of blebbing. Based on these data, it was concluded that gambogic acid induces apoptotic morphological changes in T47D cells.

***Example 94*** (Comparative Example)

***Gambogic Acid Induces Nuclear Fragmentation in T47D Breast Cancer Cells***

T47D cells were grown and plated as described in Example 90. The cells were treated with 10 μM of gambogic acid and the plate was incubated for up to 24 h at 37˚C in a 5% $CO_2$-95% humidity incubator. At 24 h, the cells were incubated with a live cell nucleic acid stain, Syto16 (Molecular Probes) which stains DNA. After 2 washes with PBS, cells were examined under a fluorescence microscope. The nuclear staining of untreated cells showed normal nuclei (Fig. 2A) whereas the gambogic acid treated cells showed condensed and fragmented nuclei in a large population of the cells (Fig. 2B). Nuclear fragmentation is a clear indicator of cellular apoptosis.

***Example 95*** (Comparative Example)

***Gambogic Acid Induces Characteristic Apoptotic Morphology in Jurkat Cells***

Jurkat T leukemia cells were grown in RPMI 1640 media (Life Technologies, Inc.) + 10% FCS (Sigma Chemical Company) in a 5% $CO_2$-95% humidity incubator at 37˚ C, and maintained at a cell density between 4 and 8 x $10^5$ cells/ml. Cells were harvested at 200xg and resuspended at 1-2 x $10^6$ cells/ml into RPMI 1640 media + 10% FCS, and 3 ml of the cells was dispensed in each of three wells of a 6-well plate. One of the wells was treated with 10 μM caspase inhibitor cbz-Val-Asp-fink (Cytovia, Inc.; WO99/18781) and the plate was incubated at 37˚ C in a 5% $CO_2$-95% humidity incubator for 1 h prior to addition of gambogic acid. The wells with and without the caspase inhibitor were treated with 10 μM gambogic acid. The third well was treated with solvent (control cells). The plate was incubated at 37˚ C in a 5% $CO_2$-95% humidity incubator.

At 30 min. after addition of gambogic acid an aliquot of cells from each well was taken into the capillary slides and observed under a phase-contrast microscope.

The control cell samples showed normal cell morphology (Fig. 3A) whereas after 30 min. treatment with gambogic acid the cells showed blebbing and cellular fragmentation (Fig. 3B), hallmarks of apoptosis. The presence of caspase inhibitor prevented the morphological changes (Fig. 3C), indicating that the changes are due to activation of caspases in the cell.

***Example 96*** (Comparative Example)

***Activation of Caspases by Gambogic Acid in T47D Breast Cancer Cell Line and in Normal Fibroblasts MRC-5***

T47D cells and MRC-5 cells were maintained and harvested as described in Example 90. An aliquot of 45 μl of cells was added to each well of a 96-well microtiter plate. To determine the dose response of gambogic acid for inducing caspase activity, 5 μl of 20 μM gambogic acid in RPMI media was added to wells in triplicates. Two-fold serial dilutions were made for the lower concentrations. After incubation for 2 h, the samples were removed from the 5% $CO_2$-95% humidity incubator and caspase activity was determined by addition of a fluorogenic substrate as described in Example 90.

The dose response (Fig. 4) indicated that the human breast cancer cell line T47D is more sensitive to induction

of caspase activity than a normal fibroblast cell line MRC-5 by gambogic acid. Therefore, there is a potential therapeutic index with gambogic acid treatment.

***Example 97*** (Comparative Example)

***Gambogic Acid Induces Caspase Activity in a Variety of Solid Tumor Cell Lines Which Is Inhibited by a Caspase Inhibitor***

T47D, ZR-75, PC3, SHP-77 and A-549 cells were maintained and harvested as described in Example 90. Cells were added to 96-well plates as described in Example 90. The cells were treated with 10 μM gambogic acid, in the presence and in the absence of 10 μM caspase inhibitor cbz-Val-Asp-fink (Cytovia, Inc.; WO99/18781). The plate was incubated up to 24 h at 37° C in a 5% $CO_2$-95% humidity incubator. Caspase activity was determined by addition of a fluorogenic substrate as described in Example 90.

Gambogic acid induced caspase activity in a ratio of greater than 2.5 (+) above untreated cell levels in all the tested cancer cell lines (Table IV). The caspase activity detected was inhibited by the caspase inhibitor (+), confirming that the fluorescent signal was due to caspase activity.

**Table IV. Gambogic Acid as Caspase Inducers in Solid Tumor Cells**

| ***Cell line*** | **Caspase activity** | **Inhibition of caspase activity by Inhibitor** |
|---|---|---|
| **T47D** | + | + |
| **ZR-75** | + | + |
| **PC-3** | + | + |
| **SHP-77** | + | + |
| **A-549** | + | + |

***Example 98*** (Comparative Example)

***Induction of PARP Cleavage by Gambogic Acid in Human Tumor Cells***

Cleavage of the enzyme poly(ADP)ribose polymerase (PARP) by caspase-3 and related proteases is considered to be one of the molecular hallmarks of caspase-mediated apoptosis. Therefore, the ability of gambogic acid to induce PARP cleavage in four different human tumor cell lines (Jurkat cells, HL-60 cells, T47D cells and PC3 cells) was determined.

Cells were cultured in complete growth medium containing 10% FBS and treated with gambogic acid at concentrations of 2.5 μM or 5 μM for 2 to 4 h. Control cultures were treated with a drug vehicle (DMSO), or the well-characterized apoptosis inducer, staurosporine. At the end of the apoptosis induction period, the cells were harvested, washed once with PBS, quick-frozen on dry ice, and stored at -80° C. The cells were then lysed in a standard immunoblotting lysis buffer and samples of the lysates were electrophoresed on 4% to 20% gradient polyacrylamide gels. The proteins in the gels were then transferred to PVDF membranes and probed with a commercially-available rabbit polyclonal antibody to PARP.

Figs. 5A-D illustrate the results of these experiments. A 2 h treatment with 2.5 μM gambogic acid induced almost complete PARP cleavage in both Jurkat cells (Fig. 5A) and HL-60 cells (Fig. 5B). 2.5 μM gambogic acid was as effective as 1 μM staurosporine, one of the most potent apoptosis inducers known. There was no PARP cleavage in cells treated with drug vehicle (DMSO) or another inactive control.

Fig. 5C shows the effect of gambogic acid on PARP cleavage in T47D cells. Within 2 h of treatment, using 2.5 μM gambogic acid, moderate induction of PARP cleavage is observed; almost complete PARP cleavage is observed with 5 μM gambogic acid. Within 4 h of treatment, both concentrations of gambogic acid give almost complete PARP cleavage. Under the same conditions, no cleavage of PARP was observed for cells treated with 1 μM staurosporine.

PC3 cells, a human prostate cancer cell line, were more resistant to the induction of PARP cleavage by gambogic acid (Fig. 5D). Within 2 h of treatment, neither concentration (2.5 μM and 5 μM) of drug was effective. Within 4 h of treatment, a moderate amount of PARP cleavage product could be observed with the highest dose of gambogic acid (5 μM). Under the same conditions, no cleavage of PARP was observed for cells treated with 1 μM staurosporine.

Based on these experiments, it was concluded that gambogic acid triggers PARP cleavage in all four human tumor cell lines tested. These results indicate that gambogic acid is an effective inducer of caspase-mediated apoptosis

in tumor cells under normal growth conditions.

## Claims

**1.** Use of a compound having one of the Formulae I-III:

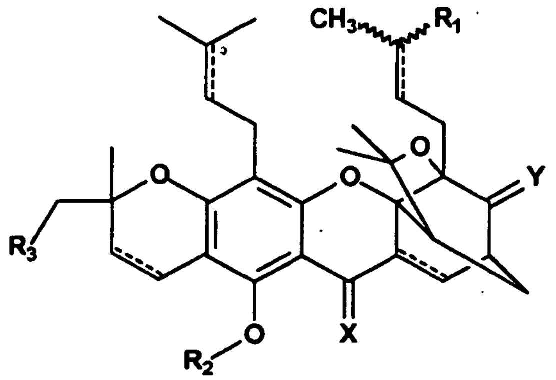

(I)

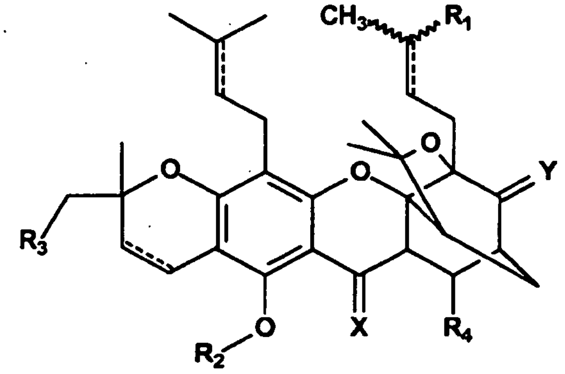

(II)

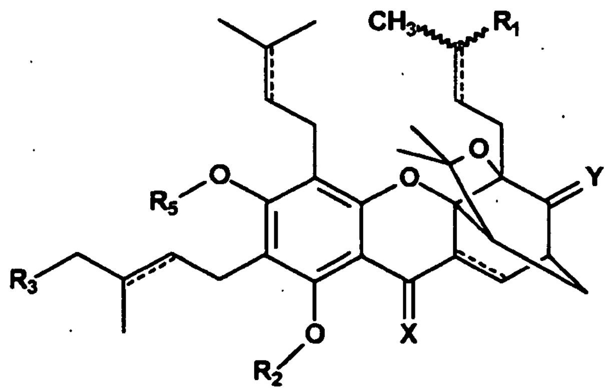

(III)

or a pharmaceutically acceptable salt or prodrug thereof, wherein:

the dotted lines are single bonds, double bonds or epoxy groups;
X together with the attached carbon is a carbonyl;
Y together with the attached carbon is a carbonyl;
$R_1$ is acyl ($R_aCO$), optionally substituted alkoxycarbonyl ($R_aOCO$), optionally substituted alkylthiocarbonyl, optionally substituted aminocarbonyl (carbamyl, $R_bR_cNCO$) or hydroxyaminocarbonyl, where $R_a$ is optionally substituted $C_{1-10}$ alkyl, optionally substituted aryl, N-succinimidyl or optionally substituted $C_{6-14}$ aryl($C_{1-10}$)alkyl group; $R_b$ and $R_c$ are independently hydrogen, optionally substituted heteroalkyl, optionally substituted $C_{1-10}$ alkyl, optionally substituted aryl, optionally substituted heteroaryl or optionally substituted $C_{6-14}$ aryl($C_{1-10}$)alkyl groups; or $R_b$ and $R_c$ may be taken together with the attached N to form an optionally substituted, saturated or partially saturated 5-7 membered heterocyclo group;
$R_2$ is hydrogen, optionally substituted alkyl, acyl ($R_aCO$), carbamyl ($R_bR_cNCO$) or sulfonyl ($R_dSO_2$), where $R_a$,

$R_b$, and $R_c$ are defined above; $R_d$ is hydrogen, optionally substituted $C_{1-10}$ alkyl, optionally substituted aryl, or optionally substituted $C_{6-14}$ aryl($C_{1-10}$)alkyl groups;
$R_3$ is hydrogen or prenyl;
$R_4$ is hydrogen, halogen, hydroxy, optionally substituted alkyl, cycloalkyl, alkoxy, alkylthio or amino; and
$R_5$ is hydrogen, optionally substituted alkyl or acyl ($R_aCO$), carbamyl ($R_bR_cNCO$) or sulfonyl ($R_dSO_2$), where $R_a$, $R_b$, $R_c$ and $R_d$ are defined above;
for the manufacture of a medicament for treating cancer in an animal;

wherein said prodrug is:

a) an ester of a hydroxy containing compound obtained by condensation with a $C_{1-4}$ carboxylic acid, $C_{3-6}$ dioic acid, or anhydride thereof; or
b) an imine of an amino containing compound obtained by condensation with a $C_{1-4}$ aldehyde or ketone.

2. The use of claim 1, wherein the dotted lines between C-9 and C-10 of a compound of Formula I or III represent a double bond, $R_4$ is not a cycloalkyl group, the other dotted lines are not epoxy groups, and $R_b$ and $R_c$ are not heteroalkyl groups.

3. The use according to claim 1, wherein $R_1$ is acetyl, propionyl, methoxycarbonyl, ethoxycarbonyl, methylthiocarbonyl, ethylthiocarbonyl, butylthiocarbonyl, dimethylcarbamyl, diethylcarbamyl; N-piperidinylcarbonyl, N-methyl-N'-piperazinylcarbonyl, 2(dimethylamino)-ethylcarbamyl or N-morpholinylcarbonyl, and the dotted lines represent double bonds.

4. The use according to claim 1, wherein $R_2$ is hydrogen, formyl, acetyl, dimethylcarbamyl, diethylcarbamyl, 2-(dimethylamino)ethyl-carbamyl, N-piperidinylcarbonyl, N-methyl-N'-piperazinylcarbonyl, N-morpholinylcarbonyl, methylsulfonyl, ethylsulfonyl, phenylsulfonyl, methyl, ethyl, 2-piperidinylethyl, 2-morpholinylethyl, 2-(dimethylamino)ethyl, or 2-(diethylamino)ethyl, and the dotted lines represent double bonds.

5. The use according to claim 1, wherein $R_4$ is methyl, ethyl, phenyl, chloro, bromo, hydroxy, hydrogen, methoxy, cthoxy, methylthio, ethylthio, butylthio, dimethylamino, diethylamino, piperidinyl, pyrrolidinyl, imidazolyl, pyrazolyl, N-methylpiperazinyl, 2-(dimethylamino)ethylamino or morpholinyl, and the dotted lines represent double bonds.

6. The use according to claim 1, wherein $R_5$ is hydrogen, acetyl, dimethylcarbamyl, diethylcarbamyl, 2-(dimethylamino) ethylcarbamyl, N-piperidinylcarbonyl, N-methyl-N'-piperazinylcarbonyl, N-morpholinyl-carbonyl, methylsulfonyl, ethylsulfonyl, phenylsulfonyl, methyl, ethyl, 2-piperidinylethyl, 2-morpholinylethyl, 2-(dimethylamino)ethyl, or 2-(diethylamino)ethyl.

7. The use according to claim 1, wherein said compound is selected from the group consisting of:

Methyl gambogate;
9,10-Dihydrogambogyl (4-methylpiperazine);
9,10-Dihydrogambogyl (2-dimethylaminoethylamine);
Gambogyl diethylamine;
Gambogyl dimethylamine;
Gambogyl amine;
Gambogyl hydroxyamine;
Gambogyl piperidine;
6-Methoxy-gambogyl piperidine;
Gambogyl morpholine;
Gambogyl (2-dimethylaminoethylamine);
10-Morpholinyl-gambogyl morpholine;
10-Morpholinyl-gambogyl piperidine;
10-(4-methylpiperazinyl)-gambogyl piperidine;
10-(4-methylpiperazinyl)-gambogyl morpholine;
10-Piperidinyl-gambogyl piperidine;
10-(4-methylpiperazinyl)-gambogyl (4-methylpiperazine);
Gambogyl (4-methylpiperazine);
Methyl-6-Methoxy-gambogate;

Methyl-10-Morpho linyl-gambogate;
N-(2-Gambogylamido-ethyl)biotinamide;
Gambogyl (2-(4-morpholinyl)ethylamine);
Gambogyl (4-(2-pyridyl)piperazine);
10-(4-(2-Pyridyl)piperazinyl)gambogyl (4-(2-pyridyl)piperazine);
8-(Gambogylamido)octanoic acid;
6-(Gambogylamido)hexanoic acid;
12-(Gambogylamido)dodecanoic acid;
10-Methoxy-gambogyl piperidine;
Gambogyl (4-(2-pyrimidyl)piperazine);
Gambogyl (bis(2-pyridylmethyl)amine);
Gambogyl (N-(3-pyridyl)-N-(2-hydroxybenzyl)amine);
Gambogyl (4-benzylpiperazine);
Gambogyl (4-(3,4-methylenedioxybenzyl)piperazine);
Gambogyl (N-methyl-5-(methylamino)-3-oxapentylamine);
Gambogyl (N-methyl-8-(methylamino)-3,6-dioxaoctylamine);
Gambogyl (N-ethyl-2-(ethylamino)ethylamine);
Gambogyl (4-isopropylpiperazine);
Gambogyl (4-cyclopentylpiperazine);
Gambogyl (N-(2-oxo-2-ethoxyethyl)-(2-pyridyl)methylamine);
Gambogyl (2,5-dimethylpiperazine);
Gambogyl (3,5-dimethylpiperazine);
Gambogyl (4-(4-acetylphenyl)piperazine);
Gambogyl (4-ethoxycarbonylpiperazine);
Gambogyl (4-(2-oxo-2-pyrrolidylethyl)piperazine);
Gambogyl (4-(2-hydroxyethyl)piperazine);
Gambogyl (N-methyl-2-(methylamino)ethylamine);
Gambogyl (N-methyl-2-(benzylamino)ethylamine);
Gambogyl (N-methyl-(6-methyl-2-pyridyl)methylamine);
Gambogyl (N-ethyl-2-(2-pyridyl)ethylamine);
Gambogyl (N-methyl-(2-pyridyl)methylamine);
Gambogyl (N-methyl-4-(3-pyridyl)butylamine);
Gambogyl (bis(3-pyridylmethyl)amine);
Gambogyl (2,4-dimethyl-2-imidazoline);
Gambogyl (4-methyl-homopiperazine);
Gambogyl (4-(5-hydroxy-3-oxapentyl)piperazine);
Gambogyl (3-dimethylaminopyrrolidine);
Gambogyl ((2-furanyl)methylamine);
Gambogyl (2-hydroxy-1-methyl-2-phenylethylamine);
Gambogyl (3,4,5-trimethoxybenzylamine);
Gambogyl (2-(2-methoxyphenyl)ethylamine);
Gambogyl (2-methoxybenzylamine);
Gambogyl (3,4-methylenedioxybenzylamine);
Gambogyl (2-(2,5-dimethoxyphenyl)ethylamine);
Gambogyl (2-(3-methoxyphenyl)ethylamine);
Gambogyl (3-(piperidinyl)propylamine);
Gambogyl (2-(piperidinyl)ethylamine);
Gambogyl (3,4-dimethoxybenzylamine);
Gambogyl ((2-tetrahydrofuranyl)methylamine);
Gambogyl ((N-ethyl-2-pyrrolidinyl)methylamine);
Gambogyl (2-diethylaminoethylamine);
Gambogyl (2,2-dimethyl-3-dimethylaminopropylamine);
Gambogyl ((N-ethoxycarbonyl-4-piperidinyl)amine);
Gambogyl (2-carbamylpyrrolidine);
Gambogyl (3-(homopiperidinyl)propylamine);
Gambogyl ((N-benzyl-4-piperidinyl)amine);
Gambogyl (2-(4-methoxyphenyl)ethylamine);
Gambogyl (4-oxa-hex-5-enylamine);

Ganbogyl (6-hydroxyhexylamine);
Gambogyl (2-(3,5-dimethoxyphenyl)ethylamine);
Gambogyl (3,5-dimethoxybenzylamine); and
Gambogyl (2-carbamyl-2-(4-hydroxyphenyl)ethylamine).

**8.** The use according to claim 1, wherein the medicament is for treating or preventing Hodgkin's disease, non-Hodgkin's lymphomas, acute and chronic lymphocytic leukemias, multiple myeloma, neuroblastoma, breast carcinomas, ovarian carcinomas, lung carcinomas, Wilms' tumor, cervical carcinomas, testicular carcinomas, soft-tissue sarcomas, chronic lymphocytic leukemia, primary macroglobulinemia, bladder carcinomas, chronic granulocytic leukemia, primary brain carcinomas, malignant melanoma, small-cell lung carcinomas, stomach carcinomas, colon carcinomas, malignant pancreatic insulinoma, malignant carcinoid carcinomas, malignant melanomas, choriocarcinomas, mycosis fungoides, head and neck carcinomas, osteogenic sarcoma, pancreatic carcinomas, acute granulocytic leukemia, hairy cell leukemia, neuroblastoma, rhabdomyosarcoma, Kaposi's sarcoma, genitourinary carcinomas, thyroid carcinomas, esophageal carcinomas, malignant hypercalcemia, cervical hyperplasia, renal cell carcinomas, endometrial carcinomas, polycythemia vera, essential thrombocytosis, adrenal cortex carcinomas, skin cancer, or prostatic carcinomas.

**9.** A use of a compound having one of the Formulae I-III:

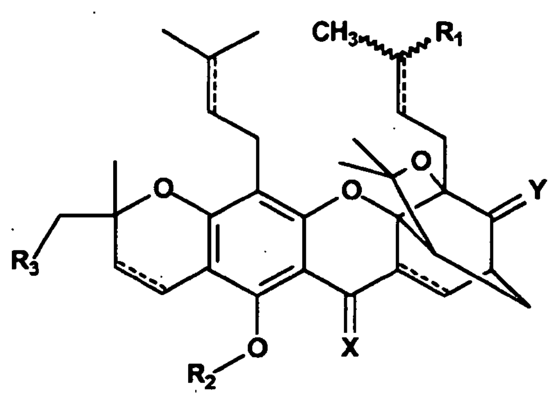

(I)

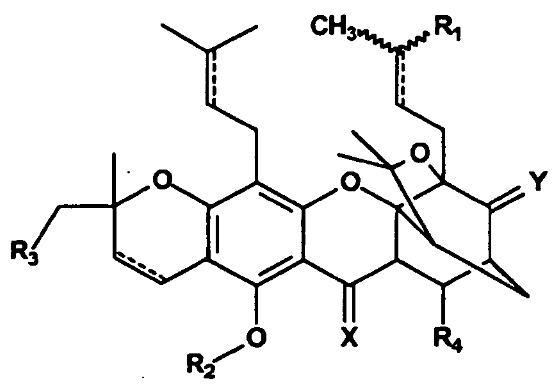

(II)

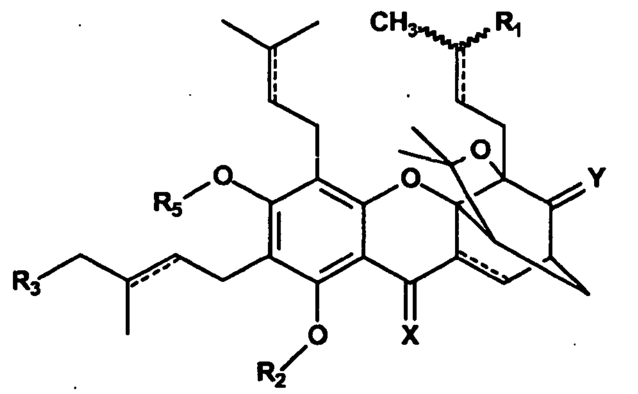

(III)

or a pharmaceutically acceptable salt or prodrug thereof, wherein:

the dotted lines are single bonds, double bonds or epoxy groups;
X together with the attached carbon is a carbonyl;
Y together with the attached carbon is a carbonyl;
$R_1$ is acyl ($R_aCO$), optionally substituted alkoxycarbonyl ($R_aOCO$), optionally substituted alkylthiocarbonyl, optionally substituted aminocarbonyl (carbamyl, $R_bR_cNCO$) or hydroxyaminocarbonyl, where $R_a$ is optionally substituted $C_{1-10}$ alkyl, optionally substituted aryl, N-succinimidyl or optionally substituted $C_{6-14}$ aryl($C_{1-10}$)alkyl group; $R_b$ and $R_c$ are independently hydrogen, optionally substituted heteroalkyl, optionally substituted $C_{1-10}$ alkyl, optionally substituted aryl, optionally substituted heteroaryl or optionally substituted $C_{6-14}$ aryl($C_{1-10}$)alkyl groups; or $R_b$ and $R_c$ may be taken together with the attached N to form an optionally substituted, saturated or partially saturated 5-7 membered heterocyclo group;
$R_2$ is hydrogen, optionally substituted alkyl, acyl ($R_aCO$), carbamyl ($R_bR_cNCO$) or sulfonyl ($R_dSO_2$), where $R_a$, $R_b$ and $R_c$ are defined above; $R_d$ is optionally substituted $C_{1-10}$ alkyl, optionally substituted aryl, or optionally substituted $C_{6-14}$ aryl($C_{1-10}$)alkyl groups;
$R_3$ is hydrogen or prenyl;
$R_4$ is hydrogen, halogen, hydroxy, optionally substituted alkyl, cycloalkyl, alkoxy, alkylthio or amino; and
$R_5$ is hydrogen, optionally substituted alkyl or acyl ($R_aCO$), carbamyl ($R_bR_cNCO$) or sulfonyl ($R_dSO_2$), where $R_a$, $R_b$, $R_c$ and $R_d$ are defined above;
for the manufacture of a medicament for treating drug resistant cancer in an animal;
wherein said prodrug is:

a) an ester of a hydroxy containing compound obtained by condensation with a $C_{1-4}$ carboxylic acid, $C_{3-6}$ dioic acid, or anhydride thereof; or
b) an imine of an amino containing compound obtained by condensation with a $C_{1-4}$ aldehyde or ketone.

**10.** The use of claim 9, wherein the dotted lines between C-9 and C-10 of a compound of Formula I or III represent a double bond, $R_4$ is not a cycloalkyl group, the other dotted lines are not epoxy groups, and $R_b$ and $R_c$ are not heteroalkyl groups.

**11.** The use according to claim 1 or 9, wherein said compound is to be administered together with at least one known cancer chemotherapeutic agent, or a pharmaceutically acceptable salt of said agent.

**12.** The use according to claim 11, wherein said known cancer chemotherapeutic agent is selected from the group consisting of busulfan, cis-platin, mitomycin C, carboplatin, colchicine, vinblastine, paclitaxel, docetaxel, camptothecin, topotecan, doxorubicin, etoposide, 5-azacytidine, 5-fluorouracil, methotrexate, 5-fluoro-2'-deoxy-uridine, ara-C, hydroxyurea, thioguanine, melphalan, chlorambucil, cyclophosamide, ifosfamide, vincristine, mitoguazone, epirubicin, aclarubicin, bleomycin, mitoxantrone, elliptinium, fludarabine, octreotide, retinoic acid, tamoxifen, Herceptin, Rituxan and alanosine.

**13.** The use according to claim 1 or 9, wherein said compound is to be administered together with radiation-therapy.

**14.** The use according to claim 1 or 9, wherein said compound is to be administered after surgical treatment for cancer.

**15.** A compound having the Formula I:

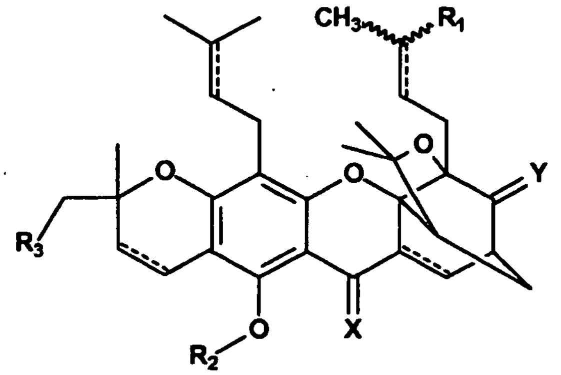

(I)

or a pharmaceutically acceptable salt or prodrug thereof, wherein:

the dotted lines are single bonds, double bonds or epoxy groups;
X together with the attached carbon is a carbonyl;
Y together with the attached carbon is a carbonyl;
$R_1$ is acyl ($R_aCO$), optionally substituted alkoxycarbonyl ($R_aOCO$), optionally substituted alkylthiocarbonyl, optionally substituted aminocarbonyl (carbamyl, $R_bR_cNCO$) or hydroxyaminocarbonyl, where $R_a$ is optionally substituted $C_{1-10}$ alkyl, optionally substituted aryl, N-succinimidyl or optionally substituted $C_{6-14}$ aryl($C_{1-10}$)alkyl group; $R_b$ and $R_c$ are independently hydrogen, optionally substituted heteroalkyl, optionally substituted $C_{1-10}$ alkyl, optionally substituted aryl, optionally substituted heteroaryl or optionally substituted $C_{6-14}$ aryl($C_{1-10}$)alkyl groups; or $R_b$ and $R_c$ may be taken together with the attached N to form an optionally substituted, saturated or partially saturated 5-7 membered heterocyclo group;
$R_2$ is hydrogen, optionally substituted alkyl, acyl ($R_aCO$), carbamyl ($R_bR_cNCO$) or sulfonyl ($R_dSO_2$), where $R_a$, $R_b$ and $R_c$ are defined above; $R_d$ is hydrogen, optionally substituted $C_{1-10}$ alkyl, optionally substituted aryl, or optionally substituted $C_{6-14}$ aryl($C_{1-10}$)alkyl groups; and $R_3$ is hydrogen or prenyl;

wherein said prodrug is:

a) an ester of a hydroxy containing compound obtained by condensation with a $C_{1-4}$ carboxylic acid, $C_{3-6}$ dioic acid, or anhydride thereof; or
b) an imine of an amino containing compound obtained by condensation with a $C_{1-4}$ aldehyde or ketone.

**16.** A compound of claim 15, wherein the dotted lines between C-9 and C-10 represent a double bond, the other dotted lines are not epoxy groups, and $R_b$ and $R_c$ are not heteroalkyl groups.

**17.** A compound according to claim 15, wherein $R_1$ is acetyl, propionyl, methoxycarbonyl, ethoxycarbonyl, methylthiocarbonyl, ethylthiocarbonyl, butylthiocarbonyl, dimethylcarbamyl, diethylcarbamyl, N-piperidinylcarbonyl, N-methyl-N'.-piperazinylcarbonyl, 2-(dimethylamino)-ethylcarbamyl or N-morpholinylcarbonyl, and the dotted lines represent double bonds.

**18.** A compound according to claim 15, wherein $R_2$ is hydrogen, formyl, acetyl, dimethylcarbamyl, diethylcarbamyl, 2-(dimethylamino)ethylcarbamyl, N-piperidinylcarbonyl, N-methyl-N'-piperazinylcarbonyl, N-morpholinylcarbonyl, methylsulfonyl, ethylsulfonyl, phenylsulfonyl, methyl, ethyl, 2-piperidinylethyl, 2-morpholinylethyl, 2-(dimethylamino) ethyl, or 2-(diethylamino)ethyl, and the dotted lines represent double bonds.

**19.** A compound having the Formula II:

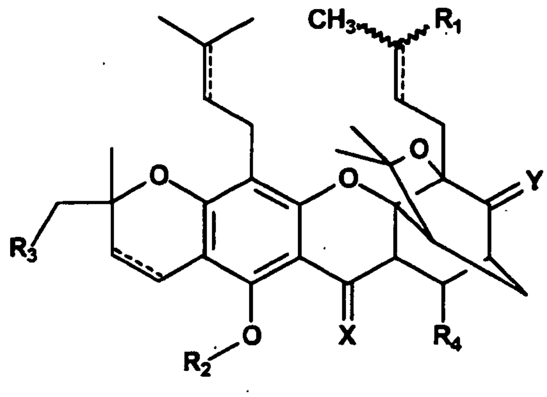

(II)

or a pharmaceutically acceptable salt or prodrug thereof, wherein:

the dotted lines are single bonds, double bonds or epoxy groups;
X together with the attached carbon is a carbonyl;
Y together with the attached carbon is a carbonyl;
$R_1$ is acyl ($R_aCO$), optionally substituted alkoxycarbonyl ($R_aOCO$), optionally substituted alkylthiocarbonyl, optionally substituted aminocarbonyl (carbamyl, $R_bR_cNCO$) or hydroxyaminocarbonyl, where $R_a$ is optionally substituted $C_{1-10}$ alkyl, optionally substituted aryl, N-succinimidyl or optionally substituted $C_{6-14}$ aryl($C_{1-10}$)alkyl group; $R_b$ and $R_c$ are independently hydrogen, optionally substituted heteroalkyl, optionally substituted $C_{1-10}$ alkyl, optionally substituted aryl, optionally substituted heteroaryl or optionally substituted $C_{6-14}$ aryl($C_{1-10}$)alkyl groups; or $R_b$ and $R_c$ may be taken together with the attached N to form an optionally substituted, saturated or partially saturated 5-7 membered heterocyclo group;
$R_2$ is hydrogen, optionally substituted alkyl, acyl ($R_aCO$), carbamyl ($R_bR_cNCO$) or sulfonyl ($R_dSO_2$), where $R_a$, $R_b$ and $R_c$ are defined above; $R_d$ is hydrogen, optionally substituted $C_{1-10}$ alkyl, optionally substituted aryl, or optionally substituted $C_{6-14}$ aryl($C_{1-10}$)alkyl groups;
$R_3$ is hydrogen or prenyl; and
$R_4$ is hydrogen, halogen, hydroxy, optionally substituted alkyl, cycloalkyl, alkoxy, alkylthio or amino;

wherein said prodrug is:

a) an ester of a hydroxy containing compound obtained by condensation with a $C_{1-4}$ carboxylic acid, $C_{3-6}$ dioic acid, or anhydride thereof; or
b) an imine of an amino containing compound obtained by condensation with a $C_{1-4}$ aldehyde or ketone.

**20.** A compound of claim 19, wherein $R_4$ is not a cycloalkyl group, the other dotted lines are not epoxy groups, and $R_b$ and $R_c$ are not heteroalkyl groups.

**21.** A compound according to claim 19, wherein $R_1$ is acetyl, propionyl, methoxycarbonyl, ethoxycarbonyl, methylthiocarbonyl, ethylthiocarbonyl, butylthiocarbonyl, dimethylcarbamyl, diethylcarbamyl, N-piperidinylcarbonyl, N-methyl-N'-piperazinylcarbonyl, 2-(dimethylamino)-ethylcarbamyl or N-morpholinylcarbonyl, and the dotted lines represent double bonds.

**22.** A compound according to claim 19, wherein $R_2$ is hydrogen, formyl, acetyl, dimethylcarbamyl, diethylcarbamyl, 2-(dimethylamino)ethylcarbamyl, N-piperidinylcarbonyl, N-methyl-N'-piperazinylcarbonyl, N-morpholinylcarbonyl, methylsulfonyl, ethylsulfonyl, phenylsulfonyl, methyl, ethyl, 2-piperidinylethyl, 2-morpholinylethyl, 2-(dimethylamino) ethyl, or 2-(diethylamino)ethyl, and the dotted lines represent double bonds.

**23.** A compound according to claim 19, wherein $R_4$ is methyl, ethyl, phenyl, chloro, bromo, hydroxy, hydrogen, methoxy, ethoxy, methylthio, ethylthio, butylthio, dimethylamino, diethylamino, piperidinyl, pyrrolidinyl, imidazolyl, pyrazolyl, N-methylpiperazinyl, 2-(dimethylamino)ethylamino or morpholinyl, and the dotted lines represent double bonds.

**24.** A compound according to claim 15, wherein said compound is selected from the group consisting of:

Gambogyl dimethylamine;
Gambogyl amine;
Gambogyl diethylamine;
Gambogyl hydroxyamine;
Gambogyl piperidine;
6-Methoxy-gambogyl piperidine;
Gambogyl morpholine;
Gambogyl (2-dimethylaminoethylamine);
Gambogyl (4-methylpiperazine);
N-(2-Gambogylamido-ethyl)biotinamide;
Gambogyl (2-(4-morpholinyl)ethylamine);
Gambogyl (4-(2-pyridyl)piperazine);
N-Hydroxysuccinimidyl gambogate;
8-(Gambogylamido)octanoic acid;
6-(Gambogylamido)hexanoic acid;
12-(Gambogylamido)dodecanoic acid;
N-Hydroxysuccinimidyl-8-(gambogylamido)octanoate;
N-Hydroxysuccinimidyl-6-(gambogylamido)hexanoate;
N-Hydroxysuccinimidyl-12-(gambogylamido)dodecanoate;
Gambogyl (4-(2-pyrimidyl)piperazine);
Gambogyl (bis(2-pyridylmethyl)amine);
Gambogyl (N-(3-pyridyl)-N-(2-hydroxybenzyl)amine);
Gambogyl (4-benzylpiperazine);
Gambogyl (4-(3,4-methylenedioxybenzyl)piperazine);
Gambogyl (N-methyl-5-(methylamino)-3-oxapentylamine);
Gambogyl (N-methyl-8-(methylamino)-3,6-dioxaoctylamine);
Gambogyl (N-ethyl-2-(ethylamino)ethylamine);
Gambogyl (4-isopropylpiperazine);
Gambogyl (4-cyclopentylpiperazine);
Gambogyl (N-(2-oxo-2-ethoxyethyl)-(2-pyridyl)methylamine);
Gambogyl (2,5-dimethylpiperazine);
Gambogyl (3,5-dimethylpiperazine);
Gambogyl (4-(4-acetylphenyl)piperazine);
Gambogyl (4-ethoxycarbonylpiperazine);
Gambogyl (4-(2-oxo-2-pyrrolidylethyl)piperazine);
Gambogyl (4-(2-hydroxyethyl)piperazine);
Gambogyl (N-methyl-2-(methylamino)ethylamine);
Gambogyl (N-methyl-2-(benzylamino)ethylamine);
Gambogyl (N-methyl-(6-methyl-2-pyridyl)methylamine);
Gambogyl (N-ethyl-2-(2-pyridyl)ethylamine);
Gambogyl (N-methyl-(2-pyridyl)methylamine);
Gambogyl (N-methyl-4-(3-pyridyl)butylamine);
Gambogyl (bis(3-pyridylmethyl)amine);
Gambogyl (2,4-dimethyl-2-imidazoline);
Gambogyl (4-methyl-homopiperazine);
Gambogyl (4-(5-hydroxy-3-oxapentyl)piperazine);
Gambogyl (3-dimethylaminopyrrolidine);
Gambogyl ((2-furanyl)methylamine);
Gambogyl (2-hydroxy-1-methyl-2-phenylethylamine);
Gambogyl (3,4,5-trimethoxybenzylamine);
Gambogyl (2-(2-methoxyphenyl)ethylamine);
Gambogyl (2-methoxybenzylamine);
Gambogyl (3,4-methylenedioxybenzylamine);
Gambogyl (2-(2,5-dimethoxyphenyl)ethylamine);
Gambogyl (2-(3-methoxyphenyl)ethylamine);
Gambogyl (3-(piperidinyl)propylamine);
Gambogyl (2-(piperidinyl)ethylamine);
Gambogyl (3,4-dimethoxybenzylamine);

Gambogyl ((2-tetrahydrofuranyl)methylamine);
Gambogyl ((N-ethyl-2-pyrrolidinyl)methylamine);
Gambogyl (2-diethylaminoethylamine);
Gambogyl (2,2-dimethyl-3-dimethylaminopropylamine);
Gambogyl ((N-ethoxycarbonyt-4-piperidinyl)amine);
Gambogyl (2-carbamylpyrrolidine);
Gambogyl (3-(homopiperidinyl)propylamine);
Gambogyl ((N-benzyl-4-piperidinyl)amine);
Gambogyl (2-(4-methoxyphenyl)ethylamine);
Gambogyl (4-oxa-hex-5-enylamine);
Ganbogyl (6-hydroxyhexylamine);
Gambogyl (2-(3,5-dimethoxyphenyl)ethylamine);
Gambogyl (3,5-dimethoxybenzylamine); and
Gambogyl (2-carbamyl-2-(4-hydroxyphenyl)ethylamine).

**25.** A compound according to claim 19, wherein said compound is selected from the group consisting of:

9,10-Dihydrogambogyl (4-methylpiperazine);
9,10-Dihydrogambogyl (dimethylamino)ethylamine;
10-(4-Methylpiperazinyl)-gambogyl piperidine;
10-(4-Methylpipecazinyl)-gambogyl morpholine;
10-Piperidinyl-gambogyl piperidine;
10-(4-Methylpiperazinyl)-gambogyl (4-methylpiperazine);
Methyl-10-Morpholinyl-gambogate;
10-Morpholinyl-gambogyl morpholine;
10-Morpholinyl-gambogyl piperidine;
10-Methoxy-gambogyl piperidine; and
10-(4-(2-Pyridyl)piperazinyl)gambogyl (4-(2-pyridyl)piperazine).

**26.** A pharmaceutical composition, comprising a compound of claim 15 or 19, and a pharmaceutically acceptable carrier.

**27.** The pharmaceutical composition of claim 26, further comprising at least one known cancer chemotherapeutic agent, or a pharmaceutically acceptable salt of said agent.

**28.** The pharmaceutical composition of claim 26, wherein said known cancer chemotherapeutic agentis selected from the group consisting of busulfan, cis-platin, mitomycin C, carboplatin, colchicine, vinblastine, paclitaxel, docetaxel, camptothecin, topotecan, doxorubicin, etoposide, 5-azacytidine, 5-fluorouracil, methotrexate, 5-fluoro-2'-deoxy-uridine, ara-C, hydroxyurea, thioguanine, melphalan, chlorambucil, cyclophosamide, ifosfamide, vincristine, mitoguazone, epirubicin, aclarubicin, bleomycin, mitoxantrone, elliptinium, fludarabine, octreotide, retinoic acid, tamoxifen, Herceptin, Rituxan and alanosine.

**29.** A compound having one of the Formulae I-III, or a pharmaceutically acceptable salt or prodrug thereof, as defined in claim 1, for use in treating cancer in an animal.

**30.** A compound having one of the Formulae I-III, or a pharmaceutically acceptable salt or prodrug thereof, as defined in claim 1, for use in treating drug resistant cancer in an animal.

**Patentansprüche**

**1.** Verwendung einer Verbindung mit einer der Formeln I bis III:

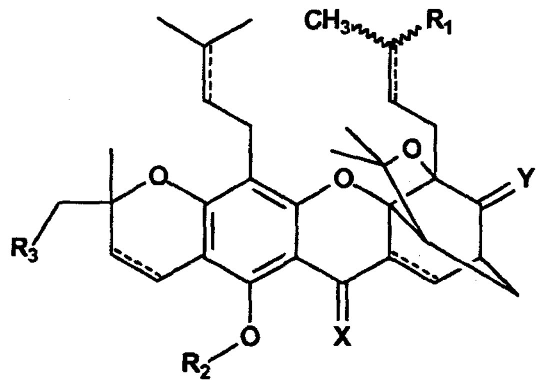

(I)

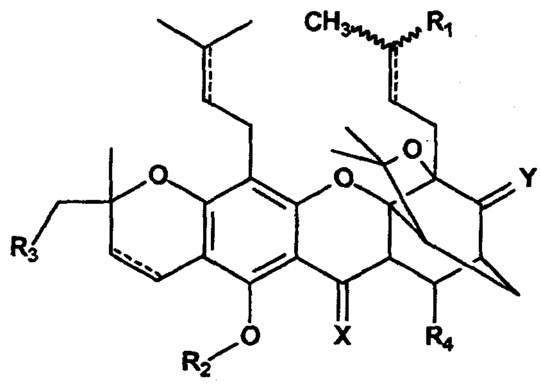

(II)

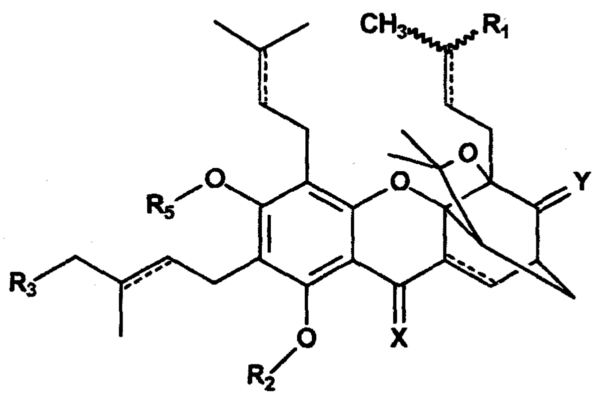

(III)

oder eines pharmazeutisch verträglichen Salzes oder Propharmakons davon, wobei:

die gestrichelten Linien Einfachbindungen, Doppelbindungen oder Epoxygruppen sind;
X zusammen mit dem gebundenen Kohlenstoff ein Carbonyl ist;
Y zusammen mit dem gebundenen Kohlenstoff ein Carbonyl ist;
$R_1$ Acyl ($R_aCO$), gegebenenfalls substituiertes Alkoxycarbonyl ($R_aOCO$), gegebenenfalls substituiertes Alkylthiocarbonyl, gegebenenfalls substituiertes Aminocarbonyl (Carbamyl, $R_bR_cNCO$) oder Hydroxyaminocarbonyl ist, wobei $R_a$ gegebenenfalls substituiertes $C_{1-10}$-Alkyl, gegebenenfalls substituiertes Aryl, N-Succinimidyl oder gegebenenfalls substituierter $C_{6-14}$-Aryl($C_{1-10}$)alkylrest ist; $R_b$ und
$R_c$ unabhängig voneinander Wasserstoff, gegebenenfalls substituiertes Heteroalkyl, gegebenenfalls substituiertes $C_{1-10}$-Alkyl, gegebenenfalls substituiertes Aryl, gegebenenfalls substituiertes Heteroaryl oder gegebenenfalls substituierte $C_{6-14}$-Aryl($C_{1-10}$)alkylreste sind; oder $R_b$ und $R_c$ mit dem gebundenen N zusammengenommen werden können, um einen gegebenenfalls substituierten, gesättigten oder teilweise gesättigten 5- bis

7-gliedrigen Heterocyclorest zu bilden;
$R_2$ Wasserstoff, gegebenenfalls substituiertes Alkyl, Acyl ($R_aCO$), Carbamyl ($R_bR_cNCO$) oder Sulfonyl ($R_dSO_2$) ist, wobei $R_a$, $R_b$ und $R_c$ wie vorstehend definiert sind; $R_d$ Wasserstoff, gegebenenfalls substituiertes $C_{1-10}$-Alkyl, gegebenenfalls substituiertes Aryl oder gegebenenfalls substituierte $C_{6-14}$-Aryl($C_{1-10}$)alkylreste ist;
$R_3$ Wasserstoff oder Prenyl ist;
$R_4$ Wasserstoff, Halogen, Hydroxy, gegebenenfalls substituiertes Alkyl, Cycloalkyl, Alkoxy, Alkylthio oder Amino ist; und
$R_5$ Wasserstoff, gegebenenfalls substituiertes Alkyl oder Acyl ($R_aCO$), Carbamyl ($R_bR_cNCO$) oder Sulfonyl ($R_dSO_2$) ist, wobei $R_a$, $R_b$, $R_c$ und $R_d$ wie vorstehend definiert sind;
zur Herstellung eines Medikaments zur Behandlung von Krebs in einem Lebewesen (animal);

wobei das Propharmakon ist:

a) ein Ester von einer hydroxyhaltigen Verbindung, welcher erhalten wird durch Kondensation mit einer $C_{1-4}$-Carbonsäure, $C_{3-6}$-Disäure oder einem Anhydrid davon; oder
b) ein Imin von einer aminohaltigen Verbindung, welches erhalten wird durch Kondensation mit einem $C_{1-4}$-Aldehyd oder -Keton.

**2.** Verwendung nach Anspruch 1, wobei die gestrichelten Linien zwischen C-9 und C-10 einer Verbindung der Formel I oder III eine Doppelbindung darstellen, $R_4$ kein Cycloalkylrest ist, die anderen gestrichelten Linien keine Epoxygruppen sind und $R_b$ und $R_c$ keine Heteroalkylreste sind.

**3.** Verwendung gemäß Anspruch 1, wobei $R_1$ Acetyl, Propionyl, Methoxycarbonyl, Ethoxycarbonyl, Methylthiocarbonyl, Ethylthiocarbonyl, Butylthiocarbonyl, Dimethylcarbamyl, Diethylcarbamyl, N-Piperidinylcarbonyl, N-Methyl-N'-piperazinylcarbonyl, 2-(Dimethylamino)ethylcarbamyl oder N-Morpholinylcarbonyl ist und die gestrichelten Linien Doppelbindungen darstellen.

**4.** Verwendung gemäß Anspruch 1, wobei $R_2$ Wasserstoff, Formyl, Acetyl, Dimethylcarbamyl, Diethylcarbamyl, 2-(Dimethylamino)ethylcarbamyl, N-Piperidinylcarbonyl, N-Methyl-N'-piperazinylcarbonyl, N-Morpholinylcarbonyl, Methylsulfonyl, Ethylsulfonyl, Phenylsulfonyl, Methyl, Ethyl, 2-Piperidinylethyl, 2-Morpholinylethyl, 2-(Dimethylamino) ethyl oder 2-(Diethylamino)ethyl ist und die gestrichelten Linien Doppelbindungen darstellen.

**5.** Verwendung gemäß Anspruch 1, wobei $R_4$ Methyl, Ethyl, Phenyl, Chlor, Brom, Hydroxy, Wasserstoff, Methoxy, Ethoxy, Methylthio, Ethylthio, Butylthio, Dimethylamino, Diethylamino, Piperidinyl, Pyrrolidinyl, Imidazolyl, Pyrazolyl, N-Methylpiperazinyl, 2-(Dimethylamino)ethylamino oder Morpholinyl ist und die gestrichelten Linien Doppelbindungen darstellen.

**6.** Verwendung gemäß Anspruch 1, wobei $R_5$ Wasserstoff, Acetyl, Dimethylcarbamyl, Diethylcarbamyl, 2-(Dimethylamino)ethylcarbamyl, N-Piperidinylcarbonyl, N-Methyl-N'-piperazinylcarbonyl, N-Morpholinylcarbonyl, Methylsulfonyl, Ethylsulfonyl, Phenylsulfonyl, Methyl, Ethyl, 2-Piperidinylethyl, 2-Morpholinylethyl, 2-(Dimethylamino)ethyl oder 2-(Diethylamino)ethyl ist.

**7.** Verwendung gemäß Anspruch 1, wobei die Verbindung aus der Gruppe ausgewählt ist, welche besteht aus:

Methylgambogat;
9,10-Dihydrogambogyl(4-methylpiperazin);
9,10-Dihydrogambogyl(2-dimethylaminoethylamin);
Gambogyldiethylamin;
Gambogyldimethylamin;
Gambogylamin;
Gambogylhydroxyamin;
Gambogylpiperidin;
6-Methoxygambogylpiperidin;
Gambogylmorpholin;
Gambogyl(2-dimethylaminoethylamin);
10-Morpholinylgambogylmorpholin;
10-Morpholinylgambogylpiperidin;
10-(4-Methylpiperazinyl)gambogylpiperidin;

10-(4-Methylpiperazinyl)gambogylmorpholin;
10-Piperidinylgambogylpiperidin;
10-(4-Methylpiperazinyl)gambogyl(4-methylpiperazin);
Gambogyl(4-methylpiperazin);
Methyl-6-methoxygambogat;
Methyl-10-morpholinylgambogat;
N-(2-Gambogylamidoethyl)biotinamid;
Gambogyl(2-(4-morpholinyl)ethylamin);
Gambogyl(4-(2-pyridyl)piperazin);
10-(4-(2-Pyridyl)piperazinyl)gambogyl(4-(2-pyridyl)piperazin);
8-(Gambogylamido)octansäure;
6-(Gambogylamido)hexansäure;
12-(Gambogylamido)dodecansäure;
10-Methoxygambogylpiperidin;
Gambogyl(4-(2-pyrimidyl)piperazin);
Gambogyl(bis(2-pyridylmethyl)amin);
Gambogyl(N-(3-pyridyl)-N-(2-hydroxybenzyl)amin);
Gambogyl(4-benzylpiperazin);
Gambogyl(4-(3,4-methylendioxybenzyl)piperazin);
Gambogyl(N-methyl-5-(methylamino)-3-oxapentylamin);
Gambogyl(N-methyl-8-(methylamino)-3,6-dioxaoctylamin);
Gambogyl(N-ethyl-2-(ethylamino)ethylamin);
Gambogyl(4-isopropylpiperazin);
Gambogyl(4-cyclopentylpiperazin);
Gambogyl(N-(2-oxo-2-ethoxyethyl)-(2-pyridyl)methylamin);
Gambogyl(2,5-dimethylpiperazin);
Gambogyl(3,5-dimethylpiperazin);
Gambogyl(4-(4-acetylphenyl)piperazin);
Gambogyl(4-ethoxycarbonylpiperazin);
Gambogyl(4-(2-oxo-2-pyrrolidylethyl)piperazin);
Gambogyl(4-(2-hydroxyethyl)piperazin);
Gambogyl(N-methyl-2-(methylamino)ethylamin);
Gambogyl(N-methyl-2-(benzylamino)ethylamin);
Gambogyl(N-methyl-(6-methyl-2-pyridyl)methylamin);
Gambogyl(N-ethyl-2-(2-pyridyl)ethylamin);
Gambogyl(N-methyl-(2-pyridyl)methylamin);
Gambogyl(N-methyl-4-(3-pyridyl)butylamin);
Gambogyl(bis(3-pyridylmethyl)amin);
Gambogyl(2,4-dimethyl-2-imidazolin);
Gambogyl(4-methylhomopiperazin);
Gambogyl(4-(5-hydroxy-3-oxapentyl)piperazin);
Gambogyl(3-dimethylaminopyrrolidin);
Gambogyl((2-furanyl)methylamin);
Gambogyl(2-hydroxy-1-methyl-2-phenylethylamin);
Gambogyl(3,4,5-trimethoxybenzylamin);
Gambogyl(2-(2-methoxyphenyl)ethylamin);
Gambogyl(2-methoxybenzylamin);
Gambogyl(3,4-methylendioxybenzylamin);
Gambogyl(2-(2,5-dimethoxyphenyl)ethylamin);
Gambogyl(2-(3-methoxyphenyl)ethylamin);
Gambogyl(3-(piperidinyl)propylamin);
Gambogyl(2-(piperidinyl)ethylamin);
Gambogyl(3,4-dimethoxybenzylamin);
Gambogyl((2-tetrahydrofuranyl)methylamin);
Gambogyl((N-ethyl-2-pyrrolidinyl)methylamin);
Gambogyl(2-diethylaminoethylamin);
Gambogyl(2,2-dimethyl-3-dimethylaminopropylamin);
Gambogyl((N-ethoxycarbonyl-4-piperidinyl)amin);

Gambogyl(2-carbamylpyrrolidin);
Gambogyl(3-(homopiperidinyl)propylamin);
Gambogyl((N-benzyl-4-piperidinyl)amin;
Gambogyl(2-(4-methoxyphenyl)ethylamin);
Gambogyl(4-oxahex-5-enylamin);
Gambogyl(6-hydroxyhexylamin);
Gambogyl(2-(3,5-dimethoxyphenyl)ethylamin);
Gambogyl(3,5-dimethoxybenzylamin), und
Gambogyl(2-carbamyl-2-(4-hydroxyphenyl)ethylamin).

**8.** Verwendung gemäß Anspruch 1, wobei das Medikament zur Behandlung oder Vorbeugung von Morbus Hodgkin, non-Hodgkin-Lymphomen, akuten und chronischen lymphatischen Leukämien, multiplem Myelom, Neuroblastom, Brustkarzinomen, Ovarialkarzinomen, Lungenkarzinomen, Wilms-Tumor, Zervixkarzinomen, Hodenkarzinomen, Weichgewebesarkomen, chronischer lymphatischer Leukämie, primärer Makroglobulinämie, Blasenkarzinomen, chronischer granulozytärer Leukämie, primären Hirnkarzinomen, malignem Melanom, kleinzelligen Lungenkarzinomen, Magenkarzinomen, Kolonkarzinomen, malignem Pankreasinsulinom, malignen karzinoiden Karzinomen, malignen Melanomen, Chorionkarzinomen, Mycosis fungoides, Kopf- und Nackenkarzinomen, Osteosarkom, Pankreaskarzinomen, akuter granulozytärer Leukämie, Haarzellenleukämie, Neuroblastom, Rhabdomyosarkom, Kaposi-Sarkom, Urogenitalkarzinomen, Schilddrüsenkarzinomen, Speiseröhrenkarzinomen, maligner Hyperkalzämie, Zervixhyperplasie, hypemephroiden Nierenkarzinomen, Endometriumkarzinomen, Polycythaemia vera, essentieller Thrombozytose, Nebennierenkarzinomen, Hautkrebs oder Prostatakarzinomen ist.

**9.** Verwendung einer Verbindung mit einer der Formeln I bis III:

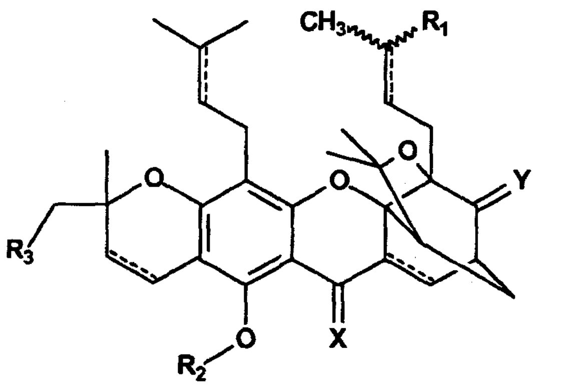

(I)

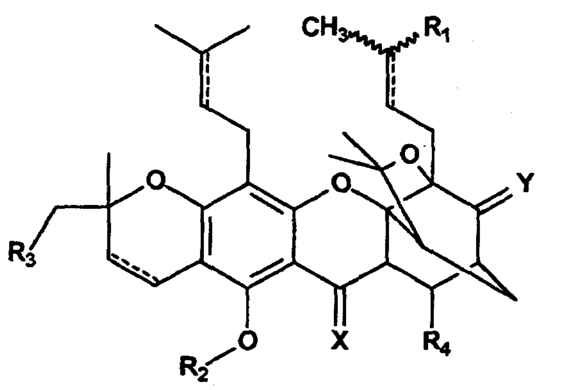

(II)

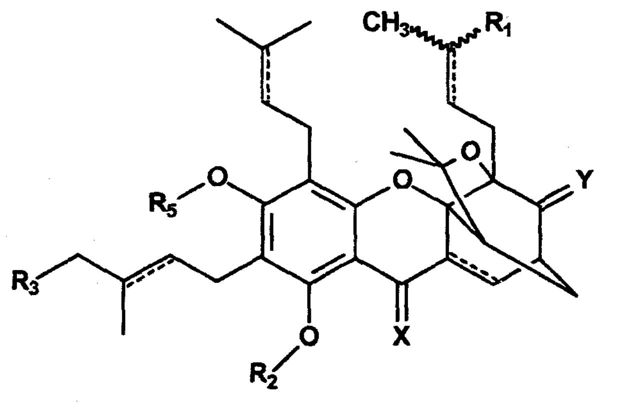

(III)

oder eines pharmazeutisch verträglichen Salzes oder Propharmakons davon, wobei:

die gestrichelten Linien Einfachbindungen, Doppelbindungen oder Epoxygruppen sind;
X zusammen mit dem gebundenen Kohlenstoff ein Carbonyl ist;
Y zusammen mit dem gebundenen Kohlenstoff ein Carbonyl ist;
$R_1$ Acyl ($R_aCO$), gegebenenfalls substituiertes Alkoxycarbonyl ($R_aOCO$), gegebenenfalls substituiertes Alkylthiocarbonyl, gegebenenfalls substituiertes Aminocarbonyl (Carbamyl, $R_bR_cNCO$) oder Hydroxyaminocarbonyl ist, wobei $R_a$ gegebenenfalls substituiertes $C_{1-10}$-Alkyl, gegebenenfalls substituiertes Aryl, N-Succinimidyl oder gegebenenfalls substituierter $C_{6-14}$-Aryl($C_{1-10}$)alkylrest ist; $R_b$ und
$R_c$ unabhängig voneinander Wasserstoff, gegebenenfalls substituiertes Heteroalkyl, gegebenenfalls substituiertes $C_{1-10}$-Alkyl, gegebenenfalls substituiertes Aryl, gegebenenfalls substituiertes Heteroaryl oder gegebenenfalls substituierte $C_{6-14}$-Aryl($C_{1-10}$)alkylreste sind; oder $R_b$ und $R_c$ mit dem gebundenen N zusammengenommen werden können, um einen gegebenenfalls substituierten, gesättigten oder teilweise gesättigten 5- bis 7-gliedrigen Heterocyclorest zu bilden;
$R_2$ Wasserstoff, gegebenenfalls substituiertes Alkyl, Acyl ($R_aCO$), Carbamyl ($R_bR_cNCO$) oder Sulfonyl ($R_dSO_2$) ist, wobei $R_a$, $R_b$ und $R_c$ wie vorstehend definiert sind; $R_d$ gegebenenfalls substituiertes $C_{1-10}$-Alkyl, gegebenenfalls substituiertes Aryl oder gegebenenfalls substituierte $C_{6-14}$-Aryl($C_{1-10}$)alkylreste ist;
$R_3$ Wasserstoff oder Prenyl ist;
$R_4$ Wasserstoff, Halogen, Hydroxy, gegebenenfalls substituiertes Alkyl, Cycloalkyl, Alkoxy, Alkylthio oder Amino ist; und
$R_5$ Wasserstoff, gegebenenfalls substituiertes Alkyl oder Acyl ($R_aCO$), Carbamyl ($R_bR_cNCO$) oder Sulfonyl ($R_dSO_2$) ist, wobei $R_a$, $R_b$, $R_c$ und $R_d$ wie vorstehend definiert sind;
zur Herstellung eines Medikaments zur Behandlung von arzneistoffresistentem Krebs in einem Lebewesen (animal);

wobei das Propharmakon ist:

a) ein Ester von einer hydroxyhaltigen Verbindung, welcher erhalten wird durch Kondensation mit einer $C_{1-4}$-Carbonsäure, $C_{3-6}$-Disäure oder einem Anhydrid davon; oder
b) ein Imin von einer aminohaltigen Verbindung, welches erhalten wird durch Kondensation mit einem $C_{1-4}$-Aldehyd oder -Keton.

**10.** Verwendung nach Anspruch 9, wobei die gestrichelten Linien zwischen C-9 und C-10 einer Verbindung der Formel I oder III eine Doppelbindung darstellen, $R_4$ kein Cycloalkylrest ist, die anderen gestrichelten Linien keine Epoxygruppen sind und $R_b$ und $R_c$ keine Heteroalkylreste sind.

**11.** Verwendung gemäß Anspruch 1 oder 9, wobei die Verbindung zusammen mit mindestens einem bekannten chemotherapeutischen Krebsmittel oder einem pharmazeutisch verträglichen Salz von dem Mittel verabreicht wird.

**12.** Verwendung gemäß Anspruch 11, wobei das bekannte chemotherapeutische Krebsmittel aus der Gruppe ausgewählt ist, welche aus Busulfan, Cisplatin, Mitomycin C, Carboplatin, Colchicin, Vinblastin, Paclitaxel, Docetaxel,

Camptothecin, Topotecan, Doxorubicin, Etoposid, 5-Azacytidin, 5-Fluoruracil, Methotrexat, 5-Fluor-2'-deoxyuridin, Ara-C, Hydroxyhamstoff, Thioguanin, Melphalan, Chlorambucil, Cyclophosamid, Ifosfamid, Vincristin, Mitoguazon, Epirubicin, Aclarubicin, Bleomycin, Mitoxantron, Elliptinium, Fludarabin, Octreotid, Retinolsäure, Tamoxifen, Herceptin, Rituxan und Alanosin besteht.

**13.** Verwendung gemäß Anspruch 1 oder 9, wobei die Verbindung zusammen mit Strahlentherapie verabreicht wird.

**14.** Verwendung gemäß Anspruch 1 oder 9, wobei die Verbindung nach einer chirurgischen Krebsbehandlung verabreicht wird.

**15.** Verbindung mit der Formel I:

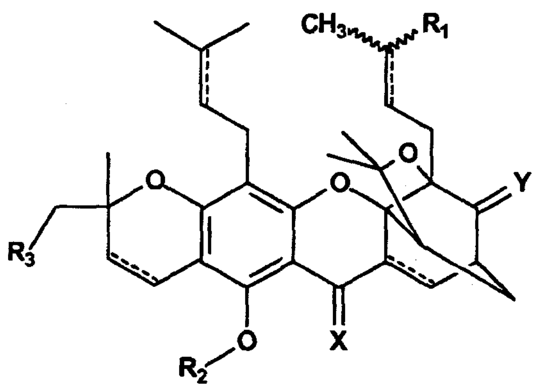

(I)

oder ein pharmazeutisch verträgliches Salz oder Propharmakon davon, wobei:

die gestrichelten Linien Einfachbindungen, Doppelbindungen oder Epoxygruppen sind;
X zusammen mit dem gebundenen Kohlenstoff ein Carbonyl ist;
Y zusammen mit dem gebundenen Kohlenstoff ein Carbonyl ist;
$R_1$ Acyl ($R_aCO$), gegebenenfalls substituiertes Alkoxycarbonyl ($R_aOCO$), gegebenenfalls substituiertes Alkylthiocarbonyl, gegebenenfalls substituiertes Aminocarbonyl (Carbamyl, $R_bR_cNCO$) oder Hydroxyaminocarbonyl ist, wobei $R_a$ gegebenenfalls substituiertes $C_{1-10}$-Alkyl, gegebenenfalls substituiertes Aryl, N-Succinimidyl oder gegebenenfalls substituierter $C_{6-14}$-Aryl($C_{1-10}$)alkylrest ist; $R_b$ und
$R_c$ unabhängig voneinander Wasserstoff, gegebenenfalls substituiertes Heteroalkyl, gegebenenfalls substituiertes $C_{1-10}$-Alkyl, gegebenenfalls substituiertes Aryl, gegebenenfalls substituiertes Heteroaryl oder gegebenenfalls substituierte $C_{6-14}$-Aryl($C_{1-10}$)alkylreste sind; oder $R_b$ und $R_c$ mit dem gebundenen N zusammengenommen werden können, um einen gegebenenfalls substituierten, gesättigten oder teilweise gesättigten 5- bis 7-gliedrigen Heterocyclorest zu bilden;
$R_2$ Wasserstoff, gegebenenfalls substituiertes Alkyl, Acyl ($R_aCO$), Carbamyl ($R_bR_cNCO$) oder Sulfonyl ($R_dSO_2$) ist, wobei $R_a$, $R_b$ und $R_c$ wie vorstehend definiert sind; $R_d$ Wasserstoff, gegebenenfalls substituiertes $C_{1-10}$-Alkyl, gegebenenfalls substituiertes Aryl oder gegebenenfalls substituierte $C_{6-14}$-Aryl($C_{1-10}$)alkylreste ist; und
$R_3$ Wasserstoff oder Prenyl ist;

wobei das Propharmakon ist:

c) ein Ester von einer hydroxyhaltigen Verbindung, welcher erhalten wird durch Kondensation mit einer $C_{1-4}$-Carbonsäure, $C_{3-6}$-Disäure oder einem Anhydrid davon; oder
d) ein Imin von einer aminohaltigen Verbindung, welches erhalten wird durch Kondensation mit einem $C_{1-4}$-Aldehyd oder -Keton.

**16.** Verbindung nach Anspruch 15, wobei die gestrichelten Linien zwischen C-9 und C-10 eine Doppelbindung darstellen, die anderen gestrichelten Linien keine Epoxygruppen sind und $R_b$ und $R_c$ keine Heteroalkylreste sind.

**17.** Verbindung gemäß Anspruch 15, wobei $R_1$ Acetyl, Propionyl, Methoxycarbonyl, Ethoxycarbonyl, Methylthiocarbonyl,

Ethylthiocarbonyl, Butylthiocarbonyl, Dimethylcarbamyl, Diethylcarbamyl, N-Piperidinylcarbonyl, N-Methyl-N'-piperazinylcarbonyl, 2-(Dimethylamino)ethylcarbamyl oder N-Morpholinylcarbonyl ist und die gestrichelten Linien Doppelbindungen darstellen.

**18.** Verbindung gemäß Anspruch 15, wobei $R_2$ Wasserstoff, Formyl, Acetyl, Dimethylcarbamyl, Diethylcarbamyl, 2-(Dimethylamino)ethylcarbamyl, N-Piperidinylcarbonyl, N-Methyl-N'-piperazinylcarbonyl, N-Morpholinylcarbonyl, Methylsulfonyl, Ethylsulfonyl, Phenylsulfonyl, Methyl, Ethyl, 2-Piperidinylethyl, 2-Morpholinylethyl, 2-(Dimethylamino) ethyl oder 2-(Diethylamino)ethyl ist und die gestrichelten Linien Doppelbindungen darstellen.

**19.** Verbindung mit der Formel II:

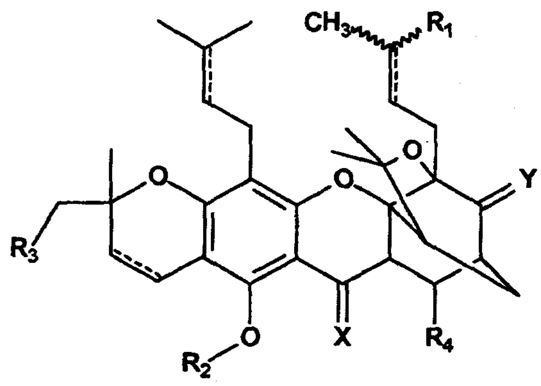

**(II)**

oder ein pharmazeutisch verträgliches Salz oder Propharmakon davon, wobei:

die gestrichelten Linien Einfachbindungen, Doppelbindungen oder Epoxygruppen sind;
X zusammen mit dem gebundenen Kohlenstoff ein Carbonyl ist;
Y zusammen mit dem gebundenen Kohlenstoff ein Carbonyl ist;
$R_1$ Acyl ($R_aCO$), gegebenenfalls substituiertes Alkoxycarbonyl ($R_aOCO$), gegebenenfalls substituiertes Alkylthiocarbonyl, gegebenenfalls substituiertes Aminocarbonyl (Carbamyl, $R_bR_cNCO$) oder Hydroxyaminocarbonyl ist, wobei $R_a$ gegebenenfalls substituiertes $C_{1-10}$-Alkyl, gegebenenfalls substituiertes Aryl, N-Succinimidyl oder gegebenenfalls substituierter $C_{6-14}$-Aryl($C_{1-10}$)alkylrest ist; $R_b$ und
$R_c$ unabhängig voneinander Wasserstoff, gegebenenfalls substituiertes Heteroalkyl, gegebenenfalls substituiertes $C_{1-10}$-Alkyl, gegebenenfalls substituiertes Aryl, gegebenenfalls substituiertes Heteroaryl oder gegebenenfalls substituierte $C_{6-14}$-Aryl($C_{1-10}$)alkylreste sind; oder $R_b$ und $R_c$ mit dem gebundenen N zusammengenommen werden können, um einen gegebenenfalls substituierten, gesättigten oder teilweise gesättigten 5- bis 7-gliedrigen Heterocyclorest zu bilden;
$R_2$ Wasserstoff, gegebenenfalls substituiertes Alkyl, Acyl ($R_aCO$), Carbamyl ($R_bR_cNCO$) oder Sulfonyl ($R_dSO_2$) ist, wobei $R_a$, $R_b$ und $R_c$ wie vorstehend definiert sind; $R_d$ Wasserstoff, gegebenenfalls substituiertes $C_{1-10}$-Alkyl, gegebenenfalls substituiertes Aryl oder gegebenenfalls substituierte $C_{6-14}$-Aryl($C_{1-10}$)alkylreste ist;
$R_3$ Wasserstoff oder Prenyl ist; und
$R_4$ Wasserstoff, Halogen, Hydroxy, gegebenenfalls substituiertes Alkyl, Cycloalkyl, Alkoxy, Alkylthio oder Amino ist;

wobei das Propharmakon ist:

a) ein Ester von einer hydroxyhaltigen Verbindung, welcher erhalten wird durch Kondensation mit einer $C_{1-4}$-Carbonsäure, $C_{3-6}$-Disäure oder einem Anhydrid davon; oder
b) ein Imin von einer aminohaltigen Verbindung, welches erhalten wird durch Kondensation mit einem $C_{1-4}$-Aldehyd oder -Keton.

**20.** Verbindung nach Anspruch 19, wobei $R_4$ kein Cycloalkylrest ist, die anderen gestrichelten Linien keine Epoxygruppen sind und $R_b$ und $R_c$ keine Heteroalkylreste sind.

**21.** Verbindung gemäß Anspruch 19, wobei $R_1$ Acetyl, Propionyl, Methoxycarbonyl, Ethoxycarbonyl, Methylthiocarbonyl, Ethylthiocarbonyl, Butylthiocarbonyl, Dimethylcarbamyl, Diethylcarbamyl, N-Piperidinylcarbonyl, N-Methyl-N'-piperazinylcarbonyl, 2-(Dimethylamino)ethylcarbamyl oder N-Morpholinylcarbonyl ist und die gestrichelten Linien Doppelbindungen darstellen.

**22.** Verbindung gemäß Anspruch 19, wobei $R_2$ Wasserstoff, Formyl, Acetyl, Dimethylcarbamyl, Diethylcarbamyl, 2-(Dimethylamino)ethylcarbamyl, N-Piperidinylcarbonyl, N-Methyl-N'-piperazinylcarbonyl, N-Morpholinylcarbonyl, Methylsulfonyl, Ethylsulfonyl, Phenylsulfonyl, Methyl, Ethyl, 2-Piperidinylethyl, 2-Morpholinylethyl, 2-(Dimethylamino)ethyl oder 2-(Diethylamino)ethyl ist und die gestrichelten Linien Doppelbindungen darstellen.

**23.** Verwendung gemäß Anspruch 19, wobei $R_4$ Methyl, Ethyl, Phenyl, Chlor, Brom, Hydroxy, Wasserstoff, Methoxy, Ethoxy, Methylthio, Ethylthio, Butylthio, Dimethylamino, Diethylamino, Piperidinyl, Pyrrolidinyl, Imidazolyl, Pyrazolyl, N-Methylpiperazinyl, 2-(Dimethylamino)ethylamino oder Morpholinyl ist und die gestrichelten Linien Doppelbindungen darstellen.

**24.** Verbindung gemäß Anspruch 15, wobei die Verbindung aus der Gruppe ausgewählt ist, welche besteht aus:

Gambogyldimethylamin;
Gambogylamin;
Gambogyldiethylamin;
Gambogylhydroxyamin;
Gambogylpiperidin;
6-Methoxygambogylpiperidin;
Gambogylmorpholin;
Gambogyl(2-dimethylaminoethylamin);
Gambogyl(4-methylpiperazin);
N-(2-Gambogylamidoethyl)biotinamid;
Gambogyl(2-(4-morpholinyl)ethylamin);
Gambogyl(4-(2-pyridyl)piperazin);
N-Hydroxysuccinimidylgambogat;
8-(Gambogylamido)octansäure;
6-(Gambogylamido)hexansäure;
12-(Gambogylamido)dodecansäure;
N-Hydroxysuccinimidyl-8-(gambogylamido)octanoat;
N-Hydroxysuccinimidyl-6-(gambogylamido)hexanoat;
N-Hydroxysuccinimidyl-12-(gambogylamido)dodecanoat;
Gambogyl(4-(2-pyrimidyl)piperazin);
Gambogyl(bis(2-pyridylmethyl)amin);
Gambogyl(N-(3-pyridyl)-N-(2-hydroxybenzyl)amin);
Gambogyl(4-benzylpiperazin);
Gambogyl(4-(3,4-methylendioxybenzyl)piperazin);
Gambogyl(N-methyl-5-(methylamino)-3-oxapentylamin);
Gambogyl(N-methyl-8-(methylamino)-3,6-dioxaoctylamin);
Gambogyl(N-ethyl-2-(ethylamino)ethylamin);
Gambogyl(4-isopropylpiperazin);
Gambogyl(4-cyclopentylpiperazin);
Gambogyl(N-(2-oxo-2-ethoxyethyl)-(2-pyridyl)methylamin);
Gambogyl(2,5-dimethylpiperazin);
Gambogyl(3,5-dimethylpiperazin);
Gambogyl(4-(4-acetylphenyl)piperazin);
Gambogyl(4-ethoxycarbonylpiperazin);
Gambogyl(4-(2-oxo-2-pyrrolidylethyl)piperazin);
Gambogyl(4-(2-hydroxyethyl)piperazin);
Gambogyl(N-methyl-2-(methylamino)ethylamin);
Gambogyl(N-methyl-2-(benzylamino)ethylamin);
Gambogyl(N-methyl-(6-methyl-2-pyridyl)methylamin);
Gambogyl(N-ethyl-2-(2-pyridyl)ethylamin);
Gambogyl(N-methyl-(2-pyridyl)methylamin);

Gambogyl(N-methyl-4-(3-pyridyl)butylamin);
Gambogyl(bis(3-pyridylmethyl)amin);
Gambogyl(2,4-dimethyl-2-imidazolin);
Gambogyl(4-methylhomopiperazin);
Gambogyl(4-(5-hydroxy-3-oxapentyl)piperazin);
Gambogyl(3-dimethylaminopyrrolidin);
Gambogyl((2-furanyl)methylamin);
Gambogyl(2-hydroxy-1-methyl-2-phenylethylamin);
Gambogyl(3,4,5-trimethoxybenzylamin);
Gambogyl(2-(2-methoxyphenyl)ethylamin);
Gambogyl(2-methoxybenzylamin);
Gambogyl(3,4-methylendioxybenzylamin);
Gambogyl(2-(2,5-dimethoxyphenyl)ethylamin);
Gambogyl(2-(3-methoxyphenyl)ethylamin);
Gambogyl(3-(piperidinyl)propylamin);
Gambogyl(2-(piperidinyl)ethylamin);
Gambogyl(3,4-dimethoxybenzylamin);
Gambogyl((2-tetrahydrofuranyl)methylamin);
Gambogyl((N-ethyl-2-pyrrolidinyl)methylamin);
Gambogyl(2-diethylaminoethylamin);
Gambogyl(2,2-dimethyl-3-dimethylaminopropylamin);
Gambogyl((N-ethoxycarbonyl-4-piperidinyl)amin);
Gambogyl(2-carbamylpyrrolidin);
Gambogyl(3-(homopiperidinyl)propylamin);
Gambogyl((N-benzyl-4-piperidinyl)amin;
Gambogyl(2-(4-methoxyphenyl)ethylamin);
Gambogyl(4-oxahex-5-enylamin);
Gambogyl(6-hydroxyhexylamin);
Gambogyl(2-(3,5-dimethoxyphenyl)ethylamin);
Gambogyl(3,5-dimethoxybenzylamin); und
Gambogyl(2-carbamyl-2-(4-hydroxyphenyl)ethylamin).

**25.** Verbindung gemäß Anspruch 19, wobei die Verbindung aus der Gruppe ausgewählt ist, welche besteht aus:

9,10-Dihydrogambogyl(4-methylpiperazin);
9,10-Dihydrogambogyl(dimethylamino)ethylamin;
10-(4-Methylpiperazinyl)gambogylpiperidin;
10-(4-Methylpiperazinyl)gambogylmorpholin;
10-Piperidinylgambogylpiperidin;
10-(4-Methylpiperazinyl)gambogyl(4-methylpiperazin);
Methyl-10-morpholinylgambogat;
10-Morpholinylgambogylmorpholin;
10-Morpholinylgambogylpiperidin;
10-Methoxygambogylpiperidin; und
10-(4-(2-Pyridyl)piperazinyl)gambogyl(4-(2-pyridyl)piperazin).

**26.** Pharmazeutische Zusammensetzung, umfassend eine Verbindung nach Anspruch 15 oder 19 und einen pharmazeutisch verträglichen Träger.

**27.** Pharmazeutische Zusammensetzung nach Anspruch 26, ferner umfassend mindestens ein bekanntes chemotherapeutisches Krebsmittel oder ein pharmazeutisch verträgliches Salz von dem Mittel.

**28.** Pharmazeutische Zusammensetzung nach Anspruch 26, wobei das bekannte chemotherapeutische Krebsmittel aus der Gruppe ausgewählt ist, welche aus Busulfan, Cisplatin, Mitomycin C, Carboplatin, Colchicin, Vinblastin, Paclitaxel, Docetaxel, Camptothecin, Topotecan, Doxorubicin, Etoposid, 5-Azacytidin, 5-Fluoruracil, Methotrexat, 5-Fluor-2'-deoxyuridin, Ara-C, Hydroxyharnstoff, Thioguanin, Melphalan, Chlorambucil, Cyclophosamid, Ifosfamid, Vincristin, Mitoguazon, Epirubicin, Aclarubicin, Bleomycin, Mitoxantron, Elliptinium, Fludarabin, Octreotid, Retinolsäure, Tamoxifen, Herceptin, Rituxan und Alanosin besteht.

**29.** Verbindung mit einer der Formeln I bis III oder ein pharmazeutisch verträgliches Salz oder Propharmakon davon, wie in Anspruch 1 definiert, zur Verwendung in der Behandlung von Krebs in einem Lebewesen (Animal).

**30.** Verbindung mit einer der Formeln I bis III oder ein pharmazeutisch verträgliches Salz oder Propharmakon davon, wie in Anspruch 1 definiert, zur Verwendung in der Behandlung von arzneistoffresistentem Krebs in einem Lebewesen (Animal).

## Revendications

**1.** Utilisation d'un composé ayant l'une des formules I à III :

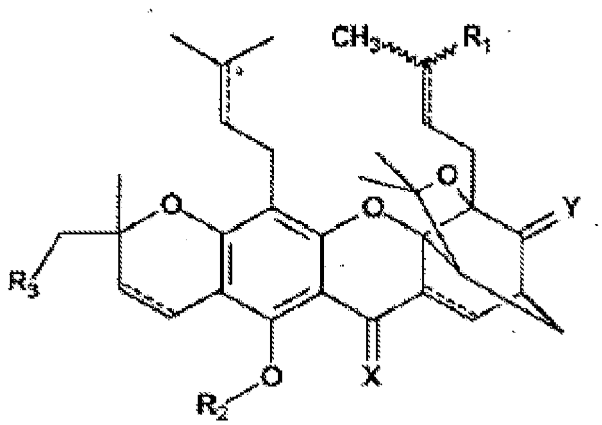

(I)

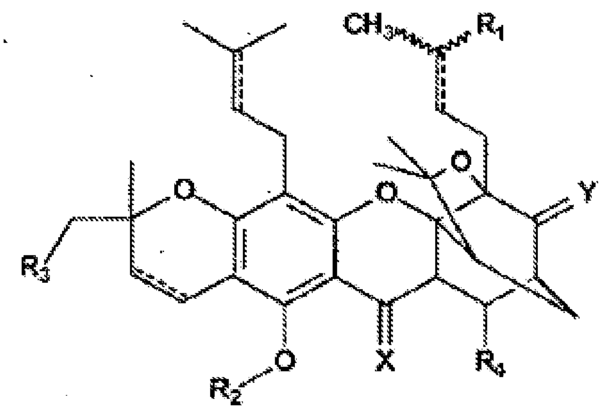

(II)

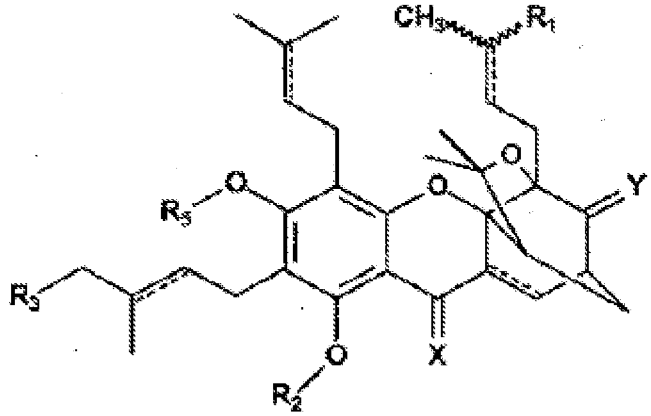

(III)

ou d'un sel pharmaceutiquement acceptable ou d'un promédicament de ceux-ci, dans lesquelles
les lignes pointillées sont des liaisons simples, des liaisons doubles ou des groupes époxy ;
X conjointement avec l'atome de carbone lié est un groupe carbonyle ;
Y conjointement avec l'atome de carbone lié est un groupe carbonyle ;
$R_1$ est un groupe acyle ($R_aCO$), alcoxycarbonyle ($R_aOCO$) éventuellement substitué, alkythiocarbonyle éventuellement substitué, aminocarbonyle éventuellement substitué (carbamyle, $R_bR_cNCO$) ou hydroxyaminocarbonyle, où $R_a$ est un groupe alkyle en $C_1$ à $C_{10}$ éventuellement substitué, aryle éventuellement substitué, un groupe N-succinimidyle ou aryle en $C_6$ à $C_{14}$-alkyle en $C_1$ à $C_{10}$ éventuellement substitué ; $R_b$ et $R_c$ sont indépendamment un atome d'hydrogène, un groupe hétéroalkyle éventuellement substitué, un groupe alkyle en $C_1$ à $C_{10}$ éventuellement substitué, aryle éventuellement substitué, hétéroaryle éventuellement substitué ou aryle en $C_6$ à $C_{14}$-alkyle en $C_1$ à $C_{10}$ éventuellement substitué ; ou $R_b$ et $R_c$ peuvent être pris conjointement avec l'atome d'azote lié pour former un groupe hétérocyclo substitué, saturé ou partiellement saturé de 5 à 7 chaînons ;
$R_2$ est un atome d'hydrogène, un groupe alkyle éventuellement substitué, acyle ($R_aCO$), carbamyle ($R_bR_cNCO$) ou sulfonyle ($R_dSO_2$), où $R_a$, $R_b$ et $R_c$ sont définis ci-dessus ; $R_d$ est un atome d'hydrogène, un groupe alkyle en $C_1$ à $C_{10}$ éventuellement substitué, aryle éventuellement substitué ou aryle en $C_6$ à $C_{14}$-alkyle en $C_1$ à $C_{10}$ éventuellement substitué ;
$R_3$ est un atome d'hydrogène ou un groupe prényle ;
$R_4$ est un atome d'hydrogène, un atome d'halogène, un groupe hydroxy, alkyle éventuellement substitué, cycloalkyle, alcoxy, alkylthio ou amino ; et
$R_5$ est un atome d'hydrogène, un groupe alkyle éventuellement substitué ou acyle ($R_aCO$), carbamyle ($R_bR_cNCO$) ou sulfonyle ($R_dSO_2$) où $R_a$, $R_b$, $R_c$ et $R_d$ sont tels que définis ci-dessus ;
pour la production d'un médicament pour traiter le cancer chez un animal ;
ledit pro-médicament étant :

a) un ester d'un composé contenant un groupe hydroxy obtenu par condensation avec un acide carboxylique en $C_1$ à $C_4$, un acide dioïque en $C_3$ à $C_6$ ou un anhydride de ceux-ci ; ou
b) une imine d'un composé contenant un groupe amino obtenue par condensation avec un aldéhyde en $C_1$ à $C_4$ ou une cétone.

**2.** Utilisation selon la revendication 1, dans laquelle les lignes pointillées entre $C_9$ à $C_{10}$ d'un composé de formule I ou III représentent une double liaison, $R_4$ n'est pas un groupe cycloalkyle, les autres lignes pointillées ne sont pas des groupes époxy et $R_b$ et $R_c$ ne sont pas des groupes hétéroalkyle.

**3.** Utilisation selon la revendication 1, dans laquelle $R_1$ est un groupe acétyle, propionyle, méthoxycarbonyle, éthoxycarbonyle, méthylthiocarbonyle, éthylthiocarbonyle, butylthiocarbonyle, diméthyl-carbamyle, diéthylcarbamyle, N-pipéridinylcarbonyle, N-méthyl-N'-pipérazinylcarbonyle, 2(diméthylamino)-éthyl-carbamyle ou N-morpholinylcarbonyle et les lignes pointillées représentent des doubles liaisons.

**4.** Utilisation selon la revendication 1, dans laquelle $R_2$ est un atome d'hydrogène, un groupe formyle, acétyle, diméthylcarbamyle, diéthylcarbamyle, 2-(diméthylamino)éthyl-carbamyle, N-pipéridinylcarbonyle, N-méthyl-N'-pipérazinylcarbonyle, N-morpholinyl-carbonyle, méthylsulfonyle, éthylsulfonyle, phénylsulfonyle, méthyle, éthyle, 2-pipéridinyléthyle, 2-morpholinyléthyle, 2-(diméthylamino)éthyle ou 2-(diéthylamino)éthyle et les lignées pointillées représentent des doubles liaisons.

**5.** Utilisation selon la revendication 1, dans laquelle $R_4$ est un groupe méthyle, éthyle, phényle, chloro, bromo, hydroxy, un atome d'hydrogène, un groupe méthoxy, éthoxy, méthylthio, éthylthio, butylthio, diméthylamino, diéthylamino, pipéridinyle, pyrrolidinyle, imidazolyle, pyrazolyle, N-méthylpipérazinyle, 2-(diméthylamino)éthylamino ou morpholinyle et les lignes pointillées représentent des doubles liaisons.

**6.** Utilisation selon la revendication 1, dans laquelle $R_5$ est un atome d'hydrogène, un groupe acétyle, diméthylcarbamyle, diéthylcarbamyle, 2-(diméthylamino)-éthylcarbamyle, N-pipéridinylcarbonyle, N-méthyl-N'-pipérazinylcarbonyle, N-morpholinyl-carbonyle, méthylsulfonyle, éthylsulfonyle, phénylsulfonyle, méthyle, éthyle, 2-pipéridinyléthyle, 2-morpholinyléthyle, 2-(diméthylamino)éthyle ou 2-(diéthylamino)éthyle.

**7.** Utilisation selon la revendication 1, dans laquelle ledit composé est choisi dans le groupe comprenant le :

méthyl-gambogate ;
9,10-dihydrogambogyl(4-méthylpipérazine) ;
9,10-dihydrogambogyl(2-diméthylaminoéthylamine) ;
gambogyl-diéthylamine ;
gambogyl-diméthylamine ;
gambogylamine ;
gambogyl-hydroxyamine ;
gambogyl-pipéridine ;
6-méthoxy-gambogyl-pipéridine ;
gambogylmorpholine ;
gambogyl(2-diméthylaminoéthylamine) ;
10-morpholinyl-gambogyl-morpholine ;
10-morpholinyl-gambogyl-pipéridine ;
10-(4-méthylpipérazinyl)-gambogyl-pipéridine ;
10-(4-méthylpiperazinyl)-gambogyl-morpholine ;
10-pipéridinyl-gambogyl-pipéridine ;
10-(4-méthylpipérazinyl)-gambogyl(4-méthylpipérazine) ;
gambogyl(4-méthylpipérazine) ;
méthyl-6-méthoxy-gambogate ;
méthyl-10-morpholinyl-gambogate ;
N-(2-gambogylamido-éthyl)biotinamide ;
gambogyl(2-(4-morpholinyl)éthylamine) ;
gambogyl(4-(2-pyridyl)pipérazine) ;
10-(4-(2-pyridyl)pipérazinyl)gambogyl(4-(2-pyridyl)pipérazine) ;
acide 8-(gambogylamido)octanoïque ;
acide 6-(gambogylamido)hexanoïque ;
acide 12-(gambogylamido)dodécanoïque ;
10-méthoxy-gambogyl-pipéridine ;
gambogyl(4-(2-pyrilidyl)pipérazine) ;
gambogyl(bis(2-pyridylméthyl)amine) ;
gambogyl(N-3-pyridyl)-N-(2-hydroxybenzyl)amine) ;
gambogyl(4-benzylpipérazine) ;
gambogyl(4-3,4-méthylènedioxybenzyl)pipérazine) ;
gambogyl(N-méthyl-5-(méthylamino)-3-oxapenthylamine) ;
gambogyl(N-méthyl-8-(méthylamino)-3,6-dioxaoctylamine) ;
gambogyl(N-éthyl-2-(éthylamino)éthylamine) ;
gambogyl(4-isopropylpipérazine) ;
gambogyl(4-cyclopentylpipérazine) ;
gambogyl(N-2-oxo-2-éthoxyéthyl)-2-pyridyl)méthylamine) ;
gambogyl(2,5-diméthylpipérazine) ;
gambogyl(3,5-diméthylpipérazine) ;
gambogyl(4-(4-acétylphényl)pipérazine) ;
gambogyl(4-éthoxycarbonylpipérazine) ;

gambogyl(4-(2-oxo-2-pyrrolidyléthyl)pipérazine) ;
gambogyl(4-(2-hydroxyéthyl)pipérazine) ;
gambogyl(N-méthyl-2-(méthylamino)éthylamine) ;
gambogyl(N-méthyl-2-(benzylamino)éthylamine) ;
gambogyl(N-méthyl-(6-méthyl-2-pyridyl)méthylamine) ;
gambogyl(N-éthyl-2-(2-pyridyl)éthylamine) ;
gambogyl(N-méthyl-(2-pyridyl)méthylamine) ;
gambogyl(N-méthyl-4-(3-pyridyl)butylamine) ;
gambogyl(bis(3-pyridylméthyl)amine ;
gambogyl(2,4-diméthyl-2-imidazoline) ;
gambogyl(4-méthyl-homopipérazine) ;
gambogyl(4-(5-hydroxy-3-oxapentyl)pipérazine) ;
gambogyl(3-diméthylaminopyrrolidine) ;
gambogyl((2-furanyl)méthylamine) ;
gambogyl(2-hydroxy-l-méthyl-2-phényléthylamine) ;
gambogyl(3,4,5-triméthoxybenzylamine) ;
gambogyl(2-(2-méthoxyphényl)éthylamine) ;
gambogyl(2-méthoxybenzylamine) ;
gambogyl(3,4-méthylènedioxybenzylamine) ;
gambogyl(2-(2,5-diméthoxyphényl)éthylamine) ;
gambogyl(2-(3-méthoxyphényl)éthylamine ;
gambogyl(3-pipéridinyl)propylamine) ;
gambogyl(2-pipéridinyl)éthylamine) ;
gambogyl(3,4-diméthoxybenzylamine) ;
gambogyl((2-tétrahydrofuranyl)méthylamine) ;
gambogyl((N-éthyl-2-pyrrolidinyl)méthylamine) ;
gambogyl(2-diéthylaminoéthylamine) ;
gambogyl(2,2-diméthyl-3-diméthylaminopropylamine) ;
gambogyl((N-éthoxycarbonyl-4-pipéridinyl)amine) ;
gambogyl(2-carbamylpyrrolidine) ;
gambogyl(3-(homopipéridinyl)propylamine) ;
gambogyl((N-benzyl-4-pipéridinyl)amine) ;
gambogyl(2-(4-méthoxyphényl)éthylamine) ;
gambogyl(4-oxa-hex-5-énylamine) ;
gambogyl(6-hydroxyhexylamine) ;
gambogyl(2-(3,5-diméthoxyphényl)éthylamine) ;
gambogyl(3,5-diméthoxybenzylamine) ; et
gambogyl(2-carbamyl-2-(4-hydroxyphényl)éthylamine).

**8.** Utilisation selon la revendication 1, dans lequel le médicament est destiné à traiter ou à prévenir la maladie de Hodgkin, les lymphomes non hodgkiniens, les leucémies lymphocytaires aiguës et chroniques, le myélome multiple, les neuroblastomes, les cancers du sein, les cancers ovariens, les cancers du poumon, les tumeurs de Wilms, les cancers du col de l'utérus, les cancers des testicules, les sarcomes des tissus mous, la leucémie lymphocytaire chronique, la macroglobulinémie primaire, les cancers de la vessie, la leucémie granulocytaire chronique, les cancers cérébraux primaires, les mélanomes malins, les carcinomes pulmonaires à petites cellules, les cancers gastriques, les cancers du côlon, l'insulinome pancréatique malin, les carcinomes carcinoïdes malins, les mélanomes malins, les choriocarcinomes, les mycoses fongoïdes, les cancers de la tête et du cou, les sarcomes ostéogéniques, les cancers du pancréas, la leucémie granulocytaire aiguë, la leucémie à tricholymphocytes, les neuroblastomes, le rhabdomyosarcome, le sarcome de Kaposi, les cancers génito-urinaires, les cancers de la thyroïde, les cancers de l'oesophage, l'hypercalcémie maligne, l'hyperplasie cervicale, les cancers des cellules rénales, les cancers de l'endomètre, l'érythrémie, la thrombocytose essentielle, les cancers des corticosurrénales, le cancer de la peau ou les cancers de la prostate.

**9.** Utilisation d'un composé ayant l'une des formules I à III :

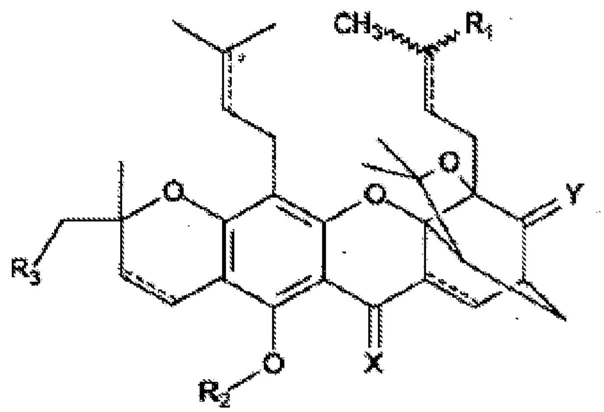

(I)

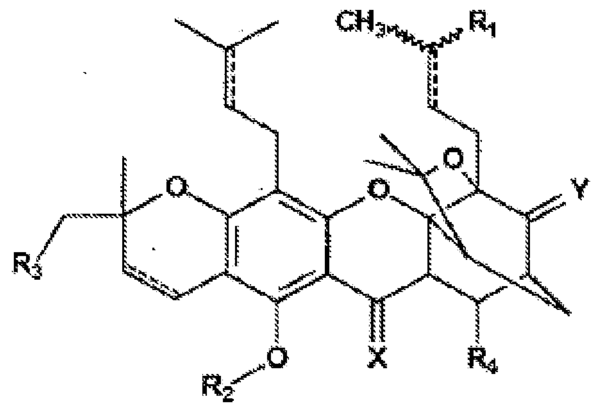

(II)

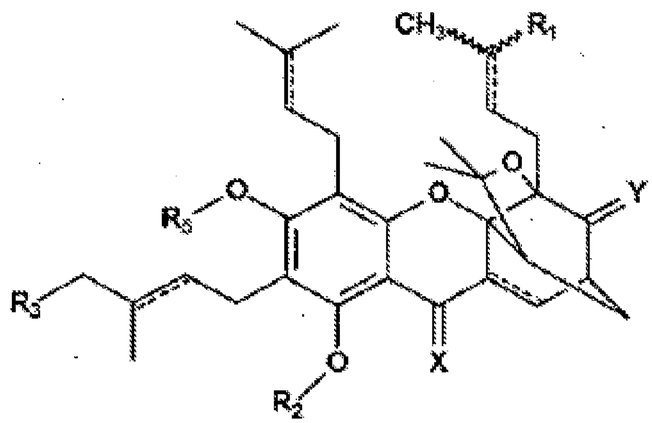

(III)

ou d'un sel pharmaceutiquement acceptable ou d'un promédicament de ceux-ci, dans lesquelles
les lignes pointillées sont des liaisons simples, des liaisons doubles ou des groupes époxy ;
X conjointement avec l'atome de carbone lié est un groupe carbonyle ;
Y conjointement avec l'atome de carbone lié est un groupe carbonyle ;
$R_1$ est un groupe acyle ($R_aCO$), alcoxycarbonyle ($R_aOCO$) éventuellement substitué, alkythiocarbonyle éventuellement substitué, aminocarbonyle éventuellement substitué (carbamyle, $R_bR_cNCO$) ou hydroxyaminocarbonyle, où $R_a$ est un groupe alkyle en $C_1$ à $C_{10}$ éventuellement substitué, aryle éventuellement substitué, un groupe N-succinimidyle ou aryle en $C_6$ à $C_{14}$-alkyle en $C_1$ à $C_{10}$ éventuellement substitué ; $R_b$ et $R_c$ sont indépendamment un atome d'hydrogène, un groupe hétéroalkyle éventuellement substitué, un groupe alkyle en $C_1$ à $C_{10}$ éventuellement substitué, aryle éventuellement substitué, hétéroaryle éventuellement substitué ou aryle en $C_6$ à $C_{14}$-alkyle

en $C_1$ à $C_{10}$ éventuellement substitué ; ou $R_b$ et $R_c$ peuvent être pris conjointement avec l'atome d'azote lié pour former un groupe hétérocyclo substitué, saturé ou partiellement saturé de 5 à 7 chaînons ;
$R_2$ est un atome d'hydrogène, un groupe alkyle éventuellement substitué, acyle ($R_aCO$), carbamyle ($R_bR_cNCO$) ou sulfonyle ($R_dSO_2$), où $R_a$, $R_b$ et $R_c$ sont définis ci-dessus ; $R_d$ est un groupe alkyle en $C_1$ à $C_{10}$ éventuellement substitué, aryle éventuellement substitué ou aryle en $C_6$ à $C_{14}$-alkyle en $C_1$ à $C_{10}$ éventuellement substitué ;
$R_3$ est un atome d'hydrogène ou un groupe prényle ;
$R_4$ est un atome d'hydrogène, un atome d'halogène, un groupe hydroxy, alkyle éventuellement substitué, cycloalkyle, alcoxy, alkylthio ou amino ; et
$R_5$ est un atome d'hydrogène, un groupe alkyle éventuellement substitué ou acyle ($R_aCO$), carbamyle ($R_bR_cNCO$) ou sulfonyle ($R_dSO_2$) où $R_a$, $R_b$, $R_c$ et $R_d$ sont tels que définis ci-dessus ;
pour la production d'un médicament pour traiter un cancer résistant aux médicaments chez un animal ;
ledit pro-médicament étant :

a) un ester d'un composé contenant un groupe hydroxy obtenu par condensation avec un acide carboxylique en $C_1$ à $C_4$, un acide dioïque en $C_3$ à $C_6$ ou un anhydride de ceux-ci ; ou
b) une imine d'un composé contenant un groupe amino obtenue par condensation avec un aldéhyde en $C_1$ à $C_4$ ou une cétone.

**10.** Utilisation selon la revendication 9, dans laquelle les lignes pointillées entre $C_9$ à $C_{10}$ d'un composé de formule I ou III représentent une double liaison, $R_4$ n'est pas un groupe cycloalkyle, les autres lignes pointillées ne sont pas des groupes époxy et $R_b$ et $R_c$ ne sont pas des groupes hétéroalkyle.

**11.** Utilisation selon la revendication 1 ou 9,
dans laquelle ledit composé doit être administré conjointement avec au moins un agent chimiothérapeutique connu ou un sel pharmaceutiquement acceptable dudit agent.

**12.** Utilisation selon la revendication 11, dans laquelle ledit agent chimiothérapeutique connu est choisi dans le groupe comprenant le busulfan, le cis-platine, la mitomycine C, le carboplatine, la colchicine, la vinblastine, le paclitaxel, le dodétaxel, la camptothécine, le topotécan, la doxorubicine, l'étoposide, la 5-azacytidine, le 5-fluoro-uracile, le méthotrexate, la 5-fluoro-2'-désoxy-uridine, l'ara-C-hydroxyurée, la thioguanine, le melphalan, le chlorambucil, le cyclophosamide, l'ifosfamide, la vincristine, la mitoguazone, l'épirubicine, l'aclarubicine, la bléomycine, la mitoxantrone, l'elliptinium, la fludarabine, l'octréotide, l'acide rétinoïque, le tamoxifène, l'herceptine, le rituxane et l'alanosine.

**13.** Utilisation selon la revendication 1 ou 9, dans laquelle ledit composé doit être administré conjointement avec une radiothérapie.

**14.** Utilisation selon la revendication 1 ou 9, dans laquelle ledit composé doit être administré après un traitement chirurgical du cancer.

**15.** Composé ayant la formule I :

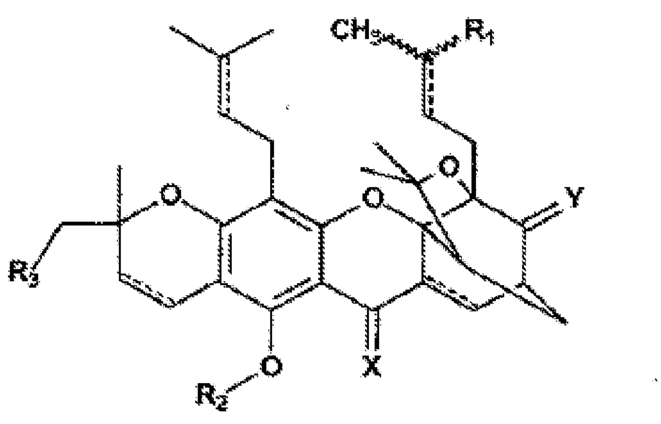

(I)

ou sel pharmaceutiquement acceptable ou promédicament de ceux-ci, dans lesquelles

les lignes pointillées sont des liaisons simples, des liaisons doubles ou des groupes époxy ;
X conjointement avec l'atome de carbone lié est un groupe carbonyle ;
Y conjointement avec l'atome de carbone lié est un groupe carbonyle ;
$R_1$ est un groupe acyle ($R_aCO$), alcoxycarbonyle ($R_aOCO$) éventuellement substitué, alkythiocarbonyle éventuellement substitué, aminocarbonyle éventuellement substitué (carbamyle, $R_bR_cNCO$) ou hydroxyaminocarbonyle, où $R_a$ est un groupe alkyle en $C_1$ à $C_{10}$ éventuellement substitué, aryle éventuellement substitué, un groupe N-succinimidyle ou aryle en $C_6$ à $C_{14}$-alkyle en $C_1$ à $C_{10}$ éventuellement substitué ; $R_b$ et $R_c$ sont indépendamment un atome d'hydrogène, un groupe hétéroalkyle éventuellement substitué, un groupe alkyle en $C_1$ à $C_{10}$ éventuellement substitué, aryle éventuellement substitué, hétéroaryle éventuellement substitué ou aryle en $C_6$ à $C_{14}$-alkyle en $C_1$ à $C_{10}$ éventuellement substitué ; ou $R_b$ et $R_c$ peuvent être pris conjointement avec l'atome d'azote lié pour former un groupe hétérocyclo substitué, saturé ou partiellement saturé de 5 à 7 chaînons ;
$R_2$ est un atome d'hydrogène, un groupe alkyle éventuellement substitué, acyle ($R_aCO$), carbamyle ($R_bR_cNCO$) ou sulfonyle ($R_dSO_2$), où $R_a$, $R_b$ et $R_c$ sont définis ci-dessus ; $R_d$ est un atome d'hydrogène, un groupe alkyle en $C_1$ à $C_{10}$ éventuellement substitué, aryle éventuellement substitué ou aryle en $C_6$ à $C_{14}$-alkyle en $C_1$ à $C_{10}$ éventuellement substitué ;
$R_3$ est un atome d'hydrogène ou un groupe prényle ;
ledit promédicament étant :

a) un ester d'un composé contenant un groupe hydroxy obtenu par condensation avec un acide carboxylique en $C_1$ à $C_4$, un acide dioïque en $C_3$ à $C_6$ ou un anhydride de ceux-ci ; ou
b) une imine d'un composé contenant un groupe amino obtenue par condensation avec un aldéhyde en $C_1$ à $C_4$ ou une cétone.

**16.** Utilisation selon la revendication 15, dans laquelle les lignes pointillées entre $C_9$ à $C_{10}$ représentent une double liaison, les autres lignes pointillées ne sont pas des groupes époxy et $R_b$ et $R_c$ ne sont pas des groupes hétéroalkyle.

**17.** Composé selon la revendication 15, dans lequel $R_1$ est un groupe acétyle, propionyle, méthoxycarbonyle, éthoxycarbonyle, méthylthiocarbonyle, éthylthiocarbonyle, butylthiocarbonyle, diméthylcarbamyle, diéthylcarbamyle, N-pipéridinylcarbonyle, N-méthyl-N'-pipérazinylcarbonyle, 2-(diméthylamino)-éthylcarbamyle ou N-morpholinylcarbonyle et les lignes pointillées représentent des doubles liaisons.

**18.** Composé selon la revendication 15, dans lequel $R_2$ est un atome d'hydrogène, un groupe formyle, acétyle, diméthylcarbamyle, diéthylcarbamyle, 2-(diméthylamino)éthylcarbamyle, N-pipéridinylcarbonyle, N-méthyl-N'-pipérazinylcarbonyle, N-morpholinyl-carbonyle, méthylsulfonyle, éthymsulfonyle, phénylsulfonyle, méthyle, éthyle, 2-pipéridinyléthyle, 2-morpholinyléthyle, 2-(diméthylamino)éthyle ou 2-(diéthylamino)éthyle et les lignes pointillées représentent des doubles liaisons.

**19.** Composé ayant la formule II :

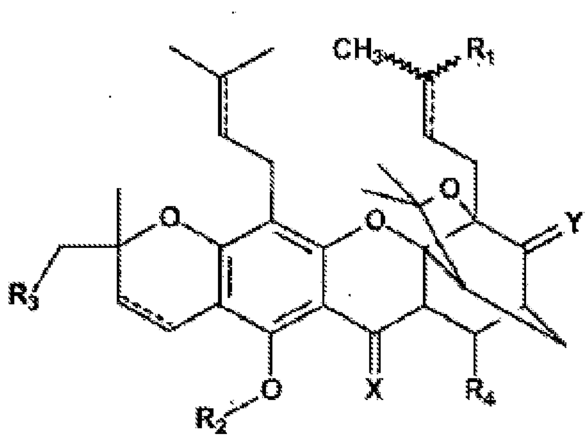

(II)

ou sel pharmaceutiquement acceptable ou promédicament de ceux-ci, dans laquelle
les lignes pointillées sont des liaisons simples, des liaisons doubles ou des groupes époxy ;

X conjointement avec l'atome de carbone lié est un groupe carbonyle ;
Y conjointement avec l'atome de carbone lié est un groupe carbonyle ;
$R_1$ est un groupe acyle ($R_aCO$), alcoxycarbonyle ($R_aOCO$) éventuellement substitué, alkythiocarbonyle éventuellement substitué, aminocarbonyle éventuellement substitué (carbamyle, $R_bR_cNCO$) ou hydroxyaminocarbonyle, où $R_a$ est un groupe alkyle en $C_1$ à $C_{10}$ éventuellement substitué, aryle éventuellement substitué, un groupe N-succinimidyle ou aryle en $C_6$ à $C_{14}$-alkyle en $C_1$ à $C_{10}$ éventuellement substitué ; $R_b$ et $R_c$ sont indépendamment un atome d'hydrogène, un groupe hétéroalkyle éventuellement substitué, un groupe alkyle en $C_1$ à $C_{10}$ éventuellement substitué, aryle éventuellement substitué, hétéroaryle éventuellement substitué ou aryle en $C_6$ à $C_{14}$-alkyle en $C_1$ à $C_{10}$ éventuellement substitué ; ou $R_b$ et $R_c$ peuvent être pris conjointement avec l'atome d'azote lié pour former un groupe hétérocyclo substitué, saturé ou partiellement saturé de 5 à 7 chaînons ;
$R_2$ est un atome d'hydrogène, un groupe alkyle éventuellement substitué, acyle ($R_aCO$), carbamyle ($R_bR_cNCO$) ou sulfonyle ($R_dSO_2$), où $R_a$, $R_b$ et $R_c$ sont définis ci-dessus ; $R_d$ est un atome d'hydrogène, un groupe alkyle en $C_1$ à $C_{10}$ éventuellement substitué, aryle éventuellement substitué ou aryle en $C_6$ à $C_{14}$-alkyle en $C_1$ à $C_{10}$ éventuellement substitué ;
$R_3$ est un atome d'hydrogène ou un groupe prényle ;
$R_4$ est un atome d'hydrogène, un atome d'halogène, un groupe hydroxy, alkyle éventuellement substitué, cycloalkyle, alcoxy, alkylthio ou amino ;
où ledit promédicament est :

a) un ester d'un composé contenant un groupe hydroxy obtenu par condensation avec un acide carboxylique en $C_1$ à $C_4$, un acide dioïque en $C_3$ à $C_6$ ou un anhydride de ceux-ci ; ou
b) une imine d'un composé contenant un groupe amino obtenue par condensation avec un aldéhyde en $C_1$ à $C_4$ ou une cétone.

**20.** Composé selon la revendication 19, dans lequel $R_4$ n'est pas un groupe cycloalkyle, les autres lignes pointillées ne sont pas des groupes époxy et $R_b$ et $R_c$ ne sont pas des groupes hétéroalkyle.

**21.** Composé selon la revendication 19, dans lequel $R_1$ est un groupe acétyle, propionyle, méthoxycarbonyle, éthoxycarbonyle, méthylthiocarbonyle, éthylthiocarbonyle, butylthiocarbonyle, diméthyl-carbamyle, diéthylcarbamyle, N-pipéridinylcarbonyle, N-méthyl-N'-pipérazinylcarbonyle, 2(diméthylamino)-éthyl-carbamyle ou N-morpholinylcarbonyle et les lignes pointillées représentent des doubles liaisons.

**22.** Composé selon la revendication 19, dans lequel $R_2$ est un atome d'hydrogène, un groupe formyle, acétyle, diméthylcarbamyle, diéthylcarbamyle, 2-(diméthylamino)éthylcarbamyle, N-pipéridinylcarbonyle, N-méthyl-N'-pipérazinylcarbonyle, N-morpholinyl-carbonyle, méthylsulfonyle, éthylsulfonyle, phénylsulfonyle, méthyle, éthyle, 2-pipéridinyléthyle, 2-morpholinyléthyle, 2-(diméthylamino)éthyle ou 2-(diéthylamino)éthyle et les lignes pointillées représentent des doubles liaisons.

**23.** Composé selon la revendication 19, dans lequel $R_4$ est un groupe méthyle, éthyle, phényle, chloro, bromo, hydroxy, un atome d'hydrogène, un groupe méthoxy, éthoxy, méthylthio, éthylthio, butylthio, diméthylamino, diéthylamino, pipéridinyle, pyrrolidinyle, imidazolyle, pyrazolyle, N-méthylpipérazinyle, 2-(diméthylamino) éthylamino ou morpholinyle et les lignes pointillées représentent des doubles liaisons.

**24.** Composé selon la revendication 15, ledit composé étant choisi dans le groupe comprenant les :

gambogyl-diméthylamine ;
gambogylamine ;
gambogyl-diéthylamine ;
gambogyl-hydroxyamine ;
gambogyl-pipéridine ;
6-méthoxy-gambogyl-pipéridine ;
gambogylmorpholine ;
gambogyl(2-diméthylaminoéthylamine) ;
gambogyl(4-méthylpipérazine) ;
N-(2-gambogylamido-éthyl)biotinamide ;
gambogyl(2-(4-morpholinyl)éthylamine) ;
gambogyl(4-(2-pyridyl)pipérazine) ;
N-hydroxysuccinimidyl-gambogate ;

acide 8-(gambogylamido)octanoïque ;
acide 6-(gambogylamido)hexanoïque ;
acide 12-(gambogylamido)dodécanoïque ;
N-hydroxysuccinimidyl-8-(gambogylamido)octanoate ;
N-hydroxysuccinimidyl-6-(gambogylamido)hexanoate ;
N-hydroxysuccinimidyl-12-(gambogylamido)dodécanoate ;
gambogyl(4-(2-pyrilidyl)pipérazine) ;
gambogyl(bis(2-pyridylméthyl)amine) ;
gambogyl(N-3-pyridyl)-N-(2-hydroxybenzyl)amine) ;
gambogyl(4-benzylpipérazine) ;
gambogyl(4-3,4-méthylènedioxybenzyl)pipérazine) ;
gambogyl(N-méthyl-5-(méthylamino)-3-oxapenthylamine) ;
gambogyl(N-méthyl-8-(méthylamino)-3,6-dioxaoctylamine) ;
gambogyl(N-éthyl-2-(éthylamino)éthylamine) ;
gambogyl(4-isopropylpipérazine) ;
gambogyl(4-cyclopentylpipérazine) ;
gambogyl(N-2-oxo-2-éthoxyéthyl)-2-pyridyl)méthylamine) ;
gambogyl(2,5-diméthylpipérazine) ;
gambogyl(3,5-diméthylpipérazine) ;
gambogyl(4-(4-acétylphényl)pipérazine) ;
gambogyl(4-éthoxycarbonylpipérazine) ;
gambogyl(4-(2-oxo-2-pyrrolidyléthyl)pipérazine) ;
gambogyl(4-(2-hydroxyéthyl)pipérazine) ;
gambogyl(N-méthyl-2-(méthylamino)éthylamine) ;
gambogyl(N-méthyl-2-(benzylamino)éthylamine) ;
gambogyl(N-méthyl-(6-méthyl-2-pyridyl)méthylamine) ;
gambogyl(N-éthyl-2-(2-pyridyl)éthylamine) ;
gambogyl(N-méthyl-(2-pyridyl)méthylamine) ;
gambogyl(N-méthyl-4-(3-pyridyl)butylamine) ;
gambogyl(bis(3-pyridylméthyl)amine ;
gambogyl(2,4-diméthyl-2-imidazoline) ;
gambogyl(4-méthyl-homopipérazine) ;
gambogyl(4-(5-hydroxy-3-oxapentyl)pipérazine) ;
gambogyl(3-diméthylaminopyrrolidine) ;
gambogyl((2-furanyl)méthylamine) ;
gambogyl(2-hydroxy-1-méthyl-2-phényléthylamine) ;
gambogyl(3,4,5-triméthoxybenzylamine) ;
gambogyl(2-(2-méthoxyphényl)éthylamine) ;
gambogyl(2-méthoxybenzylamine) ;
gambogyl(3,4-méthylènedioxybenzylamine) ;
gambogyl(2-(2,5-diméthoxyphényl)éthylamine) ;
gambogyl(2-(3-méthoxyphényl)éthylamine ;
gambogyl(3-pipéridinyl)propylamine) ;
gambogyl(2-pipéridinyl)éthylamine) ;
gambogyl(3,4-diméthoxybenzylamine) ;
gambogyl((2-tétrahydrofuranyl)méthylamine) ;
gambogyl((N-éthyl-2-pyrrolidinyl)méthylamine) ;
gambogyl(2-diéthylaminoéthylamine) ;
gambogyl(2,2-diméthyl-3-diméthylaminopropylamine) ;
gambogyl((N-éthoxycarbonyl-4-pipéridinyl)amine) ;
gambogyl(2-carbamylpyrrolidine) ;
gambogyl(3-(homopipéridinyl)propylamine) ;
gambogyl((N-benzyl-4-pipéridinyl)amine) ;
gambogyl(2-(4-méthoxyphényl)éthylamine) ;
gambogyl(4-oxa-hex-5-énylamine) ;
gambogyl(6-hydroxyhexylamine) ;
gambogyl(2-(3,5-diméthoxyphényl)éthylamine) ;
gambogyl(3,5-diméthoxybenzylamine) ; et

gambogyl(2-carbamyl-2-(4-hydroxyphényl)éthylamine).

**25.** Composé selon la revendication 19, ledit composé étant choisi dans le groupe comprenant les :

9,10-dihydrogambogyl(4-méthylpipérazine) ;
9,10-dihydrogambogyl(diméthylamino)éthylamine ;
10-(4-méthylpipérazinyl)-gambogyl-pipéridine ;
10-(4-méthylpipérazinyl)-gambogyl-morpholine ;
10-pipéridinyl-gambogyl-pipéridine ;
10-(4-méthylpipérazinyl)-gambogyl-(4-méthylpipérazine) ;
méthyl-10-morpholinyl-gamboate ;
10-morpholinyl-gambogyl-morpholine ;
10-morpholinyl-gambogyl-pipéridine ;
10-méthoxy-gambogyl-pipéridine ; et
10-(4-(2-pyridyl)pipérazinyl)gambogyl-(4-(2-pyridyl)pipérazine).

**26.** Composition pharmaceutique, comprenant un composé selon la revendication 15 ou 19, et un véhicule pharmaceutiquement acceptable.

**27.** Composition pharmaceutique selon la revendication 26, comprenant en outre au moins un agent chimiothérapeutique connu ou un sel pharmaceutiquement acceptable dudit agent.

**28.** Composition pharmaceutique selon la revendication 26, dans laquelle ledit agent chimiothérapeutique connu est choisi dans le groupe comprenant le busulfan, le cis-platine, la mitomycine C, le carboplatine, la colchicine, la vinblastine, le paclitaxel, le dodétaxel, la camptothécine, le topotécan, la doxorubicine, l'étoposide, la 5-azacytidine, le 5-fluoro-uracile, le méthotrexate, la 5-fluoro-2'-désoxy-uridine, l'ara-C-hydroxyurée, la thioguanine, le melphalan, le chlorambucil, le cyclophosamide, l'ifosfamide, la vincristine, la mitoguazone, l'épirubicine, l'aclarubicine, la bléomycine, la mitoxantrone, l'elliptinium, la fludarabine, l'octréotide, l'acide rétinoïque, le tamoxifène, l'herceptine, le rituxane et l'alanosine.

**29.** Composé ayant l'une des formules I à III ou sel ou promédicament pharmaceutiquement acceptable de ceux-ci défini à la revendication 1, à utiliser pour traiter le cancer chez un animal.

**30.** Composé ayant l'une des formules I à III ou sel ou promédicament pharmaceutiquement acceptable de ceux-ci défini à la revendication 1, à utiliser pour traiter un cancer résistant aux médicaments chez un animal.

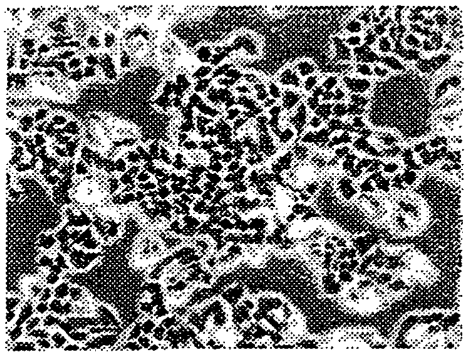

FIG.1A

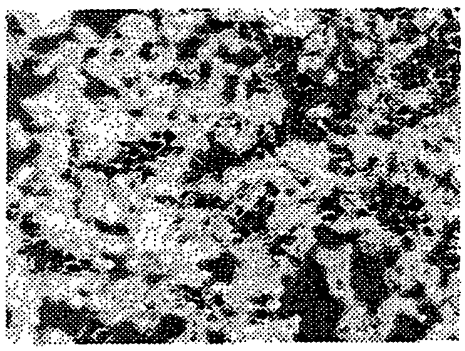

FIG.1B

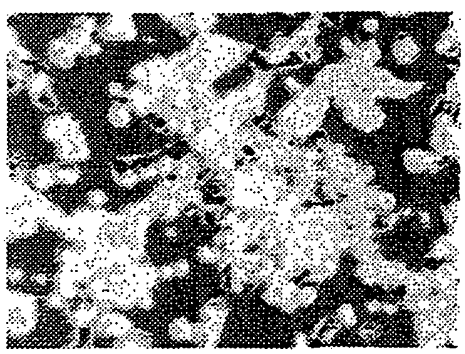

FIG.1C

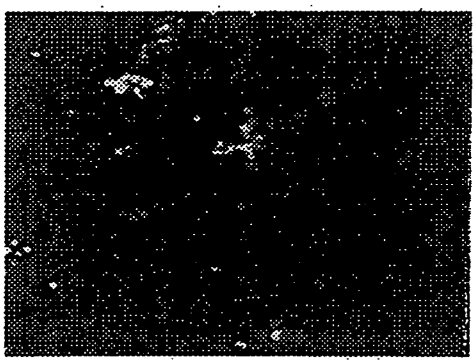

FIG.2A

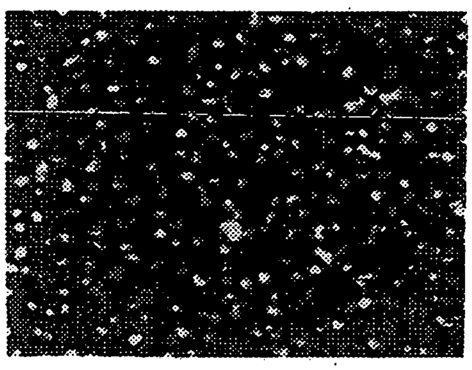

FIG.2B

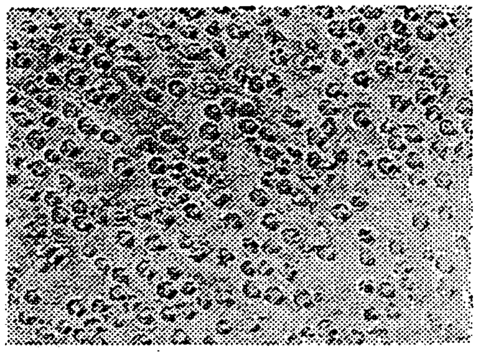

FIG.3A

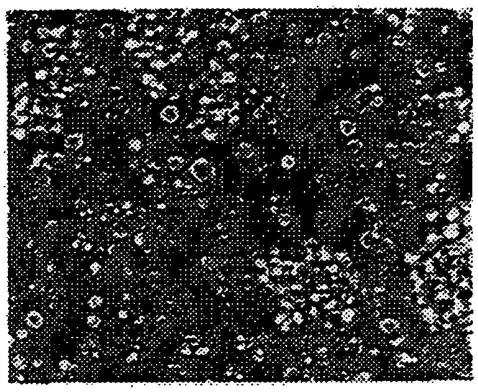

FIG.3B

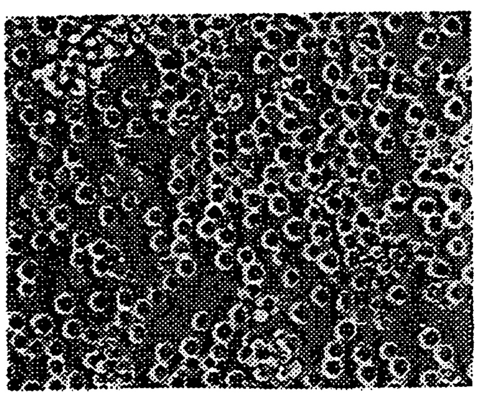

FIG.3C

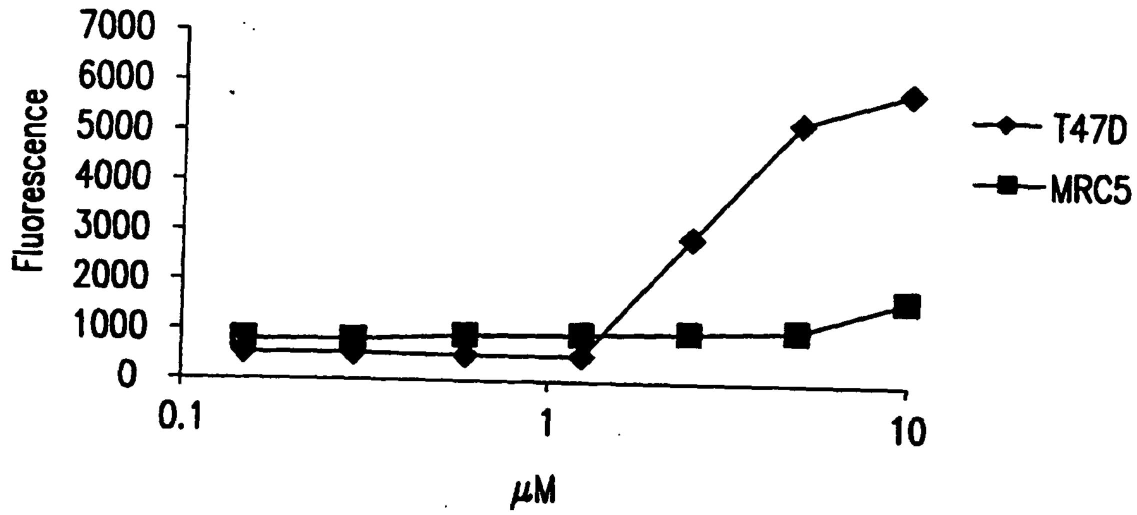
7000
6000
5000
4000
3000
2000
1000
0
Fluorescence
0.1
1
10
μM
T47D
MRC5

FIG.4

FIG.5A

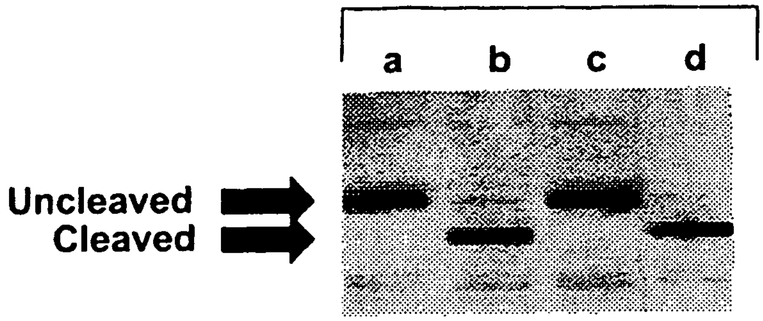


FIG.5B

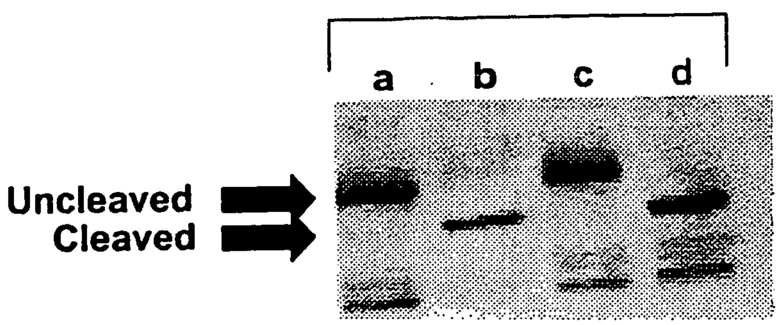

FIG.5C

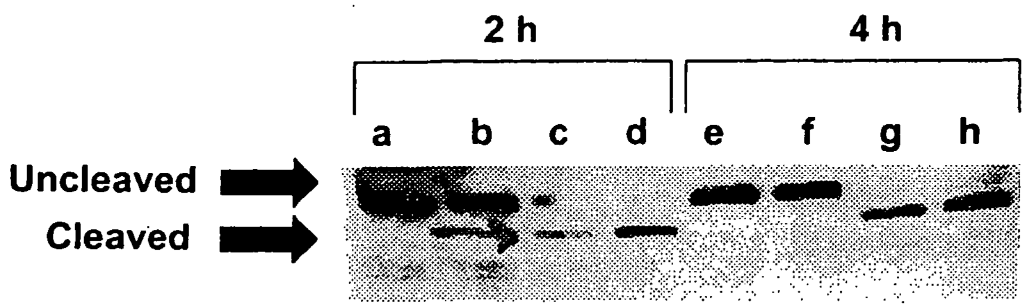
2 h
4 h
a
b
c
d
e
f
g
h
Uncleaved
Cleaved

FIG.5D

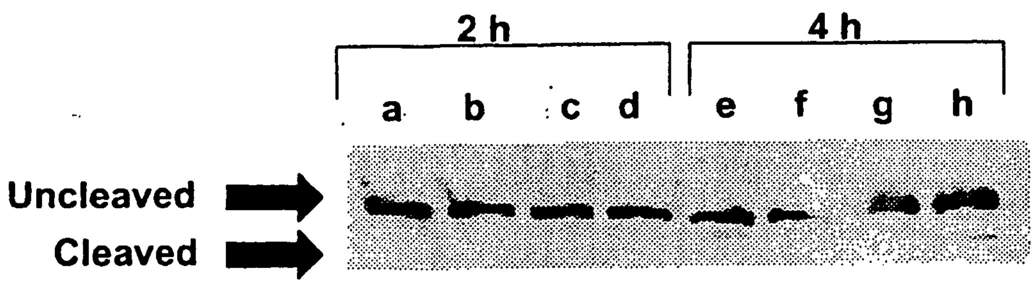
2 h
4 h
a
b
c
d
e
f
g
h
Uncleaved
Cleaved

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9620721 A
- US 3989816 A
- US 4444762 A
- WO 9918856 A
- WO 9918781 A


### Non-patent literature cited in the description


- **Glucksmann, A.** *Biol. Rev. Cambridge Philos. Soc.,* 1951, vol. 26, 59-86
- **Glucksmann, A.** *Archives de Biologie,* 1965, vol. 76, 419-437
- **Ellis et al.** *Dev.,* 1991, vol. 112, 591-603
- **Vaux et al.** *Cell,* 1994, vol. 76, 777-779
- **Orrenius, S.** *J Internal Medicine,* 1995, vol. 237, 529-536
- **Wyllie, A.H.** Cell Death in Biology and Pathology. Chapman and Hall, 1981, 9-34
- **Wyllie et al.** *Int. Rev. Cyt.,* 1980, vol. 68, 251
- **Ellis et al.** *Ann. Rev. Cell Bio.,* 1991, vol. 7, 663
- **Thornberry.** *Chemistry and Biology,* 1998, vol. 5, R97-R103
- **Thornberry.** *British Med Bull.,* 1996, vol. 53, 478-490
- Apoptosis and Cancer Chemotherapy. Humana Press, 1999
- **Schmitt et al.** *Biochem. Cell. Biol.,* 1997, vol. 75, 301-314
- **Los et al.** *Blood,* 1997, vol. 90 (8), 3118-3129
- **Friesen et al.** *Nat. Med.,* 1996, vol. 2, 574
- **Hardman et al.** Goodman and Gilman's The Pharmacological Basis of Therapeutics. McGraw-Hill, 1996, 1225-1287
- **Ahmad, S.A. et al.** *J Chem. Soc. (C),* 1966, 772-779
- **Ollis, W.D. et al.** *Tetrahedron,* 1965, vol. 21, 1453-1470
- **Asano J. et al.** *Phytochemistry,* 1996, vol. 41, 815-820
- **Lin, L.-J. et al.** *Magn. Reson. Chem.,* 1993, vol. 31, 340-347
- **Cao, S.-G. et al.** *Tetrahedron,* 1998, vol. 54, 10915-10924
- *Tetrahedron Letters,* 1998, vol. 39, 3353-3356
- **Adawadkar, P.D. et al.** *Indian Journal of Chemistry,* 1976, vol. 14B, 19-21
- **Yates, P. et al.** *Tetrahedron Letters,* 1963, vol. 24, 1623-1629
- **Subba Rao, G.S.R. et al.** *Proc. Indian Acad. Sci.,* 1978, vol. 87A, 75-86
- **Asano, J. et al.** *Phytochemistry,* 1996, vol. 41, 815-820
- **Lu, G.B. et al.** *Yao Hsueh Hsueh Pao,* 1984, vol. 19, 636-639
- **Bhat et al.** *Indian J. Chem.,* 1964, vol. 2, 405-409
- **Rao et al.** *Proc. Indian Acad Sci.,* 1978, vol. 87A, 75-86
- **Adawadkar et al.** *Indian J. Chem.,* 1976, vol. 14B, 19-21
- **Cao, S.-G. et al.** *Tetrahedron,* 1998, vol. 54 (36), 10915-10924
- **Cao, S.-G. et al.** *Tetrahedron Lett.,* 1998, vol. 39 (20), 3353-3356
- **Leong, Y.-W. et al.** *J Chem. Res., Synop.,* 1996, 392-393
- **Ohsako, S. ; Elkon, K.B.** *Cell Death Differ,* 1999, vol. 6 (1), 13-21
- **Infante, A.J. et al.** *J Pediatr.,* 1998, vol. 133 (5), 629-633
- **Vaishnaw, A.K. et al.** *J Clin. Invest.,* 1999, vol. 103 (3), 355-363
- **Lopez-Hoyos, M. et al.** *Int. J. Mol. Med.,* 1998, vol. 1 (2), 475-483
- **O'Reilly, L.A. ; Strasser, A.** *Inflamm Res,* 1999, vol. 48 (1), 5-21
- **Batteux, F. et al.** *J Immunol.,* 1999, vol. 162 (1), 603-608
- **Zhou T. et al.** *Nat Med,* 1999, vol. 5 (1), 42-8
- **Coven et al.** *Photodermatol Photoimmunol Photomed,* 1999, vol. 15 (1), 22-7
- **Ozawa et al.** *J. Exp. Med,* 1999, vol. 189 (4), 711-718
- **Heenen et al.** *Arch. Dermatol. Res.,* 1998, vol. 290 (5), 240-245
- **Wakisaka et al.** *Clin. Exp. Immunol.,* 1998, vol. 114 (1), 119-28
- **Savill, J.** *J. Leukoc. Biol.,* 1997, vol. 61 (4), 375-80

- **Boirivant et al.** *Gastroenterology,* 1999, vol. 116 (3), 557-65

SEQUENCE LISTING

<110> CYTOVIA, INC.
CAI, SUI XIONG
ZHANG, HAN-ZHONG
WANG, YAN
TSENG, BEN
KASIBHATLA, SHAILAJA
DREWE, JOHN A.

<120> GAMBOGIC ACID, ANALOGS AND DERIVATIVES AS ACTIVATORS OF CASPASES AND INDUCERS OF APOPTOSIS

<130> 1735.032PC03

<140>
<141>

<160> 1

<170> PatentIn Ver. 2.1

<210> 1
<211> 4
<212> PRT
<213> Artificial Sequence

<220>
<223> Description of Artificial Sequence: synthetic peptide

<400> 1

```
Asp Glu Val Asp
 1
```